(19) Europäisches Patentamt
European Patent Office
Office européen des brevets

(11) Publication number: 0 111 814
A2

(12) EUROPEAN PATENT APPLICATION

(21) Application number: 83112205.6

(22) Date of filing: 05.12.83

(51) Int. Cl.[3]: C 12 N 15/00
C 12 P 21/02, C 12 N 1/20
//C12R1/19

(30) Priority: 16.12.82 US 450306

(43) Date of publication of application:
27.06.84 Bulletin 84/26

(84) Designated Contracting States:
BE CH DE FR GB IT LI NL

(71) Applicant: MOLECULAR GENETICS, INC.
10320 Bren Road East
Minnetonka Minnesota 55434(US)

(72) Inventor: Glassman, Donald Lee
1417 East 80th Street
Bloomington Minnesota 55420(US)

(72) Inventor: Pilacinski, William Peter
6990 Crest Drive
Maple Grove Minnesota 55369(US)

(72) Inventor: George, Henry Joseph
5627 Green Circle Drive
Minnetonka Minnesota 55343(US)

(72) Inventor: Krzyzek, Richard Anthony
5305 Woodlawn Boulevard
Minneapolis Minnesota 55417(US)

(72) Inventor: Salstrom, John Stuart
4914 Bruce Avenue South
Edina Minnesota 55424(US)

(72) Inventor: Newman, Dawn
1010 Westbrook Way Apt. 7
Hopkins Minnesota(US)

(74) Representative: Wächtershäuser, Günter, Dr.
Tal 29
D-8000 München 2(DE)

(54) **The cloning and expression of nucleotide sequences encoding bovine growth hormone.**

(57) Methods and compositions are provided for the cloning and expression of bovine growth hormone (BGH) genetic sequences in single-cell host organisms. Also described are methods for culturing these novel single cell organisms to produce polypeptides having growth hormone activity and methods for purification of the gene product. Recombinant plasmids were constructed so as to provide an "optimal" system for the expression of BGH gene sequences in compatible host cells. The BGH related proteins produced by the recombinant DNA techniques described herein may be used in animal husbandry and in veterinary medicine.

EP 0 111 814 A2

Croydon Printing Company Ltd.

# THE CLONING AND EXPRESSION OF NUCLEOTIDE SEQUENCES ENCODING BOVINE GROWTH HORMONE



## TABLE OF CONTENTS


| | Page |
|---|---|
| 1. Field of the Invention | 4 |
| 2. Background of the Invention | 5 |
| 2.1. Recombinant DNA Technology and Gene Expression | 5 |
| 2.1.1. Efficient Gene Expression | 8 |
| 2.1.2. The Lactose and Tryptophan Operons. | 10 |
| 2.1.3. Ori Mutations | 15 |
| 2.2. Bovine Growth Hormone | 17 |
| 3. Summary of the Invention | 18 |
| 4. Brief Description of the Figures | 20 |
| 5. Description of the Invention | 24 |
| 5.1. Identification and Isolation of BGH Genes | 25 |
| 5.2. Insertion of the BGH Gene into An Expression Vector | 26 |
| 5.2.1. Construction of an Hybrid Promoter. | 29 |
| 5.2.2. Construction of BGH with Synthetic Front End | 30 |
| 5.2.3. Insertion of the SFE-BGH into an Expression Vector | 31 |
| 5.2.4. Insertion of A/T Rich Segment | 32 |
| 5.2.5. Transcriptional and Translational Termination | 32 |
| 5.3. Construction of pTRP Vectors Regulated by the LAC Operator (LacO) | 33 |
| 5.4. Preparation of Fusion Proteins | 34 |
| 5.5. Identification of the Gene Product | 36 |
| 5.6. Purification of Gene Product | 36 |

Page

5.7. Preparation of Unfused BGH.................. 39
5.7.1. Co-Transformation of Host Cells.... 40
5.7.2. Transformation of Host Cells with One Plasmid Carrying Two Genes..... 41
5.7.3. Modification of the Fusion Protein Gene........................ 41
5.8. Insertion of BGH Sequences into High Copy Number Vectors.......................... 45
6. Example: Bovine Growth Hormone...................... 45
6.1. General Procedures Used for Preparation of the Plasmids.............................. 46
6.1.1. Plasmid DNA Isolation............... 47
6.1.2. Conditions for Restriction Enzyme Digestions.................. 47
6.1.3. Restriction Enzyme Buffers.......... 48
6.1.4. Modification of DNA................. 48
6.1.5. DNA Polymerase Reaction............ 50
6.1.6. Gel Purification of DNA Fragments.. 52
6.1.7. DNA Ligation....................... 52
6.1.8. Identification of BGH Proteins by Immunoprecipitation with Anti-BGH SERA...................... 53
6.2. Isolation and Molecular Cloning of the BGH Gene.................................. 54
6.2.1. Isolation of mRNA.................. 54
6.2.2. Preparation of cDNA from BGH mRNA.. 55
6.2.3. Cloning of BGH cDNA................ 58
6.3. Cloning and Expression of BGH DNA Sequences...................................... 61
6.3.1. Cloning and Expression of BGH in pJS413......................... 61
6.3.2. Cloning and Expression of BGH In pDN572 Met-Plasmids............. 64
6.3.3. Expression Vector pDN612........... 66

Page 6.3.4. Generation of Met-BGH by Employing A Synthetic HindIII-NcoI Linker.... 67
6.3.5. The Construction of the Plasmid pN-37 BY Employing a Synthetic BglII-NcoI Linker.................. 69
6.3.6. Method for the Generation of Met-BGH by Employing Primer Repair............................ 70
6.4. Construction of Synthetic Front End........ 72
6.4.1. Construction of Plasmids pSFE11, p104-14 and p102-1................. 73
6.4.2. Construction of ptac Clones........ 74
6.4.3. Insertion of A/T Rich Sequences.... 76
6.4.4. Insertion of Translational Termination Sequences.............. 78
6.4.5. Insertion of Transcriptional Termination Sequences.............. 79
6.4.6. Construction of ptac-A/T-SFE-BGH-TERM Plasmid.................... 80
6.5. Insertion of BGH into PPV Fusion Protein Plasmid........................... 81
6.5.1. Insertion of pTAC BGH Sequences into the PPV Fusion Protein Plasmid............................ 82
6.5.2 Insertion of p214-1 into the PPV-Fusion Protein Plasmid......... 82
6.5.3 Insertion of pTAC-A/T-TTS-att-BGH into the PPV Fusion Protein Plasmid............................ 83
6.5.4 Analysis of Proteins Produced by Transformants................... 84
6.5.5. Quantitation of Protein............ 85
6.6 Insertion of BGH Sequences into the High Copy Number Plasmid pORI-TET #1....... 86

Page
6.7. Construction of pTRP Expression Vectors.... 87
6.7.1. Construction of Plasmid pTC503..... 87
6.7.2. Construction of Plasmid pTNA771.... 88
6.7.3. Construction of Plasmid Polink 23456...................... 88
6.7.4. Construction of Plasmid pTNAP771... 89
6.7.5. Construction of pTRP Vector pSFE-1 Which Expresses BGH................ 89
6.7.6. Construction of pTRP Vectors Which are Regulated by the LAC Operator.. 90
6.7.7. Construction of Plasmid p407-29 Which is Regulated by LACO......... 91
6.8. Plasmids that Express the BGH Gene......... 92
7. Deposit of Microorganisms........................ 96

## 1. FIELD OF THE INVENTION

This invention is directed to processes for the production of proteins related to bovine growth hormone (BGH) and to processes for production of novel plasmid vehicles, both eucaryotic and procaryotic, which can be used to produce such proteins.

The present invention utilizes recombinant DNA techniques to insert a DNA sequence coding for BGH or a portion thereof into a DNA vector, such as viral DNA, plasmid DNA or bacteriophage DNA, such that the vector is capable of replicating and directing expression of the BGH DNA sequence in a bacterial host or other single cell system. The resulting recombinant DNA molecule is introduced into host cells to enable production of BGH by the host cells. The protein produced is then isolated, purified and modified for use as a biologically active growth promoting molecule.

## 2. BACKGROUND OF THE INVENTION

### 2.1. RECOMBINANT DNA TECHNOLOGY AND GENE EXPRESSION

Recombinant DNA technology involves insertion of specific DNA sequences into a DNA vehicle (vector) to form a recombinant DNA molecule which is capable of replication in a host cell. Generally, the inserted DNA sequence is foreign to the recipient DNA vehicle, *i.e.*, the inserted DNA sequence and the DNA vector are derived from organisms which do not exchange genetic information in nature, or the inserted DNA sequence may be wholly or partially synthetically made. In recent years several general methods have been developed which enable construction of recombinant DNA molecules. For example, U.S. Pat. No. 4,237,224 to Cohen and Boyer describes production of such recombinant plasmids using restriction enzymes and methods known as ligation. These recombinant plasmids are then introduced and replicated in unicellular organisms by means of transformation. Because of the general applicability of the techniques described therein, U.S. Pat. No. 4,237,224 is hereby incorporated by reference into the present specification.

Another method for introducing recombinant DNA molecules into unicellular organisms is described by Collins and Hohn in U.S. Pat. No. 4,304,863 which is also incorporated herein by reference. This method utilizes a packaging/transduction system with bacteriophage vectors (cosmids).

Regardless of the method used for construction, the recombinant DNA molecule must be compatible with the host cell, *i.e.*, capable of autonomous replication in the host cell. The recombinant DNA molecule should also have a marker function which allows the selection of host cells so transformed by the recombinant DNA molecule. In addition, if all of the proper replication, transcription and translation signals are correctly arranged on the plasmid, the foreign gene will be properly expressed in the transformed cells and their progeny.

These different genetic signals and processing events control many levels of gene expression, for instance, DNA transcription and messenger RNA translation. Transcription of DNA is dependent upon the presence of a promoter which is a DNA sequence that directs the binding of RNA polymerase and thereby promotes transcription. The DNA sequences of eucaryotic promoters differ from those of procaryotic promoters. Furthermore, eucaryotic promoters and accompanying genetic signals may not be recognized in or may not function in a procaryotic system.

Similarly, translation of messenger RNA (mRNA) in procaryotes depends upon the presence of the proper procaryotic signals which differ from those of eucaryotes. Efficient translation of mRNA in procaryotes requires a ribosome binding site called the Shine Dalgarno (SD) sequence on the mRNA. This sequence is a short nucleotide sequence of mRNA that is located before the chain initiation codon (AUG) which encodes the amino-terminal methionine of the protein. The SD sequences are complementary to the 3'- end of the 16S rRNA (ribosomal RNA) and probably promote binding of mRNA to ribosomes by duplexing with the rRNA to allow correct positioning of the ribosome. For a review on maximizing gene expression, see Roberts and Lauer, 1979, Methods in Enzymology 68: 473.

Many factors complicate the expression of eucaryotic genes in procaryotes even after the proper signals are inserted and appropriately positioned. A clear understanding of the nature of these factors and the mechanisms by which they operate is presently lacking. One such factor is the presence of an active proteolytic system in *E. coli* and other bacteria. This protein-degrading system appears to selectively destroy "abnormal" or foreign proteins such as eucaryotic proteins. A tremendous utility, therefore, would be afforded by the development of a means to protect eucaryotic proteins produced in bacteria from proteolytic degradation. One strategy is to construct hybrid genes in which the eucaryotic sequence is ligated in phase (*i.e.*, in the correct reading frame) with a procaryotic gene. Expression of this hybrid gene results in a fusion protein product (a protein that is a hybrid of procaryotic and foreign or eucaryotic amino acid sequences).

Construction of hybrid genes was the approach used in the molecular cloning of genes encoding a number of eucaryotic proteins, such as somatostatin, rat proinsulin, growth hormone, and ovalbumin-like protein. Additionally, procaryotic promoters have been ligated to such fusion protein gene sequences in the case of ovalbumin and ß-globin (Guarente *et al.*, 1980, Cell 20: 543). The Guarente *et al.* system involves inserting the

*lac* promoter, including the SD sequence, at varying distances in front of the ATG of the fusion protein gene. Although the molecular cloning and expression of several eucaryotic genes has been accomplished, this has not heretofore been done for the BGH gene. Nor is the state of the art such that expression of foreign or eucaryotic genes in procaryotic host cells may be routinely performed.

Successful expression of a cloned gene requires efficient transcription of DNA, translation of the mRNA and in some instances post-translational modification of the protein. Expression vectors have been used to express genes in a suitable host and to increase protein production. The cloned gene should be placed next to a strong promoter which is controllable so that transcription can be turned on when necessary. Cells can be grown to a high density and then the promoter can be induced to increase the number of transcripts. These, if efficiently translated will result in high yields of protein production. This is an especially efficient system if the foreign protein is deleterious to the host cell.

### 2.1.1. EFFICIENT GENE EXPRESSION

Recent studies have shown that the major outer membrane proteins of *E. coli* are present in large quantities in the cell. The lipoprotein (lpp) gene of *E. coli* for example, is expressed at levels reaching 700,000 to 750,000 molecules/cell, demonstrating an extremely efficient transcriptional and translational system. Other outer membrane proteins (OMP), such as *OmpA* and *OmpF* are each produced in similar quantities indicating that these systems also have very efficient machinery for gene expression.

Although the mechanisms which are responsible for the highly efficient expression of these E. coli genes are not yet fully understood, it is believed that several factors must contribute to the abundance of such proteins in the host cell.

It has been found that in comparison with other known promoter regions of E. coli genes, that the promoter regions of the outer membrane protein genes are extremely A/T rich which is believed to be essential for efficient transcription of the genes. These OMP gene sequences also have regions which strongly bind RNA polymerase and are important for the initiation and efficiency of transcription of these genes.

Further analysis has revealed that A/T rich sequences are found between the SD sequence and the ATG of the Omp genes. This region (A/U on the mRNA transcript) is thought to play an important role in the initiation of translation. The A/U richness of this segment in the mRNA transcript may be needed to destabilize the secondary structure of the mRNA within this region and thereby facilitate ribosome binding and subsequent translation along the message.

Nucleotide sequence information of highly expressed genes in E. coli and yeast, has also shown a preferred codon "bias" which correlates well with the intracellular amounts of tRNA's for those preferred codons. (i.e., a high frequency of codon usage correlates with large high tRNA pools of tRNA for that amino acid.) Being the most highly expressed genes in bacteria, the OMP sequences show an extreme bias for certain codons for most of the amino acids, thereby providing another factor for the highly efficient expression of these proteins within the bacterial cell.

Resolution of the DNA sequence at the 3'-end of the coding region of the OMP genes, has also disclosed a consensus-type bacterial transcription termination signal. The DNA immediately preceeding the site of transcription termination is GC-rich and possesses dyad symmetry. The 3'- terminus of the transcript typically contains a series of uridine residues. These factors contribute to the overall efficiency of transcription by hastening the rate of the mRNA production and by limiting the overall size of the mRNA transcribed from the DNA.

Finally, the presence of all three translational stop codons in the 3'- untranslated region of the mRNA transcript, all three of which are in the same translational reading frame as the "coding" frame of the mRNA, provide a sequence of tandem terminators of translation. Again, this factor probably contributes to the overall efficiency of translation by assuring proper translational termination with little chance of read-through into the untranslated region of the mRNA.

2.1.2. THE LACTOSE AND TRYPTOPHAN OPERONS

An operon is a coordinated unit of gene expression. The genetic elements of an operon are a regulatory gene, an operator, and the associated structural genes which are under the control of a single promoter. The regulatory gene directs the production of a repressor protein that interacts with the operator to prevent transcription of the associated structural genes. (Binding of the repressor protein to the operator gene interferes with the binding of RNA polymerase to the promoter and, therefore, prevents initiation of transcription.) Induction of transcription (derepression) can be accomplished by preventing the interaction between the repressor protein and the operator so that transcription is initiated. The resulting transcript is a single mRNA which encodes all of the associated structural genes.

For example, the genes controlling the synthesis of enzymes sequentially linked in a metabolic pathway such as the lactose (lac) genes of *E. coli*, *z*, *y*, *a* are induced or repressed together. The *z*, *y*, and *a* genes code for the enzyme ß-galactosidase, which catalyzes the hydrolysis of ß-galactosides (*e.g.*, lactose), ß-galactoside permease (a membrane protein which transports galactosides into the bacterial cell), and ß-galactoside transacetylase (which functions *in vitro* to transfer an acetyl group from acetyl-CoA to galactosides), respectively. These genes can be induced only by molecules with an intact unsubstituted galactoside residue such as lactose and thiogalactosides (isopropyl-ß-D-thiogalactoside). The inducer need not be metabolized by its enzymes (*i.e.*, a gratuitous inducer).

The regulator *i* governs the synthesis of the *lac* repressor protein. In the absence of inducer, the regulator *i* directs the synthesis of a repressor protein which binds to the operator and prevents the structural genes (*z*, *y*, *a*) from functioning. When an inducer is added to the bacterial culture, it binds to the repressor protein converting it to its inactive form (*i.e.*, changing its conformation) and therefore the repressor can no longer bind to the operator. The structural genes can now express their protein products. In the *lac* operon, this interaction between repressor protein and operator occurs at the level of transcription. When the repressor protein binds to the operator region of the DNA, no transcription will take place until an inducer is added to the cells. (For a more complete review of the lactose operon see (Lewin, 1974, Gene Expression, John Wiley and Sons, pp. 272-309.)

In a wild type E. coli cell, the tryptophan operon encodes five genes which code for enzymes that participate in the biosynthesis of tryptophan from chorismic acid. When there is not sufficient tryptophan in the cell to support normal growth, the tryptophan operon is induced and the five enzymes are synthesized and begin to convert chorismic acid to tryptophan. When tryptophan levels in the cell are high, the operon is repressed, and synthesis of the five enzymes is inhibited.

When an operon such as the tryptophan operon is repressed, transcription of mRNA is prevented and thus no proteins encoded by that operon can be synthesized. The repressor for the tryptophan operator is a protein coded for by the trp R gene, a gene that is not physically a part of the tryptophan operon, but is located quite a distance from the tryptophan operon on the E. coli chromosome. In order to repress trancription from the trp operon, the repressor protein must first complex with the amino acid tryptophan (which is referred to as the corepressor). The repressor-corepressor complex binds to the trp operator region and physically prevents the binding of RNA polymerase to the promoter site. Since RNA polymerase cannot bind to the promoter, no mRNA can be transcribed and, therefore, no proteins encoded by the structural genes of the tryptophan operon can be made. Since two components (the repressor protein and tryptophan) are required for repression, a diminution of either will result in at least partial induction (i.e., derepression) of the tryptophan operon. The tryptophan operon is really a very fine-tuned system since the entity which the operon is programmed to help produce (tryptophan) is one of the components that turns the operon off.

In addition, transcription of the trp operon is also regulated by a transcription termination site called the attenuator, that is located between the operator and the first gene in the operon (i.e., the first gene expressed in the enzyme pathway). The attenuator site complements the operator site in regulating transcription of the trp genes as follows: when tryptophan is abundant, transcription of the trp operon is blocked by the binding of the tryptophan-repressor complex to the operator. As the concentration of tryptophan in the cell decreases, the trp operon is derepressed and transcription is initiated. However, some of the RNA polymerase molecules fall off the template DNA strand at the attenuator site, whereas other RNA polymerase molecules continue to transcribe the entire trp message. The proportion of RNA polymerase molecules that proceed past the attenuator site increases as the tryptophan concentration decreases.

A number of researchers have reported the use of the tryptophan operon regulatory region (including the promoter, the operator, a ribosome binding site sequence (rbs), and an initiation ATG in plasmid vectors designed to express foreign genes in E. coli (Christie and Platt, 1980, J. Molecular Biology, 143:335; European Patent Application No. 0036776). The ribosome binding site plays a crucial role in determining the level of translation into protein of mRNA transcribed from the gene sequences associated with the regulatory region of an operon such as the tryptophan operon. The majority of expression vectors containing the tryptophan operon which are in use at the present time contain the natural ribosome binding site of either the tryptophan leader sequence (Edman et al., 1981, Nature, 291:503; Miozzari et al., 1978, J. Bacteriology, 133:1457; Kleid et al., European Patent Application EP 0 036 770 A2; Goeddel et al., 1980, Nature, 287:411) or the tryptophan E gene (Hallewell and Emtage, 1980, Gene, 9:27).

Two basic methods have been employed rather extensively to induce the transcription of "foreign" genes ligated to the controlling region of the tryptophan operon: (1) the addition of tryptophan analogs to the culture medium and (2) the dilution of tryptophan in the medium to low levels such that there will be insufficient repressor-corepressor complex to prevent transcription. Both of these methods (described below) attempt to manipulate repression by altering some function of the co-repressor.

Tryptophan analogs, such as 3-indolylacrylic acid, can bind to the tryptophan repressor protein just as tryptophan does, however the resulting repressor-indolylacrylic acid complex cannot bind to the operator and therefore does not prevent transcription of the operon. Although this method makes it possible to induce transcription of the operon, it disrupts translation and thus reduces gene expression. Specifically, although transcription proceeds rather well, the translation process is disrupted (1) because the translational machinery attempts to insert the tryptophan analog into a growing polypeptide chain at the point where the nucleic acid code calls for the addition of a tryptophan molecule; and (2) because translation is inhibited none of five enzymes are produced, therefore, no indole is converted to tryptophan (thus effectively starving the cells of tryptophan).

The second method of induction, dilution of tryptophan in the cell culture medium is not deleterious to the cell but requires that an actively growing culture harboring a recombinant plasmid carrying a foreign gene under control of the tryptophan operon be diluted 10-or 20-fold in order to reduce the tryptophan levels sufficiently to obtain significant induction. This method requires growth in large volumes of media and/or reduced yield of gene product per volume of culture.

Thus, while it is possible to obtain expression of proteins using the tryptophan operon, it would be advantageous to provide a method for optimal gene expression of using methods that neither require the addition of harmful tryptophan analogs nor the dilution of cells in culture.

### 2.1.3. ORI MUTATIONS

Most plasmid cloning vectors commonly used in *E. coli* are derivatives of ColE1-type replicons (for additional information see Oka, *et al.*, 1979, Mol. Gen. Genet. 172:151-159). The ColE1 plasmids are stably maintained in *E. coli* strains as monomeric molecules with a copy number of about 15-20 copies per chromosome. Various levels of expression of human and animal health-care protein products of foreign genes inserted into these plasmids has been obtained. However, very high expression levels must be obtained in order for it to become economically feasible to produce these protein products.

One way to obtain large amounts of a given gene product is to clone a gene on a plasmid which has a very high copy number within the bacterial cell. In theory, by increasing the number of copies of a particular gene, the cell would also increase its mRNA and therefore its production of protein. Muesing et al. (1981, Cell, 24: 235-242) have isolated the recessive copy-number mutant, Cop plasmid pOP1 6, from ColE1. This plasmid, which exists at levels of about 200-300 copies per chromosome, is characterized by a mutation on the plasmid genome about 400 b.p. from the origin of replication of a single base-pair alteration--a G-C to T-A transversion in the copA gene. The copA sequence is thought to code for a small RNA transcript (RNA1) which may act as a negative modulator of the ColE1 DNA replication machinery. It is believed that a disruption of the secondary structure of the RNA by the mutation is responsible for the loss of its negative controlling effects.

Uhlin et al. (1983, Gene, 22:255-265) have reported a series of plasmids which are double cop (copy number) mutants of the plasmid R1. These plasmids are unique in that they carry a temperature sensitive mutation which allows the plasmid to be present in moderate numbers per cell at temperatures below 35°C. Above 35°C, all control of plasmid replication is apparently lost and the number of plasmid copies per cell increases continuously to nearly 2000. Cell growth and protein synthesis continue at normal rates for 2-3 hours at the higher temperature. During this period, products of genes present on the plasmid may be overproduced. Inhibition of cell growth occurs after 2-3 hours and the cell loses viability. Eventually, plasmid DNA may account for as much as 75% of the total DNA present in the cell.

## 2.2. BOVINE GROWTH HORMONE

Growth hormone (sometimes called somatotropin) is a protein that is secreted from the adenohypophysis of the pituitary glad of mammals and affects the rate of skeletal growth and gain in body weight. A defect in growth hormone secretion or secretion of an inactive growth hormone molecule in young animals results in a greatly retarded growth rate or even a complete failure to grow. In young animals that are suffering from a lack of endogenous growth hormone, continuous administration of exogenous growth hormone results in a complete restoration of normal development.

Although the most obvious role of growth hormone in animal development is its promotion of skeletal growth, the hormone also has an effect on many other metabolic functions including increased insulin release from the pancreas, increased glucagon secretion, a lipid mobilizing effect, stimulation of reticulocytosis, a lactopoietic effect and stimulation of chondrogenesis and osteogenesis.

Another valuable benefit of growth hormone is in the cattle industry. Exogenous administration of bovine growth hormone increases the growth rate, decreases the fattening time, increases the meat-to-fat ratio and increases milk production in domestic cattle. Until the present invention, supplies of bovine growth hormone could only be obtained by tedious and expensive extraction and isolation of excised bovine pituitary glands, and the quantities of hormone that would be required for a large commercial scale administration to cattle were simply not available.

Bovine growth hormone is a protein composed of 191 amino acids having the a molecular weight of approximately 22,000 daltons. The hormone is synthesized by the cell first as a "pre-growth hormone" that contains a 26 amino acid "leader" or signal sequence which may be involved in transport to the cellular membrane. This signal sequence is enzymatically removed to produce the nature protein biologically active molecule.

The production of modified bovine growth hormone is described in U.K. Patent Application GB 2,073,245 and in European Application No. 47,600, and also by Keshet et al., Nucleic Acid Res., 9(1):19-30 1981). The molecular cloning but not the expression of DNA complementary to bovine growth hormone mRNA has been described (Miller et al., 1980, J. of Biol. Chem. 255: 7521-7524).

## 3. SUMMARY OF THE INVENTION

Methods and compositions are provided for the cloning and expression of growth hormone genetic sequences in single-cell host organisms. Also described are methods for culturing these novel single-cell organisms to produce polypeptides having growth hormone activity (herein after referred to as GH proteins) and methods for the purification of the gene product. Recombinant plasmids were constructed so as to provide an "optimal" system for the expression of bovine growth hormone (BGH) gene sequences in compatible host cells. The BGH related proteins produced by the recombinant DNA techniques described herein may be used in animal husbandry and in veterinary medicine.

The genetic sequence which encodes BGH may be obtained by isolating BGH mRNA and preparing cDNA using the mRNA as a template. The cDNA sequences can subsequently be processed and inserted into plasmid vectors to form recombinant plasmids which serve as biologically functional replication units. These recombinant plasmids are constructed so as to facilitate both the replication and expression of the BGH genes upon transformation of compatible host cells. Additionally, the plasmids provide for a one-step identification of transformed microorganisms actively expressing the BGH genes.

Methods and compositions are provided for construction of a plasmid cloning vector which has a very high copy number within the bacterial cell. By inserting the BGH gene into this expression vector the number of copies of the gene is increased and therefore production levels of protein are increased.

Methods and compositions are provided for the construction and synthesis of an expression vector containing the tryptophan operon controlling region. Expression of genetic sequences inserted into this plasmid is obtained by transforming a host cell. The <u>trp</u> vectors described herein are unique in that they contain a <u>trp</u> promoter which is regulated by a <u>lac</u> operator region. This method allows for the manipulation of repression by altering the repressor molecule.

Finally, methods are described for the isolation, stabilization, and purification of the expressed gene products. These methods take advantage of the fact that fusion proteins are resistant to degradation in the host cell. Methods are provided for the co-production of both the fusion protein and the unfused product. Methods are also provided for the isolation and purification of the co-aggregate forming proteins.

## 4. BRIEF DESCRIPTION OF THE FIGURES

The present invention may be more fully understood by reference to the following detailed descriptions of the invention, examples of specific embodiments of the invention, and the appended figures.

In all figures within this text, restriction endonuclease enzyme restriction sites are indicated by the following abbreviations: AhaIII, BamHI, BglII, ClaI EcoRI, EcoRV, HaeII, HaeIII, HincII, HindIII, HinfI, HpaII, NarI, NcoI, PstI, PvuII, RsaI, SacI, SalI, Sau3A 1, SmaI, TaqI, XbaI, XmaI, XmnI.

Unless otherwise indicated, the figures which follow are not drawn to scale.

FIG. 1 is a diagrammatic representation and partial restriction endonuclease cleavage map of BGH cDNA clone pBH27 which contains the entire coding sequence for pre-BGH and BGH. Only relevant restriction sites are indicated. Fragment sizes are denoted in base pairs (b.p.).

FIG. 2 represents the nucleotide sequence of the PstI BGH DNA insert of the positive strand of pBH27. The differences between the reported mRNA sequence of Miller et al. (1980, J. Biol. Chem., 225: 7521-7524) and the pBH27 sequence are indicated above the pBH27 sequence; nucleotides present in the pBH27 sequence but not found in the unpublished sequence are indicated by a single asterisk (*). Only relevant restriction sites are indicated.

FIG. 3 is a diagrammatic representation of the construction of the p3-30/IM Bal 31 plasmid population.

FIG. 4 is a diagrammatic representation of the construction of pRed13.

FIG. 5 is a diagrammatic representation of the construction of p572-13-1 modified-met-BGH fusion plasmid series and p572-13-1 BglII/NcoI heterogenous population.

FIG. 6 is a diagrammatic representaion of the reconstructed mature BGH.

FIG. 7 shows pertinent nucleotide and corresponding amino acid sequences of several met-plasmids.

FIG. 8 and 8A represents the construction of modified plasmids pBG18C1 and pJS413-612N met-BGH.

FIG. 9 represents the construction of pN-37.

FIG. 10 is a scheme showing the generation of met-BGH using the method of primer repair. The construction of plasmid p27 is shown in FIG. 10A. The construction of plasmid p27-112-4 is shown in FIG. 10B.

FIG. 11 is a sequence comparison of the Natural Front End (NFE) sequences from the cDNA clone pBH27 of BGH to those of the Synthetic Front End (SFE) construction.

FIG. 12 is a diagrammatic representation of pSFE-11. FIG. 12A is a diagrammatic representation of p104-14 and p102-1.

FIG. 13 shows the construction of Ptac from Ptrp and Placuv5. The -35 and -10 regions of each promoter are underlined and their respective binding sites (SD) are overlined. The initiation of transcription is labeled at the first nucleotide.

FIG. 14 and 14A represent the construction of various Ptac/BGH plasmids. FIG. 14B shows the distance between the $SD^{lac}$ and $SD^{cro}$ regions of two ptac/BGH plasmids. The SD regions are underlined.

FIG. 15 represents the construction of p146-1 which contains an A/T rich intervening sequence between the $SD^{cro}$ and the ATG of BGH.

FIG. 16 represents a comparison of the intervening sequences between the $SD^{cro}$ and ATG of BGH constructs p102-1, p128-7/2, and p146-1.

FIGS. 17 and 17A represent the construction of p166-1 and p179-3.

FIG. 18 is a diagrammatic representation of the insertion of a DNA oligomer containing three translational stop sequences (TTS) to generate p175-12.

FIG. 19 represents the insertion of the trp attenuator sequences 3'- to the TTS of BGH to generate p211-4.

FIG. 20 is a diagrammatic construction of p217-1 and p214-1.

FIG. 21 represents the construction of pPPV 1000tx.

FIG. 22 is a diagrammatic representation of the construction of p158-1 amd p195-4.

FIG. 23 is a diagrammatic representation of the construction of various recombinant plasmids which contain the PPV fusion protein and BGH coding sequences on one plasmid.

FIG. 24 represents the construction of pORI-tet#1.

FIG. 25 represents the construction of various recombinant plasmids which contain ptac-A/T-SFE-BGH-TTS-att and the ori mutation.

FIG. 26 represents the construction of pTC503. The abbreviation p/o stands for the promoter/operator sequences of trp.

FIG.27 represents the construction of pTNA771.

FIG. 28 represents the construction of plasmid Polink 23456.

FIG. 29 is a diagrammatic representation of the construction of pTNAP771.

FIG. 30 represents the nucleotide sequence of the PvuII/BglII fragment from pTNAP771.

FIG. 31 is a diagrammatic representation of pSFE-1.

FIG. 32 is a diagrammatic representation of p106-15.

FIG. 33 is a diagrammatic representation of the construction of pT248-11.

FIGS. 34 and 34A represent the construction of pMTE132 from plasmids pT248-11, p106-15, and the synthetic *lac*O-25-mer fragment. FIG. 34B represents the construction of pMTE407-29 from pMTE132 and pSFE-1. FIG. 34C represents the nucleotide sequence of the promoter/operator, *lac*O, and SD region of pMTE-407-29.

## 5. DESCRIPTION OF THE INVENTION

This invention relates to the use of recombinant DNA techniques to produce polypeptides related to BGH proteins which can be used as biologically active hormones. More specifically, the production of BGH related proteins and partially synthetic BGH related proteins is described.

The recombinant plasmids, constructed as described herein, provide for host cell production of a protein which is a BGH related polypeptide and which is stable and resistant to host cell degradation; such plasmids enable the generation of large quantities of a BGH related proteins or fusion proteins. However, the DNA compositions described herein are not limited to the production of a BGH-related protein and may be used to produce polypeptides related to any of the bovine hormone proteins.

The method of this invention may be divided into the following stages for the purpose of description: (a) isolation and identification of the BGH genetic sequences; (b) preparation and molecular cloning of the BGH genetic sequences in order to generate multiple copies of the BGH sequences or portions of the BGH sequences; (c) insertion of various portions of the cloned BGH sequence into a cloning expression vector to form a recombinant DNA molecule which is used to transform single-cell organisms; and (d) identification and purification of the gene product.

### 5.1. IDENTIFICATION AND ISOLATION OF BGH GENES

The coding sequence for BGH may be obtained from any cell or tissue which produces BGH; the most commonly known source of BGH is pituitary glands. Accordingly, pituitary glands may be homogenized and the mRNA which encodes BGH may be isolated. Reverse transcriptase may be used to convert the mRNA to cDNA; and the cDNA may then be inserted into a DNA cloning vector that is used to transform host cells. This enables the generation of multiple copies of the BGH DNA sequence so that an ample supply is available for analysis. Subsequently, the BGH DNA sequence (or portions thereof) may be subcloned into expression vectors which may then be used to transform host cells that are capable of expressing the inserted BGH gene.

When cloning the BGH gene in procaryotes, isolation of mRNA and subsequent cloning of cDNA encoding BGH is advantageous because the gene sequence is obtained in an uninterrupted form. However, alternatives to this technique include, but are not limited to, chemically synthesizing the gene DNA sequence itself (or a portion thereof), or isolating the genomic DNA that encodes BGH, or isolating the BGH gene from a recombinant vector in which the gene had previously been cloned.

## 5.2. INSERTION OF THE BGH GENE INTO AN EXPRESSION VECTOR

Once BGH cDNA-containing recombinant plasmids are identified, the BGH DNA sequences (hereinafter referred to as a BGH gene) are subcloned into appropriate expression vectors, *i.e.*, vectors which contain the necessary elements for transcription and translation of the inserted gene. To obtain efficient expression of the BGH gene (or a portion of the gene), a promoter located 5' to the inserted BGH gene must be present in the expression vector. RNA polymerase normally binds to the promoter and initiates transcription of a gene or a group of linked genes and regulatory elements. This group is called an operon. Promoters vary in their "strength", *i.e.*, their ability to promote transcription and thus produce large quantities of gene product. Depending upon the host cell system utilized, any one of a number of suitable promoters may be used. For instance, when cloning in an *E. coli*, its bacteriophages or plasmids promoters such as the *lac* promoter, *trp* promoter, *rec*A promoter, ribosomal RNA promoter, the $P_R$ and $P_L$ promoters of coliphage lambda and others including but not limited to *lac*uv5, *trp*-*lac*uv5 (*tac*) hybrid promoter, *omp*F, *bla*, *lpp* and the like, may be used to direct high levels of transcription of adjacent DNA segments. Additionally, *E. coli* promoters produced by recombinant DNA or other synthetic DNA techniques may be used to provide for transcription of the inserted gene.

When cloning in a eucaryotic host cell, enhancer sequences (*e.g.*, the 72 b.p. tandem repeat of SV40 DNA, retroviral long terminal repeats or LTRs, etc.) may be inserted to increase transcriptional effeciency. Enhancer sequences are a set of eucaryotic promoter elements that appear to increase transcriptional efficiency in a manner relatively independent of their position and orientation with respect to a nearby gene. Unlike the classic promoter elements (e.g., the polymerase binding site and the Goldberg-Hogness "TATA" box) which must be located immediately 5' to the gene, enhancer sequences have a remarkable ability to function upstream from, within, or downstream from eucaryotic genes; therefore, the position of the enhancer sequence with respect to the inserted BGH gene is less critical.

Specific initiation signals are also required for efficient translation in procaryotic and eucaryotic cells. Protein translation is initiated as the result of interaction between two mRNA sites: the ribosome binding site (SD sequence on the DNA complement) and the initiation codon AUG (ATG on the DNA complement). These translation initiation signals may vary in "strength" as measured by the quantity of gene specific protein synthesized. The DNA expression vector, in addition to containing a promoter for transcription, may also contain any combination of various "strong" translation initiation signals. Thus, any SD-ATG combination that can be utilized by host cell ribosomes may be employed; such combinations include, but are not limited to, the SD-ATG combination from the cro gene or the N gene of coliphage lambda, or from the E. coli tryptophan E, D, C, B, or A genes. Additionally, any SD-ATG combination produced by recombinant DNA or other synthetic technique may be used.

Any of the methods previously described for the insertion of DNA fragments into a vector may be used to ligate a promoter and other control elements into specific sites within the vector.

Accordingly, the BGH gene (or any portion thereof) can be ligated into an expression vector at a specific site in relation to the vector promoter and control elements so that the BGH gene sequence is in the correct translational reading frame (i.e., in phase) with respect to the vector ATG sequence. Alternatively, the vector and/or gene sequence may be modified so that the ATG (or GTG or CTG) of the gene sequence is used as the initiation signal. The resultant recombinant DNA molecule is then introduced into appropriate host cells by transformation, transduction or transfection (depending on the vector/host cell system). Transformants are selected based upon the expression of appropriate gene markers normally present in the vectors, such as ampicillin resistance or tetracycline resistance in pBR322, or thymidine kinase activity in eucaryotic host systems. Expression of such marker genes indicates that the recombinant DNA molecule is intact and is replicating. Expression vectors may be derived from cloning vectors, which usually contain a marker function; such cloning vectors may include, but are not limited to the following: SV40 and adenovirus vectors, yeast vectors, bacteriophage vectors such as lambda gt-WES-lambda B, Charon 28, Charon 4A, lambda gt-1-lambda BC, lambda gt-1-lambda B, M13mp7, M13mp8, M13mp9, or plasmid DNA vectors such as pBR322, pAC105, pVA51, pACYC177, pKH47, pACYC184, pUB110, pMB9, pBR325, Col E1, pSC101, pBR313, pML21, RSF2124, pCR1 or RP4.

In addition, host cell strains may be chosen which inhibit the action of the promoter unless specifically induced. In this way, greater than 95% of the vector's promoter's effectiveness may be inhibited in uninduced cells. As previously explained, in certain operons the addition of specific inducers is necessary for efficient transcription and translation of the inserted DNA; for example, the lac operon is induced by the addition of lactose or IPTG (i.e., isopropylthio-ß-D-galactoside). A variety of other operons, such as trp, pro, tac etc., are under different controls. The trp operon is induced when tryptophan is absent in the growth media; and the $P_L$ promoter of lambda is induced by an increase in temperature in host cells containing a temperature sensitive lambda repressor protein, e.g., c1857. Thus, expression of the genetically engineered GH protein may be controlled. This is important if production of the protein product of the cloned gene is lethal or otherwise detrimental to the host cells. In such cases, the foreign gene may be replicated but not expressed during growth of the transformants. After the cells reach a suitable density in the growth medium, the promoter can be induced for production of the protein.

### 5.2.1. CONSTRUCTION OF AN HYBRID PROMOTER

One such promoter/operator system is the so-called "tac" or trp-lac promoter/operator system (referred to as "tac"). This hybrid promoter is constructed by combining the -35 b.p. (-35 region) of the trp promoter and the -10 b.p. (-10 region or Pribnow box) of the lac promoter (the sequences of DNA which are the RNA polymerase binding site). In addition to maintaining the strong promoter characteristics of the tryptophan promoter, tac is also easily controlled by the lac repressor (from lac $I^q$). This construction may explain the higher efficiency of expression of this hybrid promoter with respect to either one of the parental promoters.

5.2.2. CONSTRUCTION OF BGH WITH SYNTHETIC FRONT END

Expression levels of BGH DNA sequences derived from the cDNA library containing natural eucaryotic coding sequences were determined to be very low (less than 0.01% of the total protein expressed by the cell) in a number of expression vector constructions. In order to increase production of BGH, a DNA fragment coding for the N-terminus of BGH (i.e., amino acids 1 (methionine) through 24) is synthesized using state-of-the-art procedures. This fragment (SFE or synthetic front end) is inserted in place of the natural front end (NFE) coding sequences of the BGH genome.

The SFE is constructed so that the "non-preferred" codons of 13 of the 24 amino acids are replaced with "preferred" codons for those amino acids. This is done for two reasons. First, in all highly expressed E. coli genes there is a certain codon "bias" for most of the amino acids of the corresponding protein. This bias correlates well with the intracellular pools of tRNA's for those preferred codons. By changing the codons in BGH which are "non-preferred" to "preferred" codons, translational efficiency should increase thereby increasing overall expression. Second, certain regions on the mRNA transcript are very important for the initiation and subsequent translation of the protein which are recognized by the host cell ribosomes. Since mRNA is single-stranded it has a tendency to fold and base pair with the complement sequences anywhere along the entire length of the transcript. If the regions critical for initiation are buried within this now folded molecule, translation, and therefore expression, will be decreased. However, if undesirable folding of the mRNA does not occur and these regions are readily accessible to the host-cell ribosomes, translation initiation and elongation of the polypeptide are subsequently increased.

### 5.2.3 INSERTION OF THE SFE-BGH INTO AN EXPRESSION VECTOR

When the SFE-BGH construction is inserted into the expression vector pDN612, a significant increase in expression levels of the met-BGH protein is found (BGH comprising greater than 0.1% of total host cell protein).

When this construction (SFE-BGH) was inserted into the expression vector p141-2, [a pDR540 modified plasmid which utilize the "tac" (trp-lac) promoter with lac and cro SD sequences] a large increase (BGH comprising greater than 1% of total host cell protein expressed) in the amount of BGH being produced was observed.

Finally, the SFE-met-BGH construct was inserted into a tryptophan expression vector pTNAP771 which utilizes the trp operon promoter and trp SD sequences for expression. Again, this construct showed a significant increase (1%) in the amount of BGH being expressed by host cells.

Since SFE constructs produced relatively stable proteins, they were modified further in order to increase stability and thus increase protein expression.

### 5.2.4. INSERTION OF A/T RICH SEGMENT

In order to improve the initiation of translation for BGH, an A/T DNA segment can be inserted between the $SD^{cro}$ and the ATG of BGH. A DNA oligomer which contains an A/T rich sequence may be synthesized using state-of-the-art nucleic acid chemistry. A particularly useful method is described in U. S. Patent Application Serial No. 491,099 filed May 3, 1983, by Kempe et al., which is incorporated herein by reference. This A/T rich segment may be needed to destabilize the secondary structure of the mRNA within this region and thereby facilitate ribosome binding and subsequent translation along the message.

### 5.2.5. TRANSCRIPTIONAL AND TRANSLATIONAL TERMINATION

Sequence analysis of the cDNA clone of BGH (pBH27) has revealed that only one translational stop codon (TAG) is present for termination of translation, and that no transcriptional-stop-like sequences are found in the 3' untranslated region of the cDNA clone.

Therefore a triple translational stop sequence, with all 3 codons can be inserted in phase with the reading frame of the protein. Again, this can be accomplished by insertion of a synthetic linker using current nucleic acid chemistry. This construction should provide for efficient termination of translation of the BGH message by assuring proper translational termination with little change of read-through into the normally untranslated region of the mRNA.

Recent studies have shown that the tryptophan attenuator region of the tryptophan operon provides for the efficient termination of mRNA transcription from the DNA. This extremely stable stem and loop structure is characteristically GC-rich and possesses dyad symmetry, followed by an oligo-(T) stretch at the site of termination similar to the well known features of rho-independent transcription termination sites in procaryotes.

A sequence coding for the attenuator portion of the tryptophan operon may be synthetically constructed or isolated from E. coli chromosomal DNA and inserted into an appropriate expression vector. In the example of the present invention, the tryptophan attenuator region was placed approximately 100 b.p. 3'- to the translational stop codons of the BGH genome. This construction may now allow for the effective termination of transcription of the mRNA from the BGH genome.

## 5.3. CONSTRUCTION OF pTRP VECTORS REGULATED BY THE LAC OPERATOR (LacO)

Expression of cloned genes in bacteria can generally be controlled by strong promoters. The ptrp vector system did not provide for significant regulation of the trp promoter by the co-repressor L-tryptophan when used for the expression of BGH. Nucleotide sequence analysis of the trp operator region demonstrated that no sequence abnormalities existed, suggesting that the lack of regulation was probably due to the low number of trp repressor molecules relative to the high number of plasmids containing trp operator regions. Since high intracellular concentrations of BGH might be toxic to the bacterial cell, the cloned BGH was placed adjacent to a strong regulatable promoter-operator region.

In order to efficiently regulate the trp promoter, a unique trp vector system was constructed in which a synthetic lac0 operator is inserted 17 nucleotides 3' to the -10 region of the trp promoter. The expression vector includes not only a trp promoter, but in addition, a lac operator which can be regulated by lac repressor in a $lacI^q$ strain of E. coli and thus efficiently repress BGH expression. The induction of this hybrid promoter-operator system is easily and efficiently accomplished by the addition of lactose or IPTG to the bacterial culture at a phase of growth where BGH synthesis is maximal. This mechanism of trp promoter regulation circumvents many of the problems associated with the derepression of the tryptophan promoter by either the addition of tryptophan analogues or by dilution of tryptophan.

## 5.4 PREPARATION OF FUSION PROTEINS

To maximize the level of gene expression in a specific transformant it may be desirable to ligate the gene in question to a gene encoding another protein, such as a host cell protein. An additional advantage is obtained if the host cell protein inherently contains an assayable function. The expression of the ligated genes results in a fusion protein product (hereinafter referred to as BGH fusion proteins) that can be identified on the basis of its large molecular weight and assayable function. For example, production of a BGH/ß-galactosidase fusion protein offers several advantages for cloning and expression of BGH in an E. coli host. First, this allows for an approximation of the level of protein production (hence expression) directed by the vector using a colorimetric assay specific for ß-galactosidase activity according to the method of Miller (pages 47-55 and 352-355 in Experiments in Molecular Genetics, Cold Spring Harbor Press, New York, N.Y., 1972). Second, fusion protein production simplifies the identification and isolation of the protein product. The unfused BGH protein produced by E. coli transformants is smaller than the BGH/ß-galactosidase fusion protein and as such may co-migrate with several other host proteins analyzed by SDS-polyacrylamide gel electrophoresis. However, the fusion protein produced can be easily detected and identified by SDS-polyacrylamide electrophoresis (SDS-PAGE) due to its unique large molecular weight.

The present invention is not limited to the production of a ß-galactosidase fusion protein; any gene of either eucaryotic or procaryotic origin may be ligated in phase with the BGH gene to provide advantages similar to the ß-galactosidase fusion protein product. Examples include, but are not limited to, galactokinase; trp D, E or leader; pilus genes; and eucaryotic genes, such as thymidine kinase, ß-globin, SV-40 T-antigen, or Rous Sarcoma Virus transforming gene.

In order to construct a gene which encodes a fusion protein, the two genes must be joined within their coding sequence such that the translational reading frame is maintained and uninterrupted by termination signals. Also, as previously explained, if the host cell is a strain which inhibits the action of the promoter, the fusion protein will be produced only in response to induction.

## 5.5. IDENTIFICATION OF THE GENE PRODUCT

Identification of the protein described in this invention is based upon two requirements. First, the BGH-related protein should be produced only in response to the induction of the promoter. Second, the BGH-related protein can be detected immunologically using a variety of polyclonal or monoclonal antibodies directed against BGH; the protein should be immunoreactive whether it results from the expression of the entire gene sequence, a portion of the gene sequence or from two or more gene sequences which are ligated to direct the production of a fusion protein. This reactivity may be demonstrated by standard immunological techniques, such as immunoprecipitations, immunodiffusion, radio-immune competition, immunoelectrophoresis or the immunological detection of proteins which were separated by polyacrylamide gel electrophoresis and then transferred to nitrocellulose.

## 5.6 PURIFICATION OF GENE PRODUCT

Cells containing the cloned gene are grown up in a large volume, and the protein produced after induction of the promoter is isolated from such cells or from the medium if the protein is excreted. The protein may be isolated and purified either by standard chromatography including ion exchange, affinity or sizing resins, by centrifugation, or by any other standard technique for the purification of proteins.

Since certain fusion proteins form aggregates when overproduced in cells and when in solution, a method for isolating aggregate-forming proteins is particularly useful for isolating the fusion proteins produced in the present invention. These fusion proteins can then be used in vaccine formulations. Purification of fusion proteins (hereinafter referred to as aggregate purification) involves the extraction, separation and/or purification of aggregate-forming proteins from protein mixtures such as lysates of cell cultures by disruption of cells followed by washing the aggregated material. Additionally, the aggregated material may be solubilized by the addition of a solvent that is both a strong hydrogen acceptor and a strong hydrogen donor (or a combination of solvents each having one of these properties); the aggregate-forming proteins may then be precipitated by dilution with a compatible buffer. Suitable protein solvents include, but are not limited to urea (from about 4M to about 8M), formamide (at least about 80%, volume/volume basis), and guanidine hydrochloride (from about 4M to about 8M). Some solvents which are capable of solubilizing aggregate-forming proteins, for example SDS (sodium dodecyl sulfate), 70% formic acid, are inappropriate for use in this procedure because subsequent precipitation of the solubilized aggregate-forming proteins has proved to be inefficient. Although guanidine hydrochloride and other similar agents are denaturants, studies indicate that this denaturation is not irreversible and that renaturation may occur upon removal (by dialysis, for example) or dilution of the denaturant, allowing re-formation of immunologically and/or biologically active protein.

One embodiment of the fusion protein isolation technique is outlined as follows (hereinafter referred to as the non-denaturing aggregate purification procedure): the cell pellet is quick frozen using dry ice/methanol, weighed, and 3-4 g of cells are resuspended in at least 25 mℓ of a buffer solution [e.g., 50 mM Tris-HCl (tris hydroxymethylaminomethane-hydrochloride), pH 8.0, 2mM EDTA

(ethylenediaminetetraacetic acid) and 200 mM NaCl]. That is, the cells are suspended in a buffer solution at an approximate concentration of from about 100 to 200 grams of cells/liter. Concentrations less than about 160 grams/liter are preferred. To this suspension lysozyme is added to a final concentration of about 130 μg/mℓ and the resulting mixture is allowed to stand at 4°C for 20 minutes with occasional shaking. Nonidet P40 (NP-40, Shell trademark, polyoxyethylene (9) p-tert-octylphenol), a non-ionic detergent used to solubilize membranes, is added to a final concentration of about 0.1% and the solution mixed. Then, the suspension is ground for approximately 1 minute using a Polytron grinder (Brinkman Instruments, Westbury, N.Y.) or equivalent.

The suspension is centrifuged at 8,000 x g for 30 minutes, and the pellet resuspended in a wash buffer, e.g., 20mM Tris-HCl (pH 7.2), 1mM EDTA, 150mM NaCl and 2% Triton-X 100 [polyoxyethylene (9-10) p-tert-octylphenol, a non-ionic detergent], and ground with the Polytron grinder. This step of centrifugation, washing, and grinding may be repeated to further wash the pellet and remove as much cellular debris as possible.

Alternatively, the pellet of aggregates may be further treated by the addition of a denaturant (hereinafter referred to as the denaturing aggregate purification procedure) as follows: The suspension is centrifuged, the supernatant removed completely and the pellet resuspended in about one-fifth volume of 6M guanidine hydrochloride (in distilled water). For instance, 3 g of cells washed with 25 mℓ of buffer should be resuspended at this step in 5 mℓ of 6M guanidine hydrochloride solution. It may be difficult to resuspend the pellet at this stage and sonication or homogenization may be required in order to obtain a homogenous solution. The solution is allowed to stand at 22°C for 20 minutes and is then centrifuged at 8,000 x g for 30 minutes to remove debris, saving the supernatant which at this point contains the fusion protein.

The fusion protein is precipitated from the guanidine hydrochloride supernatant by the addition of about four volumes of aqueous buffer. Almost any aqueous buffer may be used at this stage; however, the addition of a small amount of non-ionic detergent, such as 0.5% NP-40 or Triton X-100 may be helpful. This suspension is allowed to stand at 4°C for 30 minutes and is then centrifuged at 8,000 x g for 10 minutes. The supernatant is discarded, the pellet (containing the fusion protein precipitate) is resuspended in an appropriate buffer, e.g., Phosphate Buffered Saline (PBS) in any appropriate volume. Brief sonication or homogenization may aid in obtaining a homogenous suspension or slurry (since aggregate-forming proteins are insoluble in water).

## 5.7 PREPARATION OF UNFUSED BGH

As previously explained, transformants which produce unfused BGH proteins, generally do so in smaller quantities than transformants which produce BGH fusion proteins; this is true even when the gene sequence for the unfused protein is under the control of an inducible promoter. In addition, the unfused BGH proteins produced by bacterial transformants may be less stable than BGH fusion proteins. In an alternate embodiment of the present invention, a host cell transformant can be engineered to produce large quantities of both fused and unfused BGH related proteins which will coaggregate and can be purified easily. Three embodiments of this mode of the invention are described in the subsections below.

### 5.7.1. CO-TRANSFORMATION OF HOST CELLS

Any appropriate host cell can be co-transformed with two or more plasmids. One plasmid carries the desired unfused protein gene and one plasmid carries a fusion protein gene. Each gene should be under the control of a promoter and translational control elements. The same or different promoter systems may be used to control expression of each gene. If a different promoter is used for each gene sequence then the ratio of the expression of the sequences may be controlled by varying the degree of induction of each promoter.

In addition, the two plasmids may carry different selectable drug markers for antibiotic resistance (e.g., tetracycline and ampicillin resistance) and may be maintained within the same cell if grown in the presence of the two antibiotics. With both plasmids in the cell, expression of the fusion protein and the labile unfused protein can occur.

These transformed cells produce insoluble aggregates which contain both the unfused proteins and the large fusion protein. There is no requirement that the fusion protein be a BGH fusion protein. In fact, any combination of fusion protein with unfused protein will have the aggregation and stabilization properties.

A significant stabilization of the unfused BGH protein was observed when two plasmids were used to transform one host cell, each plasmid directing the expression of its respective protein: unfused BGH and

porcine parvovirus/ß-galactosidase fusion protein (PPV/ß-galactosidase). Pulse-chase experiments indicated that the half-life of the unfused modified-BGH proteins had increased from a 1-2 minute level to between 30 minutes and 1 hour. Additionally, when these proteins were highly expressed within the cell, they form insoluble aggregates which contain both the unfused BGH protein and the large PPV/ß-galactosidase fusion protein. Subsequent purification of the aggregate proteins indicated that the protein could be readily obtained in gram quantities.

### 5.7.2. TRANSFORMATION OF HOST CELLS WITH ONE PLASMID CARRYING TWO GENES

In one embodiment of the present invention, plasmids which carry both the unfused protein gene and the larger fusion protein gene on a single vector are designed and constructed. As described supra for the co-infection scheme, this embodiment of the invention is suitable for any combination of fusion protein and any unfused protein.

The plasmids are constructed such that each gene on the plasmid has its own promoter and expression control elements. The same or different promoter systems may be used to control expression of each gene, therefore this embodiment of the present invention also allows for the regulation of the ratio of the fusion protein and the desired gene product.

### 5.7.3. MODIFICATION OF THE FUSION PROTEIN GENE

In an alternate embodiment of the present invention, a recombinant plasmid which encodes a BGH fusion protein is altered at the junction of the two gene sequences which comprise the fusion protein gene. A chain termination sequence such as amber (TAG), ochre (TAA), or opal (TGA) is located between the two gene sequences; the chain terminator must be in phase with the translational reading frames of both gene sequences. Such an alteration may be accomplished by cleaving the plasmid at a restriction site (or sites) located between the two gene sequences and then ligating a nucleotide linker sequence encoding a chain terminator such as amber, ochre, or opal into the cleaved site on the plasmid so that the chain terminator is in phase with the translational reading frames of both gene sequences.

Introduction of these amber, ochre, or opal modified plasmids into a host cell containing the appropriate tRNA suppressors results in the synthesis of both an unfused protein as well as a fusion protein (since suppression is significantly less than 100%). A tRNA suppressor is a tRNA that has undergone a mutation that allows the tRNA to recognize the termination codon and results in the occasional but not regular insertion of an amino acid at the site of the termination codon. Therefore, host cells carrying the suppressor tRNA characteristically produce both the unfused protein and a fusion protein of normal length. Every nonsense or termination suppressor has a characteristic efficiency, indicated by the fraction of protein chains that are completed.

There are at least two ways to introduce the amber, ochre or opal modified plasmids into a suppressor cell background: (1) the transformant (i.e., a host cell transformed with amber, ochre or opal modified plasmid) can be infected with a lysogenic transducing phage that carries the appropriate suppressor tRNA gene (e.g., Ø80 pSU3 carries the SupF suppressor of amber mutations); or (2) the amber, ochre or opal modified plasmids can be used to transform cell lines which contain suppressor tRNAs of amber, ochre, or opal respectively. Examples of such strains include but are not limited to LE392 (containing supE and supF suppressors of amber mutations), YMC (containing supF), and C600 (supE). The various amber suppressor tRNA genes in E. coli include but are not limited to: supB, supC, supD, supE, supF, supG, supL, supM, supN, supO, supP, supU, supV; the various ochre suppressor tRNA genes in E. coli include but are not limited to: supB, supC, supG, supL, supM, supN, supO, supV; and the various opal suppressor tRNA genes in E. coli incude but are not limited to: supK (see Bachmann and Low, 1980, Microbiological Reviews 44(1): 1-56).

The host cells containing the appropriate suppressor tRNA gene are transformed with the amber, ochre, or opal modified plasmids and can produce the protein as both a fusion protein and as an unfused protein (the proportions of fused to unfused protein produced depends upon the extent of suppression in the host cell).

The amber, ochre, or opal modified plasmids may be further modified to ensure that translation of the corresponding mRNA transcript will terminate at the 3'- terminus of the fusion protein gene. Proper chain termination is important because it contributes to the overall efficiency of translation. A number of approaches which may be used to effect chain termination are described below. In one embodiment, all three chain termination sequences may be inserted in tandem at the 3'- region of the fusion protein gene so that they are in phase with the translational reading frame of the gene sequence. The presence of the chain terminator sequences in tandem will reduce the chance of read-through, consequently, translation will terminate at the chain termination codons on the mRNA transcript.

Alternatively, one or more chain termination sequences of the appropriate type may be inserted into the 3'- region of the fusion protein gene. For example, an amber modified plasmid may be further modified by the insertion (in phase) of one or more opal or ochre sequences at the 3'- end of the fusion protein gene. When this plasmid is inserted into a host cell containing an amber tRNA suppressor (such as supD, supE, supF, supP, supU, supV in _E. coli_) that does not suppress ochre or opal, the host cell will produce both the fusion protein and the unfused protein and translation of the fusion protein will terminate at the location of the opal and/or ochre codons located at the 3'- end of the mRNA transcript.

In a similar fashion, an opal modified plasmid may be further modified by the insertion (in phase) of one or more amber or ochre sequences at the 3'- end of the fusion protein gene. When this plasmid is inserted into a host cell containing an opal tRNA suppressor (such as supK in _E. coli_) that does not suppress amber or ochre, the host cell will produce both the fusion protein and the unfused protein and translation of the fusion protein will terminate at the location of the amber and/or ochre codons located at the 3'- end of the mRNA transcript.

Similarly, an ochre modified plasmid may be further modified by the insertion (in phase) of one or more opal sequences at the 3'- end of the fusion protein gene. When this plasmid is inserted into a host cell containing an ochre tRNA suppressor (such as supB, supC, supG, supL, supM, supN, supO, supV in _E. coli_) that does not suppress opal, the host cell will produce both the fusion protein and the unfused protein and translation of the fusion protein will terminate at the location of the opal codon (or codons) located at the 3'- end of the mRNA transcript.

## 5.8. INSERTION OF BGH SEQUENCES INTO HIGH COPY NUMBER VECTORS

The high copy number (ori) vector pOP1Δ6, (described supra in Sec. 2.1.3) can be used in order to increase the expression levels of the BGH gene by amplification of the number of plasmid (and therefore gene) copies, thereby increasing the BGH mRNA and hence the BGH protein levels within the cell. These plasmids are referred to as ori, high copy number, or runaway plasmids.

When the SFE-met-BGH construction p217-1, was inserted into the runaway plasmid vector, pORI-tet#1 (a tetracycline resistant derivative of the ampicillin resistant pOP1Δ6), plasmid copy numbers were determined to exist at levels of approximately 200-300 copies per cell as compared to 20-40 copies per cell for p217-1. Expression levels of BGH were determined by SDS-PAGE to range from 10% to 18% of the total bacterial cell protein. BGH protein in these constructions was found to be extremely stable (i.e., half-life greater than one hour) within the cell, probably due to the production of insoluble aggregate forming proteins.

## 6. EXAMPLE: Bovine Growth Hormone

According to this embodiment of the present invention the BGH mRNA was isolated and identified and a cDNA was synthesized using the mRNA as a template. The cDNA fragments of the BGH gene were then inserted into a pBR322 vector to form several recombinant plasmids. These plasmids were then used to transform E. coli bacterial hosts. Those clones positive for the insert were then identified. The plasmid containing the BGH gene was identified using two techniques (not necessarily in the order listed): (a) DNA sequencing and (b) BGH specific mRNA hybridization experiments.

After the BGH gene was identified, it was excised from the non-expression plasmid. To increase expression efficiency, a synthetic amino terminus was inserted in place of the natural amino terminus. This BGH gene with the synthetic front end (SFE-met-BGH) was then inserted into an expression plasmid pJS413 or pDN612.

To decrease the proteolytic destruction of the newly synthesized BGH by the host cell and thus increase the yield of gene product, the BGH expression plasmid was co-infected into a host along with an expression plasmid encoding the fusion protein PPV/ß-galactosidase. Alternatively, host cells were transformed with one expression plasmid encoding both the SFE-met-BGH and PPV/ß-galactosidase. The co-expression of BGH and fusion protein decreased the rate of proteolytic degradation and simplified purification of the BGH protein.

## 6.1. GENERAL PROCEDURES USED FOR PREPARATION OF THE PLASMIDS

The following subsections describe the general procedures and materials used for DNA isolation, enzyme reactions and ligation reactions.

## 6.1.1. PLASMID DNA ISOLATION

Host cell bacteria *Escherichia coli*, (*E. coli*) were transformed (Kushner, p. 17 in Genetic Engineering, ed. H.B. Boyer and S. Nicosia, Elsevier/North- Holland, Amsterdam, 1978), and large (microgram) quantities of plasmid DNA were isolated from cultures of *E. coli* transformants grown in LB-medium (Maniatis *et al*., p. 88 in Molecular Cloning: A Laboratory Manual, Cold Spring Harbor Press, New York, N.Y., 1983). Plasmids were isolated from cells in late logarithmic stage of growth using a modification of the method of Godson and Vapnek (1973, Biochim. Biophys. Acta. 229:516).

## 6.1.2. CONDITIONS FOR RESTRICTION ENZYME DIGESTIONS

Restriction enzymes used in the present application were obtained from New England Biolabs, Inc., Beverly, MA, unless otherwise indicated. An enzyme unit is defined as the amount required to digest 1.0 µg of lambda DNA in 60 minutes at 37°C in a total reaction mixture of 50 µl.

All restriction enzyme total digestions were accomplished under the following conditions: each 1 µg DNA was incubated with 2-5 units of enzyme at 37°C for 60 minutes in 20 µl buffer. Partial digestions were accomplished by modifying the conditions used for total digestion as follows: each 1 µg DNA was incubated with 0.1 units of enzyme at 37°C for 15 minutes. Reactions were terminated by the addition of 0.1% sodium dodecyl sulfate (SDS). Thus, the reaction conditions were adjusted to obtain an average of one cleavage per DNA molecule.

## 6.1.3. RESTRICTION ENZYME BUFFERS

The buffer used for HpaI or SmaI digestions consisted of: 20mM KCl, 10mM Tris-HCl (pH 7.4), 10mM $MgCl_2$ and 1mM DTT.

The buffer used for AhaIII, AvaI, BamHI, BglII, ClaI, EcoRI, EcoRV, HaeII,, HaeIII, HincII, HindIII, HinfI, NarI, NcoI, PstI, PvuII, RsaI, SacI, SalI, Sau3A 1, TaqI, XbaI or XmnI digestions consisted of: 50mM NaCl, 10mM Tris-HCl (pH 7.4), 10mM $MgCl_2$ and 1mM DTT.

It should be noted that whenever two or more restriction endonuclease reactions are performed on DNA, the reaction in low salt buffer is accomplished before the reaction in high salt buffer. If two enzymes require the same buffer, then the reactions may be performed simultaneously.

## 6.1.4. MODIFICATION OF DNA

Bal 31 nuclease is a multifunctional enzyme that contains a highly specific single-stranded endodeoxyribonuclease activity and a processive exonuclease activity that simultaneously degrades both the 3'- and 5'- termini of double-stranded DNA (dsDNA). One unit of nuclease Bal 31 (New England Biolabs, Inc. Beverly, MA) is defined as the amount required to release 1.0 µg of acid soluble nucleotides from denatured calf thymus DNA (650 µg/mℓ) in one minute at 30°C. The reaction buffer used for Bal 31 consisted of: 20mM Tris-HCl (pH 8.0), 600mM NaCl, 12mM $CaCl_2$, 12mM $MgCl_2$, 1.0mM EDTA and DNA. Incubations were at 30°C; Bal 31 digests were accomplished by incubating 30 µg of DNA with 0.5 units Bal 31 for 1 to 10 minutes.

Reactions were terminated by the addition of EDTA to 50mM or heat inactivation of Bal 31 (i.e., 65°C for 10 minutes).

The enzyme terminal deoxynucleotidyl transferase (TdT) catalyzes the polymerization of deoxynucleoside triphosphates at the 3'- termini of single-stranded DNA or oligodeoxynucleotides. By using cobalt ion instead of magnesium ion as cofactor, the specificity of the enzyme is changed so that it can be used for terminal labeling and addition of homopolymer tails to duplex DNA fragments. Such incorporation can be made at the 3' OH-terminus of duplex DNA with either base paired or staggered ends. The homopolymeric tail may be composed of any one of the four nucleotides as the repeating unit: dCTP, dGTP, dATP or dTTP. One unit of activity of TdT (Bethesda Research Laboratories, Gaithersberg, MD) is defined as the amount of enzyme that incorporates 1nmol dATP into acid precipitable material in one hour at 37°C, using $d(pA)_{50}$ as primer.

S1 nuclease and mung-bean nuclease degrade RNA or denatured DNA (i.e., single-stranded DNA) into mononucleotides, but will not (under proper conditions) degrade double-stranded DNA or DNA/RNA hybrids. One unit of S1 nuclease (Boehringer/Mannheim, Indianapolis, Ind.) is defined as the amount of enzyme required to acid solubilize 1 µg of denatured calf thymus DNA in 30 minutes at 37°C. The reaction buffer used for S1 nuclease consisted of 25mM sodium acetate (pH4.6) 100mM NaCl, 0.1mM $ZnSO_4$ and 5% glycerol. S1 digestions were accomplished by incubating 2000 units of enzyme with 0.1 µg of DNA at 37°C for 30 minutes. One unit of mung-bean nuclease (P-L Biochemicals, Milwaukee, Wis.) is defined as that amount of enzyme which produces 1 µg of acid soluble material per minute at 37°C. The reaction buffer for mung-bean nuclease consisted of 30mM NaAc (pH5.0) 200mM NaCl, 5% glycerol and 1mM $ZnSO_4$. Mung-bean digestions were accomplished by incubating 20 units of enzyme with 1.0 µg DNA at 37°C for 30 minutes.

### 6.1.5. DNA POLYMERASE REACTION

DNA polymerases catalyze the stepwise elongation of a DNA chain. Chain growth is in the 5' to 3' direction (*i.e.*, addition of nucleotides occurs at the 3'-terminus) and the sequence of the polymer is determined by that of the template because the incoming nucleotide must be complementary to the template, *i.e.*, form the correct base pair with the template strand. The known DNA polymerases cannot initiate chain synthesis, thus DNA synthesis requires a primer strand with a free 3'-hydroxyl terminus annealed to a template strand. Chain elongation proceeds by the nucleophilic attack of the 3'-hydroxyl terminus of the primer on the 5'-phosphate of the incoming nucleotide. The new chain is base paired and antiparallel to the template strand. As a result of the DNA polymerase reaction the single-stranded template strand is "filled-in" or converted to a double-stranded DNA molecule.

A second important feature of the DNA polymerases is the "proof-reading" function. A 3' to 5' exonuclease activity associated with DNA polymerase I acts on nonbase-paired termini and can degrade both single- and double-stranded DNA in the 3' to 5' direction yielding mononucleotides. Thus an incorrectly added nucleotide is removed before polymerization continues, and the fidelity of the enzyme is further enhanced. DNA polymerase I also has a 5' to 3' exonuclease activity specific for double-stranded DNA (yielding 5'- mononucleotides and oligonucleotides) which can also excise mismatched regions in DNA.

Proteolytic treatment of DNA polymerase I by the method of Klenow (Klenow et al., 1971, Eur. J. Biochem. 22: 371) yields two fragments, large and small. The large fragment or Klenow fragment (76,000 daltons) retains the polymerase and 3'- 5' exonuclease activity whereas the small fragment retains the 5'- 3' exonuclease activity. One unit of DNA polymerase I or DNA polymerase I-large fragment (New England Biolabs, Beverly, MA) is defined as the amount converting 10 nmoles of deoxyribonucleotides to an acid insoluble form in 30 minutes at 37°C. The assay conditions for both enzymes are the same except that the reaction mixture for DNA polymerase I contains 67mM $KPO_4$ (pH 7.5) while the reaction mixture for the Klenow fragment contains 40mM $KPO_4$ (pH. 7.5) in the following buffer: 6.6mM $MgCl_2$, 1.0mM ß-ME, 2mM dAT copolymer, 33µM dATP, 33µM $^3$H-dTTP and enzyme.

In the present application, when DNA polymerase large (Klenow) fragment was used to fill in single-stranded DNA or cohesive ends that result from restriction enzyme cleavage, the following procedure was employed: restriction enzyme reactions were terminated by heating at 68°C for 10 minutes. To the same reaction mixture a final concentration of 50µM of each deoxynucleoside triphosphate was added and for each microgram of DNA in the reaction mixture 10-100 units of DNA polymerase Klenow fragment was added. In other words an excess of DNA polymerase Klenow fragment was used to ensure that single-stranded ends were completely filled in.

### 6.1.6. GEL PURIFICATION OF DNA FRAGMENTS

After restriction enzyme or nuclease treatment, DNA fragments of varying sizes were separated by gel electrophoresis in either agarose or polyacrylamide slab gels at low voltage (approximately 2 volts/cm for agarose gels and 10 volts/cm for polyacrylamide gels), stained with ethidium bromide and visualized under ultraviolet light (Southern, 1979, Methods in Enzymology 68: 152).

In order to recover particular DNA fragments from gels, the appropriate bands were excised from the gel and the DNA was electroeluted into dialysis tubing. The DNA was then isolated on DEAE-cellulose, or ethanol precipitated, and resuspended in the appropriate buffer (Smith, 1980, Methods in Enzymology 65: 371).

Alternatively, DNA restriction digests were separated by low-melting point agarose (LMP agarose) gel electrophoresis (Bethesda Research Laboratories, Inc., Gaithersburg, MD). After electrophoresis the particular DNA fragment of interest was excised from the gel and the DNA was separated from the LMP agarose by heating at 65°C, followed by phenol extraction, ethanol precipitation, and resuspension in the appropriate buffer (Weislander, 1979, Anal. Biochem. 98:305).

### 6.1.7. DNA LIGATION

All ligations were accomplished using T4 DNA ligase (New England Biolabs, Inc., Beverly, MA). One unit of T4 DNA ligase is defined as the amount required to yield 50% ligation of *Hin*dIII fragments of bacteriophage lambda DNA in 30 minutes at 16°C in 20 µℓ of ligase buffer at a 5'- DNA termini concentration of 0.12µM (300 µg/mℓ).

DNA ligations were carried out in ligase buffer consisting of: 60mM Tris-HCl (pH 7.8), 10mM $MgCl_2$, 20mM dithiothreitol(DTT), 1.0mM ATP and a DNA concentration ranging from 15-50 μg/mℓ. Ligation reactions were incubated 4 to 24 hours at room temperature using approximately 300 units of T4 DNA ligase per 10 μℓ reaction volume.

### 6.1.8. IDENTIFICATION OF BGH PROTEINS BY IMMUNOPRECIPITATION WITH ANTI-BGH SERA

Cultures of clones were grown overnight in AF media (Krzyzek, R., and Rogers, P., 1972, J. Bacteriol. 110:945-954) with 1% glucose and without added methionine at 30°C. Aliquots (0.4 mℓ) were removed, washed once in AF medium with 1% glucose without added methionine and resuspended in 1.0 mℓ of the above medium. After incubation at 30°C for 30 minutes, IPTG was added to 1mM. The cultures were further incubated for 30 minutes and then [$^{35}S$]-methionine (1440 Ci/mmol) was added to a concentration of 50 μCi/mℓ for 1.5 minutes. Pulse labeling was terminated by placing the cultures in an ice water bath. Bacteria were collected by centrifugation and resuspended in 50 μℓ of solution containing 25% sucrose and 0.05M Tris-HCl (pH 8.0). A 50 μℓ solution containing 0.02M EDTA (pH 8.0) and 500 μg lysozyme was added. The cell suspension was then incubated at 4°C for 10 minutes, 400 μℓ of TET buffer [0.1M Tris-HCl (pH 8.0), 0.05M EDTA, and 2% Triton X-100 (polyoxyethylene (9-10) p-*tert*-octylphenol)] was then added for 5 minutes and lysis was completed by the addition of 500 μℓ of RIPA buffer [0.01M Tris-HCl (pH 7.5), 0.15M NaCl, 1% deoxycholate, 1% Triton X-100 and 0.1% SDS]. Formaldehyde-fixed *Staphylococcus* cells (100 μl) were added (Pansorbin Staphylococcus Aureus Cells, Calbiochem, La Jolla, CA). The cell lysate was incubated at 4°C for an additional 5 minutes and then centrifuged. The supernatant was removed and incubated with 10 μℓ of anti-BGH sera for 30 minutes at 4°C. Formaldehyde-fixed Staphylococcus cells (200 μℓ) were added, and after 10 minutes the immune complex was collected by centrifugation (Kessler, S.W., 1976, J. Immunol., 177:1482-1490). The immune complex was then washed and analyzed by SDS-PAGE (Krzyzek, R.A., et al., 1980, J. Virol., 36:805-815).

## 6.2. ISOLATION AND MOLECULAR CLONING OF THE BGH GENE

The following subsections describe the molecular cloning of BGH cDNA. A recombinant plasmid, designated pBH27 contained the entire BGH coding sequence.

### 6.2.1. ISOLATION OF mRNA

Messenger RNA was extracted in several steps. Bovine pituitaries were disrupted with a VirTis tissue homogenizer (VirTis Company, Gardiner, N.Y.) in a solution containing 4M guanidinium thiocyanate, 0.5% sodium N-lauroyl-sarcosine, 0.1M β-mercaptoethanol, 0.1% of 30% antifoam A (Sigma, St. Louis, Missouri), and 0.025M sodium citrate (pH 7.0). Cellular debris was removed by centrifugation in a Sorvall HB-4 rotor at 10,000 X g for 10 minutes at 4°C and the supernatant was layered onto a 5.7M CsCl cushion buffered with 0.025M sodium acetate (pH 5.0) and containing 0.2% diethyl pyrocarbonate. (Chirgwin, J.M. et al., 1979, Biochem., 18:5294-5299). The RNA was pelleted by centrifugation in a SW28 rotor at 90,000 X g for 16 hours at 15°C and the resultant pellet was resuspended in a solution containing 0.05M sodium acetate (pH 5.1), 0.01M ethylenediaminetetraacetic acid (EDTA), and 1% sodium dodecylsulfate (SDS), and then extracted at 56°C with an equal volume of phenol saturated with the above buffer. The RNA was precipitated twice in the presence of 0.2M sodium acetate with 2 volumes of ethanol, collected by centrifugation at 13,000 X g for 30 minutes, and suspended in TE buffer [0.01M Tris-HCl (pH 7.4)-0.01M EDTA].

Following the extraction of RNA from bovine pituitary glands, poly(A)-containing RNA was isolated from the mixture of RNAs extracted. Accordingly, an aliquot of bovine pituitary RNA was adjusted to 0.5M NaCl and 0.5% SDS and applied to an oligo(dT)-cellulose column. (Miller, W.L. and McCarthy, B.J., 1979, J. Biol. Chem. 254:742-748). The column was washed extensively with loading buffer [0.01M Tris-HCl (pH 7.5), 0.5M NaCl, 0.001M EDTA, and 0.5% SDS] and the poly(A)-containing RNA was eluted with buffer containing 0.01M Tris-HCl (pH 7.5) and 0.5% SDS and concentrated by ethanol precipitation.

### 6.2.2. PREPARATION OF cDNA FROM BGH mRNA

Following the preparation of poly(A)-containing RNA, double-stranded complementary DNA (cDNA) was prepared for cloning.

The poly(A) RNA was enzymatically converted to cDNA using the enzyme RNA-dependent DNA polymerase (reverse transcriptase). Reverse transcriptase is used chiefly to transcribe RNA into DNA, which can then be inserted into procaryotic vectors. The primer for this reaction can be oligo(dT) or a collection of randomly generated oligodeoxynucleotides. If randomly generated oligodeoxynucleotides are used as primers, the diversity of these molecules must be so large as to guarantee that some of them will be complementary to sequences in the template nucleic acid. Because different oligonucleotides bind to different sequences in the template, all parts of the template are represented in the resulting DNA at equal frequency. Furthermore, random oligonucleotides can be used as primers on any single-stranded RNA template. By contrast, oligo(dT) binds only to poly(A) located at the 3' end of the RNA molecule, and therefore primes the synthesis of DNA that is heavily biased toward sequences at the 3' end of the mRNA template.

Treatment of the RNA with reverse transcriptase results in a RNA/cDNA hybrid which must be separated (or the RNA strand destroyed) in order to retrieve the single-stranded cDNA molecule representing the gene to be cloned. The separation or RNA destruction may be accomplished by treatment with hot alkali or with ribonuclease which will solubilize or digest the RNA component; the resulting cDNA is a single-stranded DNA molecule which is then isolated from the reaction mixture using standard techniques such as rate zonal sedimentation, electrophoresis, chromatography, etc.

Single-stranded cDNA was prepared in several steps. Twenty micrograms of polyadenylated bovine pituitary RNA was reverse-transcribed into single-stranded complementary DNA in a 200 µℓ reaction containing 4 µg of oligo (dT) 12-18 primer, 0.05M Tris-HCl (pH 8.0), 0.008M $MgCl_2$, 0.028M KCl, 0.004M sodium pyrophosphate, 0.001M ß-mercaptoethanol (ß-ME), 200µM each of dGTP, dCTP, dTTP and dATP, and 40 units AMV reverse transcriptase. One unit of AMV reverse transcriptase (Bethesda Research Laboratories, Inc., Gaithersburg, MD) is defined as the amount of enzyme which incorporates one pmol of deoxythymidine-5'-triphosphate into acid-precipitable product in 1 minute at 37°C using poly(rA) • $oligo(dT)_{12-18}$ as the template primer. After incubation at 37°C for 60 minutes, EDTA was added to 0.02M, the mixture was then heated for 1 minute at 100°C, treated with pancreatic ribonuclease (40 μg/mℓ) for 30 minutes at 37°C, and extracted with phenol three times. The single-stranded cDNA was then purified by Sepharose 4B chromatography and concentrated by ethanol precipitation.

After the preparation of single-stranded cDNA, double-stranded cDNA was obtained. This was done by treating the cDNA species with E. coli DNA polymerase I. The reaction was carried out at pH 6.9 to minimize the 5' to 3' exonuclease activity of DNA polymerase I and at 15°C to minimize the possibility of synthesizing "snap back" DNA. The Klenow fragment of DNA polymerase I has also been successfully employed to synthesize the second cDNA strand. Reverse transcriptase may also be utilized to synthesize the second cDNA strand using conditions similar to those already described for the synthesis of the first cDNA strand. Both DNA polymerase I and reverse transcriptase may be used together in the conversion of single-stranded to double-stranded DNA.

After synthesis of cDNA was complete, the first and second strands were still covalently joined by the hairpin loop that was used to prime the second strand synthesis. This loop is susceptible to cleavage by the single-strand-specific nuclease, S1. However, the resulting termini are not always perfectly blunt-ended, and the efficiency of cloning is improved if they are filled in with the Klenow fragment of E. coli DNA polymerase I. Alternatively, mung-bean nuclease may be used to cleave the two strands. Although mung-bean nuclease and nuclease S1 are similar to one another in their physical and catalytic properties, there are indications that mung-bean nuclease may be less severe in its action than nuclease S1.

Second-strand synthesis was performed in a 40 μℓ reaction solution containing 200-500 ng of single-stranded cDNA, E. coli DNA polymerase I (Klenow fragment, 7 units), 300μM each of dGTP, dCTP, dTTP and dATP, 0.01M $MgCl_2$, 0.1M KCl and 0.02M HEPES (pH 6.9) and incubated for 18 hours at 15°C. Five volumes of solution containing 0.03M sodium acetate (pH 4.5), 0.3M NaCl, 0.003M $ZnCl_2$ and mung bean nuclease (1 unit) was added to digest single-stranded hairpins. The mixture was incubated for 30 minutes at room temperature, heated to 65°C for 5 minutes, and extracted three times with phenol. Double-stranded DNA was purified by Sepharose 4B chromatography and concentrated by ethanol precipitation.

### 6.2.3. CLONING OF BGH cDNA

After preparation of the BGH double-stranded cDNA, the cDNA was prepared for insertion into a suitable vector. This was accomplished by using the enzyme terminal deoxynucleotidyl transferase (TdT) to attach homopolymer tails to the double stranded cDNA molecule. The tailed BGH cDNA was then inserted into a cloning vector that has been cleaved at a unique restriction site and tailed with a homopolymer that is complementary to the 3' tails of the cDNA molecule. In the present invention, the cDNA was poly(dC)-tailed. The circular plasmid pBR322 was then cut with the restriction enzyme PstI (a unique site on the plasmid) and (dG)-tailed as described above. At least 10 dGMP nucleotides were added to each 3' end of the double-stranded plasmid DNA.

The (dC)-tailed BGH cDNA was allowed to anneal to the *PstI* cleaved, (dG)-tailed plasmid DNA to form a recircularized recombinant plasmid containing the BGH cDNA insert. The recombinant molecules were used to transform appropriate host cells. The recombinant molecules were then isolated from transformants, analyzed by restriction endonuclease digestion and gel electrophoresis in order to map or characterize the inserted BGH DNA sequence. Details are described below.

Single-stranded tracts of homopolymeric deoxycytidine were added to the 3'- ends of the double-stranded cDNA in a 50 µℓ reaction containing 10 to 100 ng of double-stranded cDNA, 0.1M sodium cacodylate (pH 7.2), 0.006M $CoCl_2$, 0.002M dCTP and 20 units TdT. The mixture was incubated for 1 minute at 37°C and the reaction was terminated by the addition of EDTA to 0.02M and heating at 65°C for 3 minutes. Plasmid pBR322 was cut with *PstI* and 500 ng was similarly "tailed" with deoxyguanosine by incubation at 37°C for 5 minutes. In order to anneal the cDNA to the plasmid, both reaction mixtures were combined, heated to 65°C for 5 minutes, incubated for 2 hours at 42°C, and then allowed to cool slowly to room temperature overnight.

*E. coli* K-12 MC1000 (2 mℓ of cells in the logarithmic phase of growth) was rendered permeable to the annealed DNA mixture by incubation in 0.1M MOPS (morpholinopropane sulfonic acid, pH 6.5), 0.05M $CaCl_2$, and 0.01M $RbCl_2$ for 30 minutes at 4°C; then 3 µℓ dimethylsulfoxide (DMSO) and 1-200 ng of annealed DNA in a volume of 10 µℓ or less of TE (10mm Tris-HCl, 1mm EDTA) pH 8.0 was added and incubated for 30 minutes at 4°C. The mixture was warmed at 42°C for 1 minute, mixed with 10 volumes of L-broth, incubated for 1 hour at 37°C without shaking, and finally plated onto L-agar containing 20 µg/µℓ tetracycline. Tetracycline resistant, ampicillin sensitive transformants containing BGH DNA were identified by in situ hybridization using [$^{125}$I] labeled pituitary poly(A)-containing RNA as a probe (Soreq, H., et al., 1979, Nucleic Acids Res., 6:2471-2481). The plasmid designated pBH27 which was isolated from one clone was found by restriction enzyme analysis to contain a large BGH cDNA insert (FIG. 1). The BGH DNA insert of the pBH27 plasmid was sequenced by the method of Maxam and Gilbert (1980, Methods in Enzymology, 65:499-580). Sequencing data (FIG. 2) indicate that the BGH DNA insert is 795 nucleotides in length and, more importantly, contains the entire coding sequence for pre-BGH as well as additional nucleotides corresponding to the 5' and 3' untranslated regions of BGH poly(A)-containing RNA.

The nucleotide sequence of BGH mRNA was reported by Miller et al. (1980, J. Biol. Chem., 225:7521-7524). The differences between the reported mRNA sequence and the pBH27 sequence are indicated in FIG. 2. The nucleotides of the published mRNA sequence that differ from those of pBH27 are indicated above the pBH27 sequence; and nucleotides present in the pBH27 sequence but not found in the published sequence are indicated above the pBH27 sequence by a single asterisk (*).

The nucleotide changes described above in the coding region of the mature BGH are all silent changes. These differences may represent allelic variability and/or are changes associated with the infidelity of reverse transcription.

Within the text that follows, DNA fragments are identified according to their length in base pairs (b.p.)

and according to their termini generated by restriction enzymes. All DNA fragment sizes which are represented in terms of base pairs are determined from DNA sequence data (FIG. 1). If a DNA fragment has a single-stranded cohesive end, the b.p. number indicated for that fragment includes the nucleotides comprising the single-stranded cohesive end. The termini of each DNA fragment containing BGH sequences are indicated on either side of a slash in the order of the direction of transcription of the BGH DNA sequence. For example, a 907 b.p. HindIII/BglII DNA fragment signifies a DNA fragment which has a HindIII cohesive end at its amino coding terminus, a BglII cohesive end at its carboxy-coding terminus, and is 907 nucleotides in length.

### 6.3. CLONING AND EXPRESSION OF BGH DNA SEQUENCES

Portions of the BGH DNA of pBH27 were subcloned into expression vectors so that the BGH DNA was placed under the control of various promoters. Some of the plasmids described below are described in U.S. application Serial No. 449,187 by J. Salstrom *et al.*, filed December 13, 1982, which is herein incorporated by reference.

#### 6.3.1. CLONING AND EXPRESSION OF BGH IN pJS413

The expression vector, pJS413 (FIG. 3), is a pBR322 derivative which contains the $amp^r$ (ß-lactamase) gene, a lac promoter (Placuv5), lac and cro ribosome binding sites ($SD^{lacZ}$ and $SD^{cro}$ are represented in all figures as $SD^z$ and $SD^{cro}$, respectively), a chain initiation ATG with 65 nucleotides of cro (cro), and a modified ß-galactosidase gene (the lac i-z gene, hereinafter referred to as the z-gene). The plasmid has unique cloning sites which span the cro-z junction:

BglII, SmaI, and BamHI. The z-gene is not in the same translational reading frame with the cro ATG, thus the intact plasmid does not direct the production of ß-galactosidase. However, when a DNA fragment of the appropriate length is inserted into any one of the cloning sites in this plasmid (3n+1), the reading frame of the z-gene may be readjusted with respect to the cro ATG. If the inserted DNA sequence contains no termination signals (e.g., TGA, TAA, or TAG) that are in phase with the cro ATG or z-gene, a ß-galactosidase fusion protein will be produced by host cell transformants.

Portions of the BGH DNA sequence from pBH27 were inserted into the expression vector pJS413 according to the following procedure. A Sau3A fragment (approximately 798 b.p.) which encodes approximately 280 b.p. of pBR322 and two-thirds of BGH (the amino terminus) were isolated from the cDNA clone pBH27 (see FIG. 3). The Sau3A fragment was then digested with SmaI which generated a small 10 b.p. fragment from the BGH 3'-end and a 785 b.p. Sau3A/SmaI fragment; the 785 b.p. fragment contains approximately 505 b.p. of the BGH DNA sequence. The expression vector, pJS413 (see FIG. 3) was digested with SmaI and BglII to generate a SmaI/BglII backbone. The 785 b.p. Sau3A/SmaI pBH27 fragment was ligated to the SmaI/BglII pJS413 backbone. (N.B. the Sau3A cohesive ends are complementary to the BglII cohesive ends.) This vector was used to transform E. coli MC1000 and plated on L-agar containing 100 μg/mℓ of ampicillin.

Ampicillin resistant transformants containing the BGH DNA were identified by in situ hybridization using a radiolabeled PvuII-PvuII fragment of pBH27. One clone which contained the BGH insert was amplified and plasmid DNA was purified. This plasmid was designated p3-30/IM.

FIG. 3 shows the construction of p3-30/IM containing approximately 280 b.p. of pBR322 and 505 b.p. encoding the amino terminal portion of BGH.

In order to remove the pBR322 portion of the inserted DNA, p3-30/IM was linearized with BglII and digested with Bal 31 for varying times (see FIG. 3). A number of linearized p3-30/IM molecules of varying lengths were generated. These were recircularized by ligation in the presence of DNA linkers which encode the BglII restriction endonuclease recognition sequence (5'-CAGATCTG-3') which resulted in a population of p3-30/IM plasmids of different sizes wherein the BglII site was restored. The ligation mixture was used to transform E. coli MC1000 and the plasmids were amplified and DNA purified. The isolated plasmids were digested with SmaI and BglII; DNA fragments of approximately 350 to 500 b.p. encoding BGH sequences were isolated by agarose gel electrophoresis (see FIG. 4).

These BglII/SmaI BGH fragments were ligated into pJS413 which had been linearized by cleavage with SmaI and BglII (FIG. 4). The ligation mixture was used to transform E. coli MC1000. Cells were plated on MacConkey agar and red colonies were isolated (Apte, B.W., et al., 1975, Nature, 257:329-331). The red colonies were screened for BGH DNA by in situ hybridization (Grunstein, M. and Hogness, D.S., 1975, Proc. Natl. Acad. Sci., 72:3961-3965) and then assayed for the expression of BGH/ß-galactosidase fusion proteins by an in situ immunoassay (Kemp, D.J. and Cowman, A.F. 1981, Proc. Natl. Acad. Sci. 78:4520-4524).

Plasmid DNA isolated from several expressing clones was cleaved with BglII/SmaI and analyzed by agarose or polyacrylamide gel electrophoresis to determine the size of the BGH DNA inserts. The BglII/SmaI DNA fragment of one clone, pRed13 (FIG. 4), was slightly larger than a DNA sequence that could encode a mature BGH protein. The cro/BGH/β-galactosidase fusion protein expressed by E. coli transformed with pRed13 contained 135 amino acid residues of BGH (i.e., amino acid residue number -3 to 132 in FIG. 2) which included 3 amino acid residues of the BGH leader sequence which are normally not present in the mature BGH protein. Plasmid pRed13 was then employed to generate several BGH DNA constructions as described in the following examples.

### 6.3.2. CLONING AND EXPRESSION OF BGH IN pDN572 MET-PLASMIDS

A series of treatments was devised starting with plasmids pJS413 and pRed13 to obtain the expression of several modified met-BGH proteins. The experimental scheme follows.

The construction of cro deletion derivatives of pJS413 is discussed in detail in U.S. application Serial No. 449,187 (supra). Briefly, one such expression vector, pDN572 is a pJS413 derivative which was prepared as follows: varying size deletions of the cro gene sequences in the plasmid pJS413 were generated by digestion with BglII followed by limited Bal 31 treatment and ligation in the presence of BglII linkers. One of the pJS413 derivatives, designated pDN572, is depicted in FIG. 5. pDN572 includes the cro ATG plus the next 7 nucleotides of cro. As discussed below, pDN572 was used to construct a series of modified met-BGH plasmids.

A BglII/SmaI BGH DNA fragment (approximately 405 b.p.) was isolated from p13EX. This fragment was then ligated to the BglII/SmaI backbone of pDN572 (FIG. 5). Transformed E. coli NF1829 were plated on L-agar containing 100 μg/mℓ of ampicillin. Clones were screened for BGH DNA by in situ hybridization utilizing a radiolabeled PvuII-PvuII fragment of pBH27. Plasmid DNA designated p572-13-1 was amplified and isolated from one clone (see FIG. 5).

In order to remove the BGH leader sequence and cro coding sequence, p572-13-1 was linearized by digestion with BglII and treated with Bal 31 for time periods of 20, 40, and 60 seconds to remove approximately 5 to 20 b.p. in each direction from the BglII site. The DNA was then ligated (the BglII recognition site is now removed) and used to transform E. coli NF1829 which was plated on MacConkey lactose agar plates. β-galactosidase positive colonies were selected and red colonies were isolated.

Plasmids isolated from transformants expressing β-galactosidase were screened by restriction analysis using the restriction enzyme HaeII. Plasmids found to be HaeII(-) (a site at the start of the mature BGH sequence - see FIG. 2) were sequenced by the dideoxy chain termination method (Sanger et al., 1977, Proc. Natl. Acad. Sci. 74:5436-5467).

The carboxyl coding terminus of the BGH was ligated into the met-plasmids in order to reconstruct the BGH gene by inserting a SmaI/EcoRI fragment from pBH27 (approximately 1042 b.p.) into the homologous sites in the met-plasmids (FIG. 6). The expression of the reconstructed BGH by host cells transformed with the modified met-plasmids was determined by immunoprecipitation with anti-BGH sera and SDS-PAGE analysis. Reconstruction of the 3'-carboxy coding terminus of BGH as provided herein results in no fusion protein production by transformants (the i-z gene is removed from the expression plasmids during this procedure). FIG. 7 shows pertinent nucleotide and corresponding amino acid sequences of several HaeII(-) plasmids. The method described above provides a general method for the insertion of the carboxy-terminal coding segment of BGH (wherein the nucleotides encode amino acid residues $arg^{133}$ through $phe^{191}$ as shown in FIG. 2).

One BGH construct designated clone pl-40/C was determined to produce BGH protein at a higher level than any other previous construct (greater than 1%). As shown in FIG. 7, the 5' end of this clone has a number of changes from the mature BGH sequences. It is interesting to note that the first five codons in the pl-40/C sequence are "preferred"-type codons i.e., those which code for the major-isoaccepting species of tRNA's within the E. coli cell.

Plasmid DNA was also isolated from clone pl-40/C which contained the gene sequences that code for the unfused modified BGH protein. This plasmid contains the gene sequences that code for ampicillin resistance. Hence, bacteria which are transformed with this plasmid are resistant to ampicillin and can produce the modified BGH protein.

### 6.3.3. EXPRESSION VECTOR pDN612

The expression vector pDN612 which is used below is a derivative of the expression vector pJS413. This vector was constructed by the removal from pJS413 of those nucleotides which corresponded to the cro gene, including, removal of the cro ATG and the two nucleotides 5'- to the cro ATG. Vector pJS413 was digested with BglII followed by limited Bal 31 treatment and recircularization by ligation in the presence of BglII linkers. All other characteristics of the pJS413 vector are retained within pDN612. The important feature of this construction is that it provides for a unique cloning site 3'- to the cro SD region. Any DNA fragment may be inserted at this site, and when provided with its own ATG, may allow for subsequent expression of the protein for which it codes by transformants.

### 6.3.4. GENERATION OF MET-BGH BY EMPLOYING A SYNTHETIC HINDIII-NCOI LINKER

The strategy for expression cloning described in the following scheme involves inserting BGH DNA into an expression vector such that a met codon is inserted 5'- to the mature BGH gene sequence.

The pUC9 expression vector which contains the lac promoter, $SD^{lac}$, a linker sequence for 10 restriction endonuclease sites, and the amino terminus of the ß-galactosedase gene was digested with PstI to linearize the plasmid (Messing et al., Gene, 1982, 19: 259-268). The linker sequence provides numerous convenient restriction sites 5'- to the BGH sequence. Plasmid pBH27 was partially digested with PstI and the large (795 b.p.) BGH fragment was isolated by agarose gel electrophoresis and ligated with the linearized plasmid pUC9 as described in FIG. 8. The ligation mixture was used to transform E. coli strain NF1829. Ampicillin resistant colonies were amplified and plasmid DNA was isolated. Plasmid DNA was analyzed by restriction digestion and one clone, pBG18C1 contained the entire leader sequence and mature BGH sequence with the exception of the first two codons (ATG ATG) of the leader sequence. pBG18C1 produced a

fusion protein consisting of the first 8 terminals amino codons of ß-galactosidase fused to amino acid -24 (ala) of the BGH leader sequence.

In order to remove the leader sequence, plasmid pBG18C1 was linearized by digestion with NarI. The linearized plasmid was treated with S1 nuclease to remove the 5' overhangs and subsequently digested with HindIII to remove the BGH leader sequence. The plasmid was then ligated in the presence of the synthetic HindIII-NcoI linker which introduces an ATG codon, and the mixture was used to transform E. coli NF1829. Plasmid DNA from one clone, pBG18N was isolated.

Plasmid pBG18N (FIG. 8A) was digested wih NcoI and SmaI and an approximately 370 b.p. fragment containing the amino terminus of the BGH sequence was isolated by agarose gel electrophoresis. The plasmid pDN612 was linearized by digestion with BglII. The linearized plasmid was digested with S1 nuclease to create blunt ends and ligated in the presence of the synthetic NcoI linker. E. coli strain NF1829 was transformed and the plasmid DNA from pJS413-612N was isolated. pJS413-612N was digested with NcoI and SmaI and the backbone was ligated with the NcoI/SmaI fragment isolated from pBG18N. The ligation mixture was used to transform E. coli NF1829 and was plated on MacConkey agar. Plasmids isolated from transformants expressing ß-galactosidase were screened and one plasmid, pJS413-612N met-BGH (FIG. 8A) was found to be expressed as a fusion protein which contained the amino terminus of mature BGH (amino acids 1-132) fused in phase with ß-galactosidase. The carboxy-terminus of the BGH was ligated into the met-plasmid by inserting a SmaI/BamHI fragment from pBH27 (approximately 1417 b.p.) into the homologous sites in the met-BGH plasmid as described supra (Sec. 6.3.1). FIG. 8A shows the steps leading to the construction of plasmid pBG511020 containing the nucleotide sequence encoding met-BGH using the synthetic HindIII-NcoI linker strategy.

### 6.3.5. THE CONSTRUCTION OF THE PLASMID pN-37 BY EMPLOYING A SYNTHETIC BglII-NcoI LINKER

The modified met-BGH plasmid pN-37 was constructed in order to insert an ATG (methionine codon) 5'- to the mature BGH sequence (FIG. 9). The pJS413 derivative p572-13-1 was digested with BglII, subjected to limited Bal 31 treatment for 20 to 60 seconds and then ligated in the presence of the synthetic BglII/NcoI linker (See FIG. 5). This procedure was used to remove the BGH leader sequence. The randomized DNA mixture was used to transform E. coli strain NF1829. Transformants were amplified and plasmid DNA isolated. (N.B., no individual colony was first isolated, the entire "randomized" DNA population was amplified and purified; an NcoI site would have been regenerated only if the linker was ligated next to a corresponding G nucleotide in the randomized sequence).

Plasmid DNA from this randomized population was digested with SmaI/BglII and approximately 400 b.p. fragments were isolated by agarose gel electrophoresis. The fragments were ligated into the SmaI/BglII backbone of pDN612 (see FIG. 9). The ligation mixture was then used to transform E. coli strain NF1829 and plated on LB plates containing 100 μg/mℓ ampicillin. Individual colonies were screened for the presence of an NcoI site. The DNA of one plasmid, p112-Nco(+), was isolated and found to contain an NcoI site.

Plasmid DNA from the p572-13-1 BglII/NcoI heterogeneous population was digested with NcoI and SmaI and fragments of approximately 400 b.p. were isolated by agarose gel electrophoresis. These NcoI/SmaI fragments were then ligated to the approximately 6.2 kb NcoI/SmaI backbone of p112-NcoI(+). The ligation mixture was used to transform E. coli strain NF1829 and plated onto MacConkey-lactose agar plates containing 100μg/mℓ ampicillin and β-galactosidase positive colonies were isolated. One such isolate, pN-37, contains the BglII/NcoI linker and amino acids 9 to 132 of the mature BGH sequence (See FIG. 9).

### 6.3.6. METHOD FOR THE GENERATION OF met-BGH BY EMPLOYING PRIMER REPAIR

This procedure uses the method of primer repair as described by Goeddel (1980, Nucleic Acids Res., 8: 4057-4074) and Messing et al. (1982, Gene, 19: 269-276) to generate a met-BGH plasmid.

Plasmid p13EX (FIG. 4) was digested with SmaI and BglII. A 405 b.p. fragment was then isolated by agarose gel electrophoresis. A 25-mer DNA single-strand primer was then synthesized. The primer contains the nucleotides (TATG) at the 5'- end and 21 nucleotides corresponding to amino acids 1 to 7 of the mature BGH (See FIG. 10). The primer reaction was performed as follows: 80 μℓ of double distilled $H_2O$ containing 0.5 μg of the 405 b.p. BglII/SmaI of fragment of p13EX, 1.0 μg of primer treated with kinase, and 10 μg of bacteriophage M13-N-37DNA were boiled for 5 minutes and then allowed to anneal at room temperature for 30 minutes. The addition of M13-N-37DNA (described below) was a modification of the basic primer repair reaction to minimize the reannealing of the complementary strands of the BglII/SmaI fragment, and thus maximize the reannealing of the 25-mer primer to the negative strand. The M13-N-37DNA was prepared by inserting the BglII/SmaI fragment of pN-37 into bacteriophage M13. Since the BglII/SmaI fragment of pN-37 (see section 6.3.5.) is deleted for the nucleotides encoding the first eight amino acids of BGH and the primer contains the nucleotide sequences corresponding to only the first seven amino acids of BGH, no reannealing will occur between these two DNA species. Instead, the M13-N-37DNA will hybridize to the positive strand of the BglII/SmaI fragment which competes with the primer for the negative strand (see FIG. 10).

The solution was adjusted to 6mM Tris-HCl (pH 7.5), 6mM $MgCl_2$, 100mM NaCl, 1mM dithiothreitol, and 0.5mM of each dATP, dCTP, dTTP and dGTP and 10 units of the Klenow fragment of DNA polymerase was then added to create blunt ends. The reaction was allowed to proceed at 30°C for 2 hours and then placed at 65°C for 10 minutes. Two units of PstI were added to digest the primer repaired fragment and the resultant 280 b.p. fragment was isolated by agarose gel electrophoresis.

In order to generate a vector having one blunt end and a PstI cohesive end backbone, the vector pCJ2 (a derivative of pJS413 in which all the nucleotide sequences of the *cro* gene are deleted except those coding for the first two amino acids) was digested with BglII/SacI and the approximately 4.5 kb fragment was isolated (FIG. 10A). Plasmid pN-37 was digested with BglII/SacI and the approximately 2.4 kb fragment which contained BGH sequences was isolated by gel electrophoresis. The pCJ2 BglII/SacI fragment and the BglII/SacI fragment of pN-37 were then ligated, the mixture was used to transform *E. coli* NF1829, and the plasmid DNA purified.

One clone, plasmid pCJ2(2-4) contained a 370 b.p. BGH insert from amino acids 9 to 132 (See FIG. 10A), pCJ2(2-4) was digested with BglII and the overhangs were filled in using Klenow fragment of DNA polymerase I and four dNTP's. The DNA was then digested with PstI and isolated by agarose gel electrophoresis. The approximately 7.5 kb DNA backbone was ligated to the 280 b.p. primer repaired/PstI digested fragment and used to transform E. coli strain NF1829. The resulting sequence of the met-BGH construct designated p27 (see FIG. 10A) contains cro sequences (including cro ATG) followed by the mature BGH sequence (ATG and amino acids 1-132).

In order to express met-BGH, the plasmid p27 was digested with SacI and BglII and the approximately 2.4 kb BGH containing fragment was isolated by agarose gel electrophoresis (FIG. 10B). Similarly, the 4.5 kb BglII/SacI fragment from the pJS413 derivative pDN612 (which contains the gene for $Amp^r$ and the site of origin) was isolated and ligated with the BglII/SacI fragment from p27 (See FIG. 10B). By this construction a plasmid was generated such that the met initiation codon was located 5'- to amino acid number 1 (GCC) of the mature BGH sequence and 3'- to the $SD^{cro}$ sequences with no cro initiator ATG codon present. The carboxy terminus of BGH was then reconstructed as previously described, in Sec. 6.3.1 supra, and this construct was identified as p27-112-4/C.

## 6.4. CONSTRUCTION OF SYNTHETIC FRONT END

In order to increase expression levels by optimizing translational initiation starts for the met-BGH constructs, a DNA fragment encoding for the N-terminus of BGH (amino acids 1-24, with the first amino acid encoding for methionine) was chemically synthesized using state-of-the-art procedures. Eight different oligonucleotides, varying in length from 15 to 25 bases, were synthesized by the phosphite-triester method of Kempe et al. (1982, Nucleic Acids Res. 10(21): 6695-6714). Overlapping regions of complimentarity were used in order to facilitate in the assembly of the various fragments. All oligonucleotide fragments were then phosphorylated using T4 polynucleotide kinase and $\gamma^{32}$P-ATP. Phosphorylated fragments were then ligated using T4 DNA ligase, and subsequently purified by gel electrophoresis. FIG. 11 shows the nucleotide sequence of the synthetic front end (hereinafter referred to as SFE) DNA fragment compared to the natural codons of the BGH gene. Codon changes for 13 of the 24 amino acids were made in this fragment.

### 6.4.1. CONSTRUCTION OF PLASMIDS pSFE11, p104-14 and p102-1

The construction of these plasmids is depicted in FIGS. 12 and 12A. An approximately 200 b.p. DNA fragment containing BGH sequences was generated by digesting pBH27 with PvuII and PstI. The DNA fragment coding for amino acids 24-91 was isolated by gel electrophoresis. The expression plasmid, pCJ2(2-4), was digested with BglII and PstI and the approximately 7 kb fragment was isolated by gel electrophoresis. The SFE sequences, the PvuII/PstI 200 b.p. BGH fragment, and the 7 kb BglII/PstI backbone of pCJ2(2-4) fragments were ligated at a 20:5:1 molar ratio using T4 DNA ligase. The ligation mixture was used to transform E. coli strain NF1929. A chloramphenicol resistant recombinant expression plasmid was identified as pSFE-11 (See FIG. 12).

Plasmid DNA from pSFE-11 was digested with BglII and SmaI and approximately 400 b.p. BGH fragment was isolated by gel electrophoresis (see FIG. 12A). The BGH plasmid p27-112-4/C was digested with BglII and SmaI and the 4.5 kb plasmid backbone was isolated by gel electrophoresis. The 400 b.p. BglII/SmaI BGH fragment from pSFE-11 and the 4.5 kb BglII/SmaI backbone of p27-112-4/C were ligated at a 5:1 molar ratio using T4 DNA ligase. The ligation mixture was used to transform E. coli strain NF1829. The recombinant BGH plasmid p104-14 was isolated from transformants (See FIG. 12A).

p104-14 was partially digested with HincII (to generate HincII blunt ends) and an approximately 3640 b.p. fragment was purified by gel electrophoresis. This fragment was then ligated in the presence of linkers which encode a HindIII recognition sequence (See FIG. 12A cont'd) at a molar ratio of 50:1 linker:fragment using T4 DNA ligase. E. coli strain NF1829 was transformed and the recombinant BGH plasmid 102-1 was identified. Plasmids p104-14 and p102-1 are identical except for the removal of a 950 b.p. sequence at the 3' end of the BGH genome from plasmid p104-14 thereby generating p102-1.

### 6.4.2. CONSTRUCTION OF ptac CLONES

Various "tac" or trp-lac promoter/operator region clones were constructed. The hybrid promoter was constructed by combining the -35 region and the -10 region (the sequences of DNA which are the RNA polymerase binding site) of and tryptophan and the lactose promoters (See FIG. 13).

Plasmid p104-14 was digested with the enzyme RsaI to generate a 549 b.p. fragment containing $SD^{cro}$/BGH

sequences (see FIG. 14). The plasmid pDR540 (obtained from PL-Biochemicals, Milwaukee, WI) contains the strong consensus tac (or trp-lac) promoter. This plasmid was linearized by digestion with BamHI and the cohesive ends filled-in using the Klenow fragment of DNA polymerase I and dNTP's. The linearized DNA was isolated by agarose gel electrophoresis and ligated to the 549 b.p. RsaI fragment. The ligation mixture was used to transform E. coli NF1829. Plasmids were studied by restriction analyses to select for the insertion of the RsaI fragment in the desired orientation (see FIG. 14). One clone selected, p121-1, contained the ptac/$SD^{cro}$/BGH sequences. Plasmid p191-2 was constructed in the identical manner.

Plasmid p104-14 was then digested with XmnI and BglII and the plasmid backbone purified and isolated by agarose gel electrophoresis (see FIG. 14A). The ptac containing fragment of p121-1 was isolated by digestion with XmnI and BglII and separated by gel electrophoresis. DNA of the plasmid p104-14 backbone and the p121-1 containing fragment were ligated and used to transform E. coli strain NF1829. Clones were analyzed for the presence of an HindIII site. One clone was selected and designated p141-2 (see FIG. 14A). Note that the distance between the $SD^{lac}$ and the $SD^{cro}$ regions in this clone was 12 nucleotides (See FIG. 14B).

p153-1 was constructed by the same procedure described for p141-1 supra except that after linearizing the pDR540 plasmid with BamHI, the 5'-overhang was treated with mung-bean nuclease to create blunt ends. The tac containing fragment from plasmid p127-10 was ligated to the p104-14 backbone and used to transform E. coli NF1829. One plasmid p153-5 was isolated and analyzed.

The distance between the two SD regions was 9 nucleotides (See FIG. 14B).

6.4.3. INSERTION OF A/T RICH SEQUENCES

An A/T rich DNA segment was inserted between the $SD^{cro}$ and the ATG of BGH.

Expression vector p102-1 DNA was digested with *BglII* and treated with mung-bean nuclease to remove the 5'-overhang and to create blunt-ends (see FIG. 15). A DNA oligomer which contains within it the recognition site for the restriction endonuclease *AhaIII*, was synthesized using the phosphite-triester method of Kempe *et al.*, *supra* (Sec. 6.4.). This linker was then ligated with the *BglII*/Mung treated linearized backbone of p102-1 in a ratio of 50:1 using T4 DNA ligase. The ligation mixture was used to transform *E. coli* strain NF1829, plated on LB-agar plates that contained 100 μg/mℓ of ampicillin and individual colonies were selected for the presence of the *AhaIII* restriction site. One such clone was designated p128-7/2 (See FIG. 15).

Following the successful insertion of this linker, the distance (or number of nucleotides) between the $SD^{cro}$ and the ATG of the BGH genome was reduced by restriction digestion of plasmid p128-7/2 with *AhaIII* and *HindIII* followed by isolation of a 900 b.p. BGH containing fragment by agarose gel electrophoresis. An approximately 2.4 kb *BglII*/Mung-treated/*HindIII* fragment containing $amp^r$ and site of origin was also isolated from plasmid p128-7/2. Ligation of the 900 b.p. fragment with the 2.4 kb backbone was performed using T4 DNA ligase and the DNA mixture was used to transform *E. coli* strain NF1829 and plated onto LB-agar plates containing 100 μg/mℓ of

ampicillin. Individual colonies were isolated and screened for the absence of the AhaIII site between the $SD^{cro}$ and the ATG of BGH. One such clone found has been given the designation p146-1 (see FIG. 15). FIG. 16 shows the number of nucleotides between he $SD^{cro}$ and the ATG of BGH in clone p128-7/2 is 12 nucleotides and the distance between the $SD^{cro}$ and the ATG of BGH in clone p146-1 is only 8 nucleotides.

Plasmid p146-1 was digested with RsaI and a 549 b.p. BGH fragment was isolated by agarose gel electrophoresis and purified (see FIG. 17). This fragment was then ligated to the BamHI/filled-in fragment of pDR540 as described supra in Sec. 6.4.2. The ligation mixture was then used to transform E. coli strain NF1829, plated onto LB-plates containing 100 μg/mℓ ampicillin and individual colonies were screened for the correct orientation of the BGH fragment. One such clone, designated here as p166-1 was isolated and plasmid DNA purified (See FIG. 17). DNA from p166-1 was digested with XmnI and SmaI and an approximately 800 b.p. fragment containing BGH sequences was isolated (see FIG. 17A). p102-1 was also digested with XmnI and SmaI and an approximately 3.0 kb fragment containing $amp^r$ and the site of origin was isolated by agarose gel electrophoresis and purified. The 800 b.p. BGH fragment and the 3.0 kb ampicillin/ori backbone were ligated in a ratio of 5:1 using T4 DNA ligase and this mixture was used to transform E. coli NF1829, plated onto LB-plates with 100 μg/mℓ of ampicillin and individual colonies were selected for the BGH insert and the tac promoter sequences. Plasmid p179-3 contains the sequences for ptac-$SD^{cro}$-A/T-BGH.

6.4.4. INSERTION OF TRANSLATIONAL TERMINATION SEQUENCES

Sequence analysis of the cDNA clone pBH27 revealed that only one stop codon (TAG) was present for the termination of translation, and that no transcriptional stop-like sequences were found in the 3'-untranslated region of the clone. Therefore, a synthetic oligomer containing a triple translational stop (hereinafter referred to as TTS) sequence of all 3 stop codons (TAG, TAA, TGA) in phase with the reading frame of the protein was synthesized using the phosphite-triester method of Kempe et al. (described supra).

In order to insert the TTS linker into the BGH sequences, p104-14 was digested with PvuII (FIG. 18). The approximately 3.4 kb backbone and the 500 b.p. BGH containing fragment were isolated by agarose gel electrophoresis and purified. The TTS linker which encodes all three stop codons followed by the HindIII recognition sequence was ligated to the backbone of p104-14 at a ratio of 50:1 using T4 DNA ligase. The ligation mixture was used to transform E. coli strain NF1829, plated onto LB-agar plates containing 100 μg/mℓ of ampicillin and individual colonies were selected and screened for the insert. Clones were also selected for the proper orientation of the linker as described in FIG. 18. One such clone has been given the designation of p164-3. This plasmid, was linearized by digestion with PvuII and isolated by agarose gel electrophoresis. The BGH PvuII 500 b.p. fragment from p104-14 was then ligated to the backbone of p164-3 at a ratio of 10:1. The ligation mixture was used to transform E. coli strain NF1829, plated onto LB-agar plates with 100 μg/mℓ ampicillin and individual colonies screened for the insertion and proper orientation of the BGH insert. One plasmid, p175-12 contains the 3 translational stop codons and provides for the efficient termination of translation of the BGH message.

### 6.4.5. INSERTION OF TRANSCRIPTIONAL TERMINATION SEQUENCES

Since no transcriptional attenuator region was found in the cDNA clone which provided for the efficient termination of transcription, a sequence coding for the attenuator portion of the tryptophan operon was placed approximately 100 b.p. 3'- to the translational stop codons of the BGH sequence (See FIG. 19).

Plasmid p175-12 was digested with *Hin*dIII, the recessed ends of the DNA were filled-in with dNTP's using the Klenow fragment of DNA polymerase I to create blunt-ended fragments and then further digested with *Xmn*I. An approximately 1100 b.p. fragment containing BGH sequences was isolated by agarose gel electrophoresis and purified. Another preparation of p175-12 was digested with *Hin*dIII and *Xmn*I and the 2000 b.p. fragment which contained the ampicillin resistance gene and the sequences for the site of origin were isolated by agarose gel electrophoresis and purified. A third plasmid, ptrpED5-1 (Hallewell, R.A. *et al*., 1980, Gene, 9: 27-47), was digested with *Hpa*I and *Hin*dIII and an approximately 2400 b.p. fragment which contains the tryptophan attenuator sequences was isolated by agarose gel electrophoresis and purified. This fragment also contains sequences for the *trp*E an the *trp*D genes. The three fragments were ligated at a ratio of 5:2:1 using T4 DNA ligase and the ligation mixture was used to transform *E. coli* strain NF1829, plated onto LB plates containing 100 μg/mℓ ampicillin, and individual colonies were isolated and screened for the insertion of the BGH sequences and the *trp* attenuator sequences. Plasmid p211-4 was shown to contain the BGH fragment followed by the trp attenuator region 3'- to the TTS.

### 6.4.6. CONSTRUCTION OF ptac-A/T-SFE-BGH-TERM PLASMID

Plasmid p217-1 was constructed to contain the following features: (1) a tac promoter to provide for a very "strong" promoter of mRNA transcription in E. coli; (2) an A/T rich intervening region to optimize translational starts; (3) a synthetic front end to optimize for a lack of secondary structure and to replace non-preferred codons with preferred codons for maximum translational efficiency; (4) a triple translational stop sequences (TTS) to provide for a unique sequence of tandem translational stops to assure proper termination; and (5) a trp attenuator to provide for effective termination of transcription which may, in addition contribute to the overall stability of the mRNA by limiting its size.

Plasmid p179-3 which contains ptac, an A/T rich region and BGH sequences as described supra, was digested with EcoRI and SmaI and the 500 b.p. ptac containing fragment was isolated by agarose gel electrophoresis and purified (See FIG. 20). Plasmid p211-4 described supra was also digested with EcoRI and SmaI and the approximately 5.5 kb plasmid backbone which contains the TTS and trp attenuator sequences was isolated by gel electrophoresis and purified. These fragments were then ligated at a molar ratio of 5:1 using T4 DNA ligase and the ligation mixture used to transform E. coli NF1829. Individual colonies were isolated and screened for the insertion of the ptac-BGH sequences into the TTS-trp attenuator backbone. Plasmid p217-1 was analyzed by restriction mapping and shown to contain ptac-A/T-BGH TTS-trp attenuator sequences.

Plasmid p214-1 was constructed in the identical manner described supra. This plasmid lacks the A/T rich region and the SFE.

## 6.5. INSERTION OF BGH INTO PPV FUSION PROTEIN PLASMID

Plasmids were constructed to carry and express the unfused BGH protein and the larger PPV/ß-galactosidase fusion protein. The construction of plasmid pPPV1000t was described in U. S. Patent Application Serial No. 456,423 by Halling et al. filed January 7, 1983. This plasmid contains the unique restriction site NcoI (CCATGG) near the end of the ß-galactosidase gene. This site was replaced by opening the plasmid at its unique NcoI site, treating the DNA with a limited amount of the nuclease Bal 31 in order to create flush ends, and inserting a linker which contains the restriction endonuclease XmnI site (FIG. 21). Clones which contain the unique XmnI site were isolated and plasmid DNA purified. One clone, designated pPPV1000tx contained the cro/PPV/ß-galactosidase fusion protein.

Plasmid DNA was isolated from clone pPPV1000tx. This plasmid carries the gene for tetracycline resistance, therefore bacteria transformed with this plasmid will be both tetracycline resistant and able to produce the PPV/ß-galactosidase fusion protein.

6.5.1. INSERTION OF pTAC BGH SEQUENCES INTO THE PPV FUSION PROTEIN PLASMID

Plasmid from BGH construct p141-2 was digested with the restriction endonuclease HindIII and the 3'-recessed ends were filled-in with dNTP's using the Klenow fragment of DNA polymerase I (see FIG. 22). An approximately 1000 b.p. fragment containing the ptac and BGH sequences were isolated by agarose gel electrophoresis and purified. The plasmid pPPV1000tx (described supra) was digested with the restriction endonuclease XmnI and the approximately 7.2 kb linearized plasmid was isolated by agarose gel electrophoresis and purified. The ptac-BGH fragment and the pPPV1000tx linearized fragment were then ligated at molar ratio of 10:1 using T4 DNA ligase. This mixture used to transform E. coli strain NF1829. The transformants plated onto LB-agar plates containing 20 µg/ℓl tetracycline, individual colonies were isolated and clones were selected for the insertion of the ptac-BGH sequences into the PPV/ß-galactosidase fusion protein vector. This procedure was also repeated using the ptac-A/T-BGH clone p179-3 (See FIG. 22).

A clone isolated from the p141-2 construct was designated p158-1. A clone isolated from the p179-3 construction was designated p195-4. Clone p158-1 and p195-4 were assayed for the production of both the PPV/ß-galactosidase fusion protein and the BGH protein.

6.5.2 INSERTION OF p214-1 INTO THE PPV-FUSION PROTEIN PLASMID

Plasmid p214-1 described supra, was digested with the restriction endonuclease EcoRV and the approximately 800 b.p. fragment containing the ptac-BGH-TTS-trp attenuator sequences was isolated by agarose gel electrophoresis and purified (see FIG. 23). Plasmid pPPV1000tx described supra, was digested with XmnI and the linearized plasmid was isolated by agarose gel electrophoresis and purified. The 800 b.p. ptac-BGH fragment and the approximately 7.2 k.b. linearized plasmid pPPV1000tx were ligated in a molar ratio of 10:1 using T4 DNA Ligase. This mixture was used to transform E. coli strain NF1829 and plated onto LB-agar plates that contained 20 μg/mℓ tetracycline. Individual colonies were isolated and screened for the insertion of the ptac-BGH sequences into the PPV backbone. Clone p231-3 was isolated and assayed for the production of both the PPV/ß-galactosidose fusion protein and the modified BGH protein (See FIG. 23).

### 6.5.3 INSERTION OF pTAC-A/T-TTS-att-BGH INTO THE PPV FUSION PROTEIN PLASMID

An approximately 900 b.p. fragment which contains the ptac-A/T-BGH-TTS-trp attenuator sequences from plasmid p217-1 described supra, was isolated by gel electrophoresis after digestion with EcoRV as described in FIG. 23. Plasmid pPPV1000tx was digested with XmnI and linears were isolated by agarose gel electrophoresis and purified. The ptac-A/T-BGH-TTS-att EcoRV fragment and the 7.2 kb pPPV1000tx linear were ligated at a molar ratio of 10:1 using T4 DNA ligase and this mixture was used to transform E. coli strain F' SK107 which contains the F'$i^q$ mutation (F factor plasmid which carries on it the $i^q$ mutation which leads to the overproduction of the lac repressor protein). Transformants were then plated onto LB-agar plates which contain 20 μg/mℓ tracycline and individual colonies were isolated and screened for the insertion of the BGH sequences. Clones p230-2/3, p230-5/3 and p230-6/2 were isolated and assayed for the production of both the PPV/ß-galactosidase fusion protein and the BGH protein.

Plasmid DNA from clone p230-6/2 was isolated and used to transform the E. coli strain SK107 (no $i^q$ mutation). Transformants were plated onto LB-agar plates that contain 20 µg/mℓ tetracycline and individual colonies were isolated. One isolate, p230-6/1, was assayed for its production of both the PPV/ß-galactosidase fusion protein and the BGH polypeptide in the absence of any inducer (i.e. IPTG or lactose).

6.5.4 ANALYSIS OF PROTEINS PRODUCED BY TRANSFORMANTS

All pPPV recombinant plasmids were used to transform E. coli strain NF1829, SK107, or F' SK107.

Transformants were isolated and assayed by restriction analysis for the insertion of the fragment. Clones which were positive for the insert were then assayed for the production of both the PPV/ß-galactosidase fusion protein and the BGH protein after induction of the promoter with IPTG or lactose.

A number of clones were identified as expression positive for both proteins. Furthermore, a significant amount of stabilization of the BGH protein was evident. Purification of the aggregate proteins also enabled the purification of the unfused protein.

The unfused protein and fusion proteins produced by E. coli transformants were isolated and purified by the non-denaturing co-aggregate purification method which is outlined in detail in Section 5.6, supra. Briefly, L-broth containing, as appropriate, either 100 µg/mℓ ampicillin and/or 20 μg/mℓ tetracycline and 1% lactose or 1mM IPTG was inoculated with select transformants. After incubation at 30°C overnight the cells were pelleted and quick frozen in dry ice/methanol. The unfused protein and fusion protein were then isolated according to the embodiment described in Section 5.6. The unfused protein can be separated from the fusion protein on the basis of size or charge.

The isolated co-aggregate proteins were resuspended in 50 μℓ distilled $H_2O$ to which 50 μℓ of 2x Sample Buffer (2x Sample Buffer is composed of 10% SDS, 40mM Tris-HCl, pH6.8, 2% ß-ME, and 0.02 volumes saturated solution of bromphenol blue) was added and mixed well. The mixture was boiled for 5 minutes and 25μℓ aliquots were applied to 10% SDS-polyacrylamide gels or electrophoresis.

After SDS-PAGE, the unfused protein was present in sufficient quantity to form a prominent stainable band. The protein can therefore be isolated and purified from the larger aggregate forming protein by standard separation techniques. Amino acid analysis of this band was performed using state-of-the-art techniques to confirm that this protein was indeed the desired product.

6.5.5. QUANTITATION OF PROTEIN

Analysis via SDS-PAGE indicated that in clones which contain and express the PPV/ß-galactosidase fusion protein that approximately 10 to 15% of the total bacterial cell protein was fusion protein.

The minimum amount of expression need to cause aggregation of the fusion protein is probably dependent on the nature of the fusion peptide utilized. For several BGH -PPV/β-galactosidase fusion constructs, a significant amount of expression (greater than 3%) was required to cause the co-aggregation of these unfused protein and fusion protein.

### 6.6 INSERTION OF BGH SEQUENCES INTO THE HIGH COPY NUMBER PLASMID pORI-TET #1

Derivatives of the runaway plasmid pOPIΔ6 were constructed in order to enhance the expression levels of BGH.

Plasmid pOPIΔ6 was digested with EcoRI/PvuII and a 1.7 kb fragment which contains the ori mutation site was isolated by agarose gel electrophoresis and subsequently purified (see FIG. 24). A 2065 b.p. EcoRI/PvuII fragment which contains the tetracycline resistance gene was isolated from pBR322. The two fragments were ligated at a 1:1 ratio and the mixture was used to transform E. coli strain NF1829. A tetracycline resistant, ampicillin sensitive colony was isolated and the plasmid DNA amplified and purified. The construction of the runaway plasmid pORI-tet#1 is outlined in FIG. 24.

Plasmid pORI-tet#1 was digested with AvaI, EcoRI of PvuII. The AvaI cohesive ends and the EcoRI cohesive ends were filled-in with dNTP's using the Klenow fragment of DNA polymerase I (see FIG. 25). The blunt-ended EcoRV fragment of p217-1 (see section 6.4.6. supra) was isolated by agarose gel electrophoresis and purified. The approximately 900 b.p. BGH fragment which contains the "tac" promoter sequences 5'- to the BGH gene and the trp attenuator sequences 3'- to the BGH gene, were ligated (in three separate reactions) to the three isolated linears from pORI-tet#1. The ligation mixture was used to transform E. coli SK107 which contains the F' factor carrying the $i^q$ mutation for the lac repressor protein.

Transformants were plated on LB agar containing 100 μg/mℓ ampicillin. Individual colonies were selected, plasmid DNA was isolated, and plasmids were selected for both the presence of the BGH insert and the high copy number phenotype after analysis by agarose gel electrophoresis.

Three constructions of the runaway plasmids p237-D2/1, p238-D3/1, p239-D5/1 are shown in FIG. 25. Aggregated BGH protein was subsequently purified from these clones using the non-denaturing aggregate purification method as described supra (Sec. 5.6.). Purified BGH protein was determined to be identical to its pituitary counterpart by comparison of tryptic peptide mapping procedures and subsequent amino acid sequence determination.

### 6.7. CONSTRUCTION OF pTRP EXPRESSION VECTORS

The operator, lacO was inserted 5'- to the SFE-BGH plasmids in order to efficiently regulate the trp promoter and increase expression of BGH.

The construction of an expression vector carrying a trp promoter fragment and lacO fragment is described below.

#### 6.7.1. CONSTRUCTION OF PLASMID pTC503

Plasmid pGC301 (Christie et al., 1981, Proc. Natl. Acad. Sci. 78:4180) is a trp vector which contains the trp promoter region, operator region, and $SD^{trp}$ as seen in FIG. 26. The vector was modified and used to express BGH. Plasmid pGC301 was digested with restriction endonuclease HinfI and the ends were made blunt by "filling-in" using the Klenow fragment of DNA Polymerase I. The blunt-end fragments were electrophoresed in 1% LMP and the approximately 965 b.p. fragment was removed from the gel and purified by phenol extraction. The fragment contains the promoter/operator regions and approximately 630 b.p. of coding sequences for the trpC gene. This fragment was ligated to pBR322 DNA which was digested with EcoRI and also rendered blunt-ended by treatment with the Klenow fragment DNA polymerase I. The ligation mixture was used to transform E. coli MC 1000 and ampicillin resistant colonies were isolated and DNA of plasmid pTC503 was purified.

### 6.7.2. CONSTRUCTION OF PLASMID PTNA771

Plasmid pTC503 was used to construct plasmid pTNA771 as described in FIG. 27. Plasmid pTC503 was cleaved with HpaI and ClaI and an approximately 4.3 kb fragment was isolated from LMP, thus removing the C gene (including the ATG) from the plasmid. Since there are several TaqI restriction sites in pTC503, a second plasmid, pTC503 was first cleaved with HpaI and BglII and the small fragment was isolated from LMP. The HpaI/BglII fragment was then cleaved with TaqI, and the 346 b.p. HpaI/TaqI fragment was isolated and ligated to the large HpaI/ClaI fragment to generate plasmid pTNA771. The plasmid pTNA771 contains the trp promoter, operator, region of initiation of transcription, and $SD^{trp}$ sequences (the trpC has been deleted).

### 6.7.3. CONSTRUCTION OF PLASMID POLINK 23456

A synthetic 71 b.p. multi-restriction site fragment (polink 23456) was inserted into pBR328. Plasmid pBR328 (Bethesda Research Laboratories, Gaithersburg, MD) was digested with EcoRI and SalI, and an approximately

3100 b.p. fragment was isolated from LMP and ligated to the polink23456 DNA. The construction of the Polink23456 vector is described in FIG. 28.

### 6.7.4. CONSTRUCTION OF PLASMID PTNAP771

Plasmid pTNAP771 was constructed to include the multi-restriction site synthetic linker described *supra*. Plasmid pTNA771 was cleaved with *Pst*I and *Cla*I and the approximately 1650 b.p. fragment was isolated from LMP (see FIG. 29). Plasmid Polink23456 was cleaved with *Pst*I and *Xba*I, the fragments were then partially "filled-in" using Klenow fragment of DNA polymerase I in the presence of dCTP and dTTP, and the 1950 b.p. fragment was isolated by agarose gel electrophoresis as described in FIG. 29. The modified *Pst*I/*Xba*I fragment of plasmid Polink23456 was ligated to the *Pst*/*Cla*I fragment of pTNA771 and the DNA mixture used to transform *E. coli* MC1000. Plasmids were screened for the presence of an *Xba*I site resulting fom cellular repair of the T-C mismatch to a T-A. One plasmid generated, pTNAP771 (shown in FIG. 29) contained the nucleotide sequence of the tryptophan promoter, operator, mRNA initiation site, and ribosome binding site in addition to several unique restriction sites as shown in FIG. 30.

### 6.7.5. CONSTRUCTION OF pTRP VECTOR pSFE-1 WHICH EXPRESSES BGH

The construction of plasmid pSFE-1 from pSFE-11 (FIG. 12) is shown in FIG 31. Plasmid pSFE-11 was digested with *Xmn*I and *Bgl*II and the 6300 b.p. fragment was isolated by agarose gel electrophoresis. Plasmid pTNAP771 was also cleaved with *Xmn*I and *Bgl*II and the approximately 750 b.p. fragment was isolated and ligated to the large fragment of pSFE-11. The ligation mixture was used to transform E. coli NF1829. Ampicillin resistant colonies were examined for production of fusion protein on indicator agar plates. Plasmid pSFE-1 produced a BGH/ß-galactosidase fusion protein.

To express BGH as an unfused protein, i.e., to remove the i-z fusion portion and to reconstruct the 3'-carboxyl-coding terminus of BGH; the following procedure was used (see FIG. 32). Plasmid pSFE-1 was cleaved with EcoRI and SmaI and the approximately 4.3 kb fragment was isolated by agarose gel electrophoresis. Plasmid p27-112-4/2C (described supra) was also cleaved with EcoRI and SmaI, the approximately 1042 b.p. fragment containing the carboxyl-terminus of BGH was isolated by agarose gel electrophoresis and ligated to the isolated fragment of pSFE-1. The ligation mixture was used to transform E. coli NF1829. The procedure used to generate plasmid p106-15 is shown in FIG. 32. Plasmid p106-15 contains the sequences for ptrp/ $SD^{trp}$/ BGH.

### 6.7.6. CONSTRUCTION OF pTRP VECTORS WHICH ARE REGULATED BY THE LAC OPERATOR

The expression of the BGH/ß-galactosidase fusion protein or metBGH in various trpR+ strains (i.e., strains which produce an active trp repressor) containing plasmids pSFE-1 or p106-15, respectively, was similar in both the presence and absence of the co-repressor L-tryptophan. These results indicated that the trp vector system lacked the ability to significantly regulate BGH expression. Therefore, the trp promoter was placed under the control of lacO by inserting a synthetic lacO nucleotide sequence seventeen nucleotides 3'- to the -10 region of ptrp. The inclusion of an F' lac episome with the lac $I^q$ mutation for lac repressor overproduction in the host strain assured sufficient lac repressor molecules to efficiently repress BGH synthesis. Induction of BGH synthesis was achieved by the addition of the inducer lactose or IPTG to the bacterial culture at a phase of growth where BGH synthesis was maximal.

### 6.7.7. CONSTRUCTION OF PLASMID p407-29 WHICH IS REGULATED BY LACO

The construction of plasmid pT248-11 which contains ptrp, $SD^{trp}$, and $SD^{cro}$ sequences is described in FIG. 33.

Plasmid SFE-11 was digested with RsaI and SmaI and the approximately 400 b.p. fragment which contains $SD^{cro}$ and the amino terminal BGH sequences (including amino acids 1-132) was isolated by agarose gel electrophoresis. Plasmid pSFE-1 was linearized by digestion with BglII and made blunt-ended using the Klenow fragment of DNA polymerase I. The two fragments were ligated and used to transform E. coli NF1829 and plasmid pT248-11 was isolated.

The construction of the ptrp vector pMTE-407-29 which is regulated by lacO is shown in FIG. 34. Plasmid pT248-11 was cleaved at the unique XbaI site and the resulting four base 5'- overhangs were made blunt-ended by treatment with mung-bean nuclease. In order to remove most of the DNA sequences between the -10 region and the SD region, the modified plasmid DNA was then subjected to limited 3'- to 5'- exonuclease digestion by treatment with $T_4$ DNA polymerase in the presence of dCTP. The thirteen nucleotide 5'- overhang remaining was then removed by digestion with mung-bean nuclease. The resultant DNA was then cleaved with EcoRI and the approximately 3.3 kb fragment was isolated by a agarose gel electrophoresis. Plasmid p106-15 (described supra) was digested with HpaI and *EcoRI*, and the 1500 b.p. fragment which contains the BGH sequence was isolated by agarose gel electrophoresis and ligated to the *EcoRI* fragment of pT248-11 in the presence of a 25-mer synthetic oligomer which encodes the sequence for *lacO* (PL Biochemicals, Milwaukee, Wis).

The ligation mixture was used to transform *E. coli* NF1829. The plasmids were then modified to encode fusion proteins in order to assay for ß-galactosidase activity on indicator agar plates. Several colonies were selected and the DNA from one plasmid, pMTE-132 (FIG. 34A) was digested with *BglII* and *EcoRI*. A 3.4 kb fragment which contained sequences for *ptrp*, *lacO*, and $SD^{trp}$ (in the correct orientation) was isolated by agarose gel electrophoresis. Plasmid pSFE-1 was then digested with *BglII*, *EcoRI*, and *XmnI* and the 3.6 kb fragment (containing BGH sequences) was isolated by agarose gel electrophoresis and ligated to the *BglII*-*EcoRI* fragment from pMTE-132 (see FIG. 34B). The ligation mixture was used to transform *E. coli* NF1829. FIG. 34C shows the sequence of the -10 region to the BGH of pMTE-407-29 construction which contains *ptrp*/*lacO*/$SD^{trp}$/BGH.

The regulation of *ptrp* by *lacO* was analyzed in the presence and absence of the inducer IPTG. In the absence of inducer, expression of BGH/ß-galactosidase fusion protein was reduced 10-15 fold, thus indicating efficient regulation of *ptrp* by *lacO*.

## 6.8. PLASMIDS THAT EXPRESS THE BGH GENE

The plasmids isolated from *E. coli* transformants described *supra* were analyzed for their ability to direct the expression of the BGH protein either as a fusion protein or as an unfused protein. Plasmids were transformed into E. coli NF1829, F' SK107, SK107, or MC1000. Since the lac promoter was transcriptionally inactive in NF1829 and F' SK107 (due to overproduction of the lac repressor), the BGH related protein could only be detected upon induction of the promoter with either 1mM IPTG or 1-10mM lactose.

Fusion proteins were examined by assaying for ß-galactosidase activity on indicator plates. Positive colonies were tested for the presence of BGH/ß-galactosidase fusion protein by SDS-PAGE analysis of total lysates of transformants induced with IPTG.

The unfused proteins were identified and purified by the non-denaturing co-aggregate procedure described supra in Sec. 5.6. To determine if the recombinant expression plasmids containing BGH sequences were capable of synthesizing the predicted BGH (ß-galactosidase fusion protein or unfused protein), total E. coli proteins from cells harboring these expression plasmids were analyzed by SDS-PAGE, followed by Coomassie Brilliant Blue staining. For example, E. coli strain F'SK107 containing either plasmid p238-D2/1 or pSFE-11, were grown in L broth nutrient medium in the absence or presence of inducer (1% lactose). Overnight cultures were harvested, lysed directly in SDS-gel sample buffer (2X sample buffer 0.14M Tris-HCl, pH 7.8, 0.0011% bromophenol blue, glycerol, 6% SDS, 10% ß-ME) by boiling for 5 minutes and aliquots were subjected to 10% SDS-PAGE. The bacterial strains utilized synthesize high molecular weight polypeptides only upon induction of the lac promoter. The proteins were the size predicted for fusion proteins of the inserted BGH sequences and/or z-gene (Table I). Proteins produced by p238-D2/1, and pSFE-11 are approximately 22,000 daltons and 120,000 daltons respectively. After SDS-PAGE, amino acid analysis of the purified BGH broth protein was performed using state-of-the-art procedures to confirm the identity of the BGH protein product.

The recombinant plasmids encoding BGH fusion protein or unfused BGH are shown in Table I. The level of expression of protein is given in terms of percent of total host cell proteins.

TABLE I

| Clone | Description | % Expression | Size of Protein (kilodaltons) |
|---|---|---|---|
| p27-112-4/2c | cro/BGH | < 0.1 | 22 |
| pRed13 | cro/BGH/ß-gal | 10 | 120 |
| pN-37 | mod BGH/ß-gal | 1-3 | 120 |
| pMet 27 | BGH/ß-gal | < 1 | 120 |
| p1-40/c | mod BGH | 0.5-1 | 21 |
| p102-1 | SFE-BGH | 0.5-1 | 22 |
| pSFE-11 | BGH/ß gal | 1-3 | 120 |
| p104-14 | SFE-BGH | 0.5-1 | 22 |
| p128-7/2 | A/T | 1-2 | 22 |
| p153-5 | tac-BGH | 1-5 | 22 |
| p141-2 | ptac | 1-5 | 22 |
| p146-1 | A/T-SFE | 1-2 | 22 |
| p179-3 | tac-A/T-BGH | 2-5 | 22 |
| p214-1 | tac-BGH-TTS-att | 5-10 | 21 |
| p217-1 | tac-A/T-SFE-BGH-TTS-att | 5-15 | 22 |
| p230-2/3 | co-agg. | 5-10 | 22 |
| p230-5/3 | co-agg. | 5-10 | 22 |
| p230-6/2 | co-agg. | 5-10 | 22 |
| p230-6/1 | co-agg | 5-10 | 22 |
| p195-4 | co-agg | 5-10 | 22 |
| p231-3 | co-agg | 5-10 | 21 |
| p238-D2/1 | ori | 10-18 | 22 |
| p239-D5/1 | ori | 10-18 | 22 |
| p237-D3/1 | ori | 10-18 | 22 |
| pMTE-407-29 | lacO | 5-10 | 120 |

## 7. DEPOSIT OF MICROORGANISMS

It is to be understood that all base pair sizes given for nucleotide sequences are approximate and are used for purposes of description. Furthermore, it is apparent that many modifications and variations of this invention as hereinbefore set forth may be made without departing from the spirit and scope thereof. The specific embodiments described are given by way of example only and the invention is limited only by the appended claims.

The following *E. coli* strains carrying the listed plasmids have been deposited with the Agricultural Research Culture Collection (NRRL), Peoria, IL and have been assigned the listed accession numbers:

| E. Coli Strain | Plasmid | Accession Numbers |
|---|---|---|
| K12, MC1000 | pBH27 | B-15648 |
| K12, NF1829 | p226-22 | B-15649 |
| K12, NF1829 | p119-1 | B-15650 |
| K12, NF1829 | p344-3 | B-15651 |
| K12, NF1829 | p106-15 | B-15652 |
| K12, NF1829 | pBG511020 | B-15653 |
| K12, NF1829 | p27-112-4/2c | B-15654 |
| K12, NF1829 | pRed13 | B-15655 |
| K12, NF1829 | p214-1 | B-15656 |
| K12, NF1829 | p128-7/2 | B-15657 |
| K12, NF1829 | p179-3 | B-15658 |
| K12, NF1829 | p102-1 | B-15659 |
| K12, NF1829 | p225-1 | B-15660 |
| K12, NF1829 | p217-1 | B-15661 |
| K12, NF1829 | p153-5 | B-15662 |
| K12, NF1829 | p146-1 | B-15663 |
| K12, NF1829 | p104-14 | B-15664 |
| K12, NF1829 | p141-2 | B-15665 |
| K12, NF1829 | pMet27 | B-15667 |
| K12, NF1829 | pSFE-11 | B-15668 |
| K12, F'SK107 | p238-D2/1 | B-15669 |
| K12, F'SK107 | p230-2/3 | B-15670 |
| K12, F'SK107 | p230-5/3 | B-15671 |
| K12, F'SK107 | p239-D5/1 | B-15672 |
| K12, F'SK107 | p237-D3/1 | B-15673 |
| K12, F'SK107 | p230-6/2 | B-15674 |
| K12, SK107 | p230-6/1 | B-15675 |
| K12, SK107 | p195-4 | B-15676 |
| K12, SK107 | pori-tet#1 | B-15677 |
| K12, SK107 | p231-3 | B-15678 |
| K12, NF1829 | pSFE-1 | B-15679 |
| K12, NF1829 | pMT-433-26 | B-15680 |
| K12, NF1829 | pMTE-407-29 | B-15681 |
| K12, NF1829 | pM425-5 | B-15682 |
| K12, NF1829 | pME474-1 | B-15683 |
| K12, NF1829 | p11 | B-15177 |
| K12, NF1829 | p83 | B-15178 |
| K12, NF1829 | p1-20F | B-15179 |
| K12, NF1829 | p1-40 | B-15180 |
| K12, NF1829 | p1-40C | B-15181 |
| K12, NF1829 | p1-60A | B-15182 |
| K12, NF1829 | p1-60B | B-15183 |
| K12, NF1829 | p112 R/D | B-15184 |
| K12, NF1829 | p112R/G | B-15185 |
| K12, NF1829 | p112 R/L | B-15186 |
| K12, NF1829 | p112 R/O | B-15187 |
| K12, NF1829 | p112 R15 | B-15188 |
| K12, NF1829 | pN-1 | B-15189 |
| K12, NF1829 | pN37 | B-15190, B-15666 |
| K12, NF1829 | pNB9 | B-15191 |
| K12, NF1829 | pDN112 | B-15192 |

A culture of the deposited microorganisms will be made available to the public upon the grant of patent based upon the present application. It is to be understood that the availability of a deposit does not constitute a license to practice the subject invention in derogation of patent rights granted by the United States government. Furthermore, the present invention is not to be limited in scope by the microorganism deposited, since the deposited embodiment is intended as a single illustration of one aspect of the invention. Indeed, various modificatons of the invention in addition to those shown and described herein will become apparent to those skilled in the art from the foregoing description and accompanying drawings. Such modifications are intended to fall within the scope of the appended claims.

28,992

WE CLAIM:

1. A process for producing a polypeptide having bovine growth hormone activity, characterized by

(a) culturing a unicellular organism containing a recombinant vector comprising a DNA sequence coding for a polypeptide having bovine growth hormone activity and capable of being replicated, transcribed and translated in the unicellular organism; and

(b) isolating said polypeptide from the culture.

2. The process according to claim 1, wherein the polypeptide is a bovine growth hormone fusion protein, an unfused bovine growth hormone, or a mature bovine growth hormone.

3. The process according to claim 1, wherein the DNA sequence is obtained by isolating mRNA coding for a bovine growth hormone protein and using reverse transcriptase to contruct a cDNA sequence having one strand complementary to the isolated mRNA.

4. The process according to claim 1, wherein said unicellular organism is a procaryotic organism such as Escherichia coli.

5. A process for preparing a unicellular organism having a DNA sequence coding for a polypeptide having bovine growth hormone activity, said process characterized by: introducing into the unicellular organism by such methods as transformation, transduction or transfection, a recombinant vector comprising a DNA sequence coding for a polypeptide having bovine growth hormone activity and capable of being replicated, transcribed and translated in the unicellular organism.

6. A recombinant DNA vector comprising the BGH DNA sequence as depicted in FIG. 2, or any portion thereof coding for a polypeptide having bovine growth hormone activity wherein the BGH DNA sequence is under the control of a transcriptional promoter, such as the lac promoter, such as the one designated p29-112-412c; the trp promoter also containing the trp operator; the trp promoter containing the trp operator and the lac operator, such as the one designated pMTE-407-29; the tac promoter, such as the one designated p153-5; or the tac promoter, such as the one designated p141-2.

7. The recombinant DNA vector according to claim 6, wherein the BGH DNA sequence is connected in the correct translational reading frame to a second DNA sequence coding for a protein, such as the vectors designated pRed13, pN-37, or pMet-27.

8. The recombinant DNA vector according to claim 6, wherein the BGH DNA sequence further comprises a synthetic front end, such as the vectors designated p102-1, pSFE-11, or p104-14.

9. The recombinant DNA vector according to claim 6 further comprising an A/T-rich DNA region located between the promoter and the BGH DNA sequence, such as said vectors designated p128-7/2, p146-1, or p179-3.

10. The recombinant DNA vector according to claim 6 further comprising a transcription attenuator DNA sequence located at the 3'-terminus of the BGH DNA sequence.

11. The recombinant DNA vector according to claim 6 further comprising tandem translation termination sequences located at the 3'-terminus of the BGH DNA sequence wherein the termination sequences are in the correct translational reading frame with the BGH DNA sequence.

12. The recombinant DNA vector according to claim 10 further comprising tandem translation termination sequences located at the 3'-terminus of the BGH DNA sequence, wherein the termination sequences are in the correct translational reading frame with the BGH DNA sequence, such as said vectors designated p214-1 or p217-1.

13. The recombinant DNA vector according to claim 6 further comprising a second DNA sequence encoding a fusion protein wherein the second DNA sequence is under the control of its own transcriptional promoter, such that the BGH DNA sequence and the second DNA sequence are capable of being replicated, transcribed and translated in a unicellular organism, such as said vectors designated p230-2/3, p230-5/3, p230-6/2, p230-6/1, p195-4 or p231-3.

14. The recombinant DNA vector of claim 6 further comprising a DNA ori sequence which allows for production of a high copy number of the recombinant DNA vector in a host cell, such as said vectors designated p238-D2/1, p239-D5/1, or p237-D3/1.

15. Unicellular organisms, such as Escherichia coli bacteria, each such organism containing one of the recombinant DNA vectors of claim 6, such as the lac promoter-vector-containing Escherichia coli bacterium, such as the one deposited with the NRRL and assigned accession No. B-15654; the trp-promoter-also-containing-the-trp-operator-vector-containing Escherichia coli bacterium; the trp-promoter-containing-the-trp-operator-and-the-lac-operator-vector-containing Escherichia coli bacterium, such as the one deposited with the NRRL and assigned accession No. B-15681; the tac-promoter-vector-containing Escherichia coli bacterium, such as the one deposited with the NRRL and assigned accession No. B-15662; and mutants, recombinants, and genetically-engineered derivatives thereof.

16. Unicellular organisms, such as Escherichia coli bacteria, each such organism containing one of the vectors of claim 7, such as the vector-containing Escherichia coli deposited with the NRRL and assigned accession No. B15655, B-15190, and B-15667; and the mutants, recombinants and genetically-engineered derivatives thereof.

17. Unicellular organisms, such as Escherichia coli bacteria, each such organisms containing one of the recombinant DNA vectors of claim 8, such as the recombinant-DNA-vector-containing Escherichia coli bacteria deposited with the NRRL and assigned accession No. B-15659, B-15668, B-15664; and the mutants, recombinants, and genetically-engineered derivatives thereof.

18. Unicellular organisms, such as Escherichia coli bacteria, each such organism containing one of the A/T rich DNA region vectors of claim 9, such as A/T-rich-DNA-region-vector-containing Escherichia coli bacteria deposited with the NRRL and assigned accession No. B-15657, B-15663, B-15658; and mutants, recombinants, and genetically-engineered derivatives thereof.

19. A unicellular organism, such as Escherichia coli bacterium, each such organism containing one of the recombinant DNA vectors of claim 10.

20. A unicellular organism, such as Escherichia coli bacterium, each such organism containing one of the recombinant DNA vectors of Claim 11.

21. Unicellular organisms, such as Escherichia coli bacteria, each such organism containing one of the recombinant DNA vectors of claim 12, such as the recombinant-DNA-vector-containing Escherichia coli bacteria deposited with the NRRL and assigned accession No. B-15656, B-15661; and mutants, recombinants, and genetically-engineered derivatives thereof.

22. Unicellular organisms, such as Escherichia coli bacteria, each such organism containing one of the recombinant DNA vectors of claim 13, such as the recombinant-DNA-vector-containing Escherichia coli bacteria deposited with the NRRL and assigned accession No. B-15670, B-15671, B-15674, B-15675, B-15676, B-15678; and mutants, recombinants, and genetically-engineered derivatives thereof.

23. Unicellular organisms, such as Escherichia coli bacteria, each such organism containing one of the recombinant DNA vectors of claim 14, such as the recombinant-DNA-vector-containing Escherichia coli bacteria deposited with the NRRL and assigned accession No. B-15669, B-15672, B-15673; and mutants, recombinants, and genetically-engineered derivatives thereof.

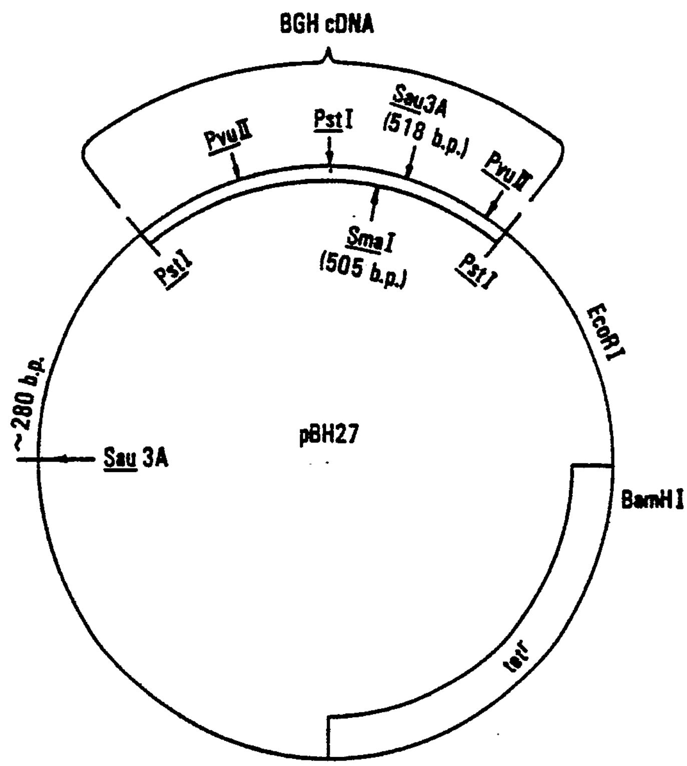
BGH cDNA
PvuII
PstI
Sau3A
(518 b.p.)
PvuII
PstI
SmaI
(505 b.p.)
PstI
EcoRI
~280 b.p.
Sau 3A
pBH27
BamHI
tet

FIG. 1

−30 −20 −10 LEADER SEQUENCE
(C)(G) PstI
AGGACTCAGGGTCCTGTGGACAGCTCACCAGCT ATG ATG GCT GCA GGC CCC CGG ACC TCC CTG 30
−26 Met Met Ala Ala Gly Pro Arg Thr Ser Leu

Start of mature BGH
BEX HaeII
CTC CTG GCT TTC GCC CTG CTC TGC CTG CCC TGG ACT CAG GTG GTG GGC GCC TTC CCA GCC 90
−16 Leu Leu Ala Phe Ala Leu Leu Cys Leu Pro Trp Thr Gln Val Val Gly Ala Phe Pro Ala

ATG N-37 (C) PruII
ATG TCC TTG TCC GGC CTG TTT GCC AAC GCT GTG CTC CGG GCT CAG CAT CTG CAC CAG CTG 150
5 Met Ser Leu Ser Gly Leu Phe Ala Asn Ala Val Leu Arg Ala Gln His Leu His Gln Leu

(U)
GCT GCT GAC ACC TTC AAA GAG TTT GAG CGC ACC TAC ATC CCG GAG GGA CAG AGA TAC TCC 210
25 Ala Ala Asp Thr Phe Lys Glu Phe Glu Arg Thr Tyr Ile Pro Glu Gly Gln Arg Tyr Ser

(C)
ATC CAG AAC ACC CAG GTT GCC TTC TGC TTC TCT GAA ACC ATC CCG GCC CCC ACG GGC AAG 270
45 Ile Gln Asn Thr Gln Val Ala Phe Cys Phe Ser Glu Thr Ile Pro Ala Pro Thr Gly Lys

AAT GAC GCC CAG CAG AAA TCA GAC TTG GAG CTG CTT CGC ATC TCA CTG CTC CTC ATC CAG 330
65 Asn Glu Ala Gln Gln Lys Ser Asp Leu Glu Leu Leu Arg Ile Ser Leu Leu Leu Ile Gln

PstI (U)
TCG TGG CTT GGG CCC CTG CAG TTC CTC AGC AGA GTC TTC ACC AAC AGC TTG GTG TTT GGC 390
85 Ser Trp Leu Gly Pro Leu Gln Phe Leu Ser Arg Val Phe Thr Asn Ser Leu Val Phe Gly

(U)
ACC TCG GAC CGT GTC TAT GAG AAG CTG AAG GAC CTG GAC GAA GGC ATC CTG GCC CTG ATG 450
105 Thr Ser Asp Arg Val Tyr Glu Lys Leu Lys Asp Leu Asp Glu Gly Ile Leu Ala Leu Met

SmaI Sau 3A
CGG GAC CTG GAA GAT GGC ACC CCC CGG GCT GGG CAG ATC CTC AAG CAG ACC TAT GAC AAA 510
125 Arg Asp Leu Glu Asp Gly Thr Pro Arg Ala Gly Gln Ile Leu Lys Gln Thr Tyr Asp Lys

TTT GAC ACA AAC ATG CGC AGT GAC GAC GCG CTG CTC AAG AAC TAC GGT CTG CTC TCC TGC 570
145 Phe Asp Thr Asn Met Arg Ser Asp Asp Ala Leu Leu Lys Asn Tyr Gly Leu Leu Ser Cys

RsaI
TTC CGG AAG GAC CTG CAT AAG ACG GAG ACG TAC CTG AGG GTC ATG AAG TGC CGC CGC TTC 630
165 Phe Arg Lys Asp Leu His Lys Thr Glu Thr Tyr Leu Arg Val Met Lys Cys Arg Arg Phe

PruII
GGG GAG GCC AGC TGT GCC TTC TAG TTGCCAGCCATCTGTTGTTTGCCCCTCCCCCGTGCCTTCCTTGACC 700
185 Gly Glu Ala Ser Cys Ala Phe ***

CTGGAAGGTGCCACTCCCACTGTCCTTTCCTAATAAAATGAGGAAATTGCATCGCAAAAAAA 762

FIG. 2

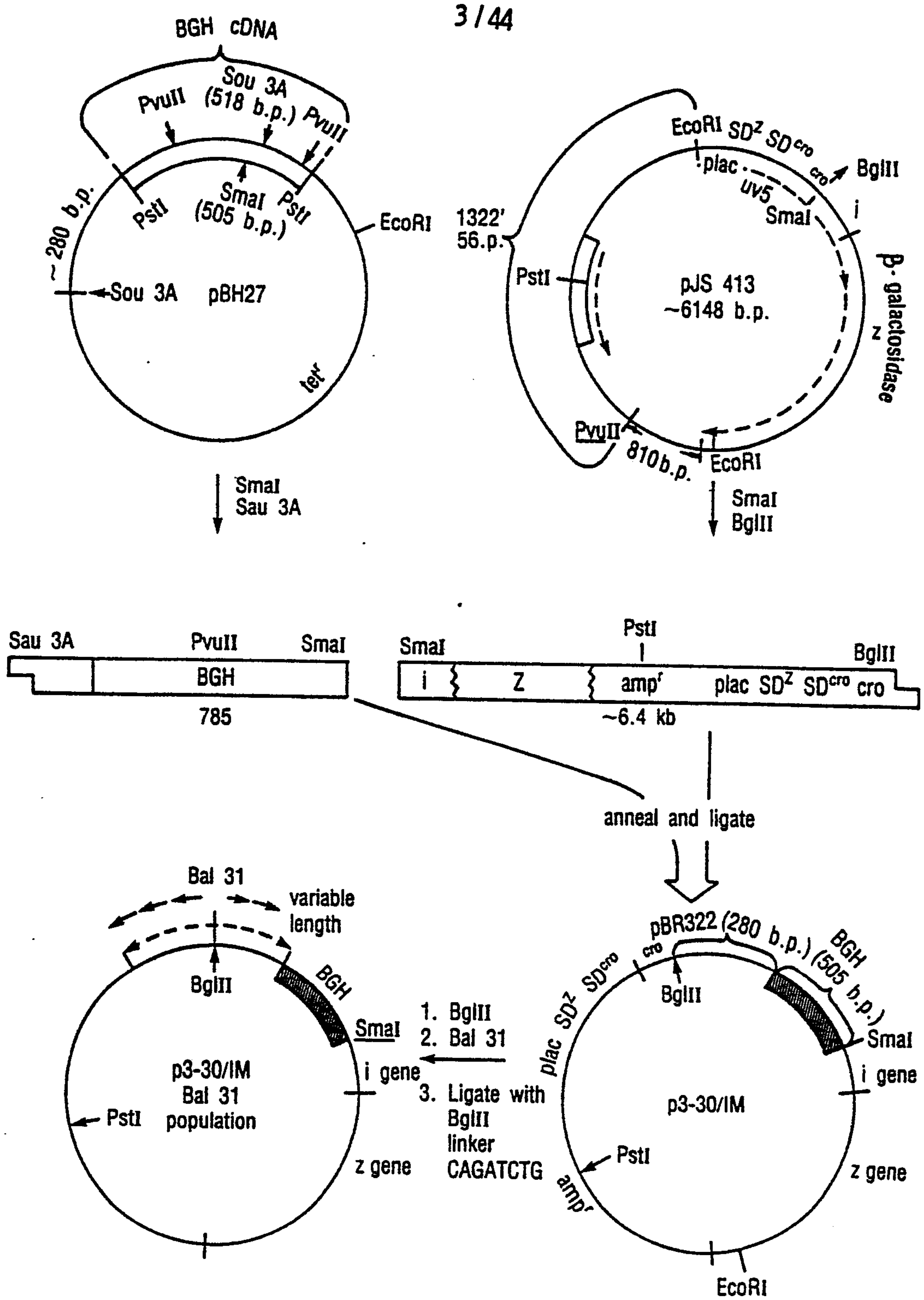
BGH cDNA
Sou 3A
PvuII
(518 b.p.)
PvuII
~280 b.p.
PstI
SmaI
(505 b.p.)
PstI
EcoRI
Sou 3A
pBH27
tet^r
SmaI
Sau 3A
EcoRI
SD^Z SD^cro
plac
cro
BglII
uv5
SmaI
i
1322'
56.p.
PstI
pJS 413
~6148 b.p.
β-galactosidase
z
PvuII
810b.p.
EcoRI
SmaI
BglII
Sau 3A
PvuII
SmaI
BGH
785
SmaI
PstI
BglII
i
Z
amp^r
plac SD^Z SD^cro cro
~6.4 kb
anneal and ligate
Bal 31
variable
length
BglII
BGH
SmaI
p3-30/IM
Bal 31
population
i gene
PstI
z gene
1. BglII
2. Bal 31
3. Ligate with
BglII
linker
CAGATCTG
pBR322 (280 b.p.)
BGH
(505 b.p.)
cro
SD^cro
plac SD^Z
BglII
SmaI
i gene
p3-30/IM
PstI
amp^r
z gene
EcoRI

FIG. 3

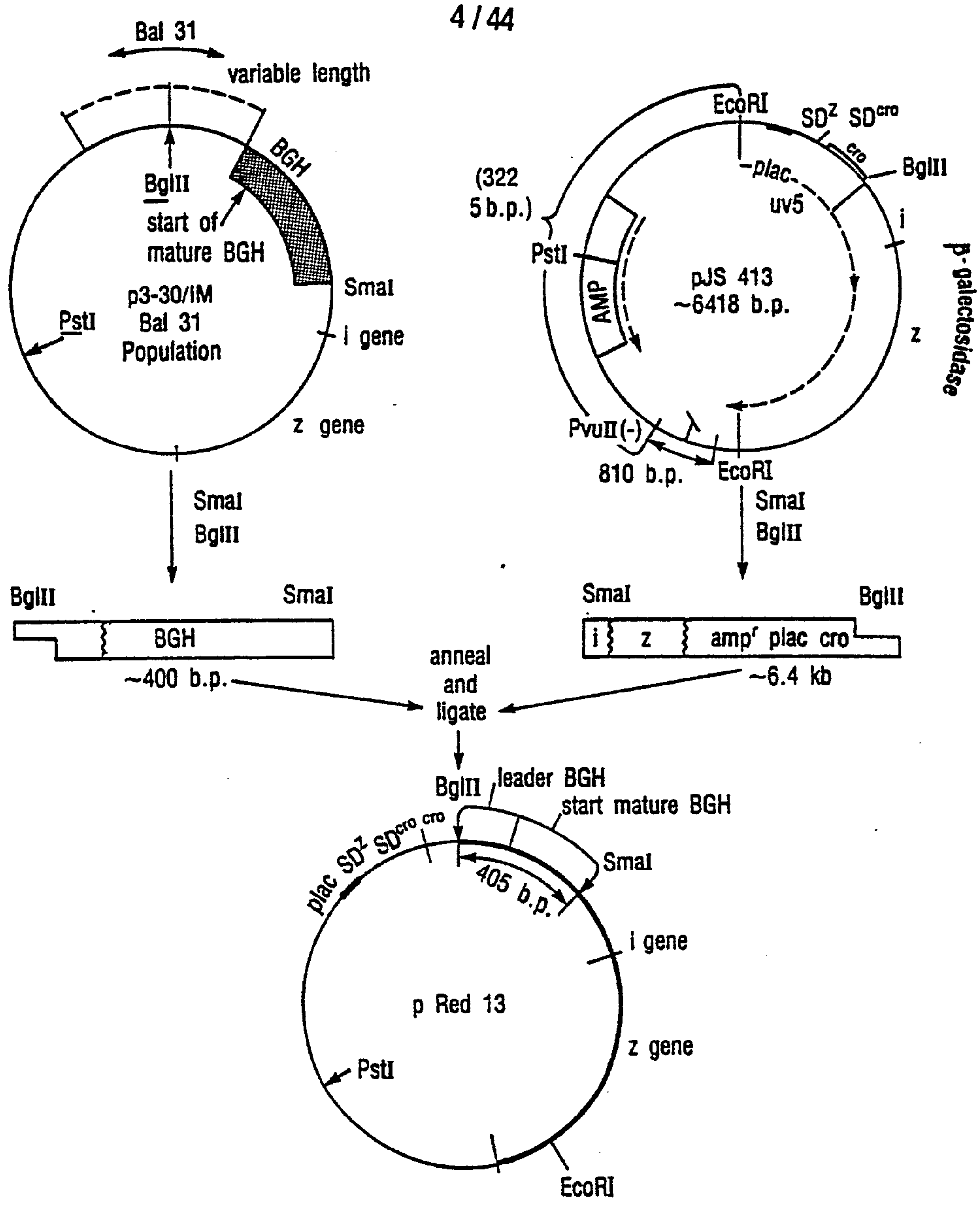
Bal 31
variable length
BGH
BglII
start of
mature BGH
SmaI
p3-30/IM
Bal 31
Population
PstI
i gene
z gene
SmaI
BglII
BglII
SmaI
BGH
~400 b.p.
EcoRI
SD^z SD^cro
cro
BglII
plac
uv5
(322
5 b.p.)
PstI
AMP
pJS 413
~6418 b.p.
i
z
β-galactosidase
PvuII(-)
810 b.p.
EcoRI
SmaI
BglII
SmaI
BglII
i
z
amp^r plac cro
~6.4 kb
anneal
and
ligate
BglII
leader BGH
start mature BGH
plac SD^z SD^cro cro
405 b.p.
SmaI
i gene
p Red 13
z gene
PstI
EcoRI

FIG. 4

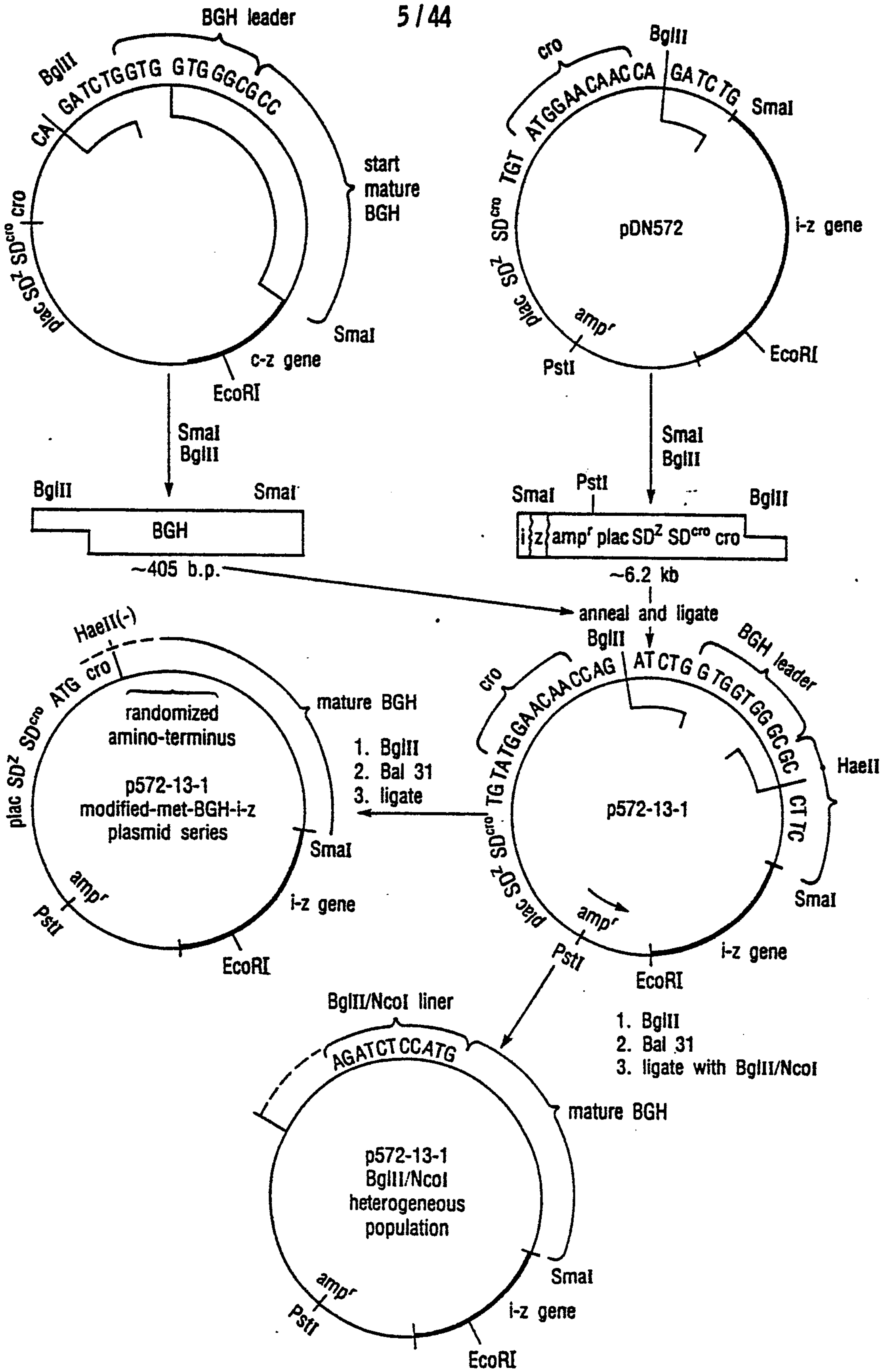
BGH leader
BglII
CA GATCTGGTG GTG GGCGCC
start mature BGH
plac SDz SDcro cro
c-z gene
SmaI
EcoRI
cro
BglII
TGT ATGGAACAACCA GATCTG
SmaI
pDN572
i-z gene
plac SDz SDcro
ampr
PstI
EcoRI
SmaI
BglII
SmaI
BglII
BglII
SmaI
BGH
~405 b.p.
SmaI
PstI
BglII
i z ampr plac SDz SDcro cro
~6.2 kb
anneal and ligate
HaeII(-)
ATG cro
randomized amino-terminus
mature BGH
plac SDz SDcro
p572-13-1
modified-met-BGH-i-z
plasmid series
SmaI
ampr
PstI
i-z gene
EcoRI
1. BglII
2. Bal 31
3. ligate
BglII
cro
TGTATGGAACAACCAG ATCTG GTGGTGGGCGC CTTC
BGH leader
HaeII
p572-13-1
plac SDz SDcro
ampr
SmaI
PstI
i-z gene
EcoRI
1. BglII
2. Bal 31
3. ligate with BglII/NcoI
BglII/NcoI liner
AGATCTCCATG
mature BGH
p572-13-1
BglII/NcoI
heterogeneous
population
SmaI
ampr
PstI
i-z gene
EcoRI

FIG. 5

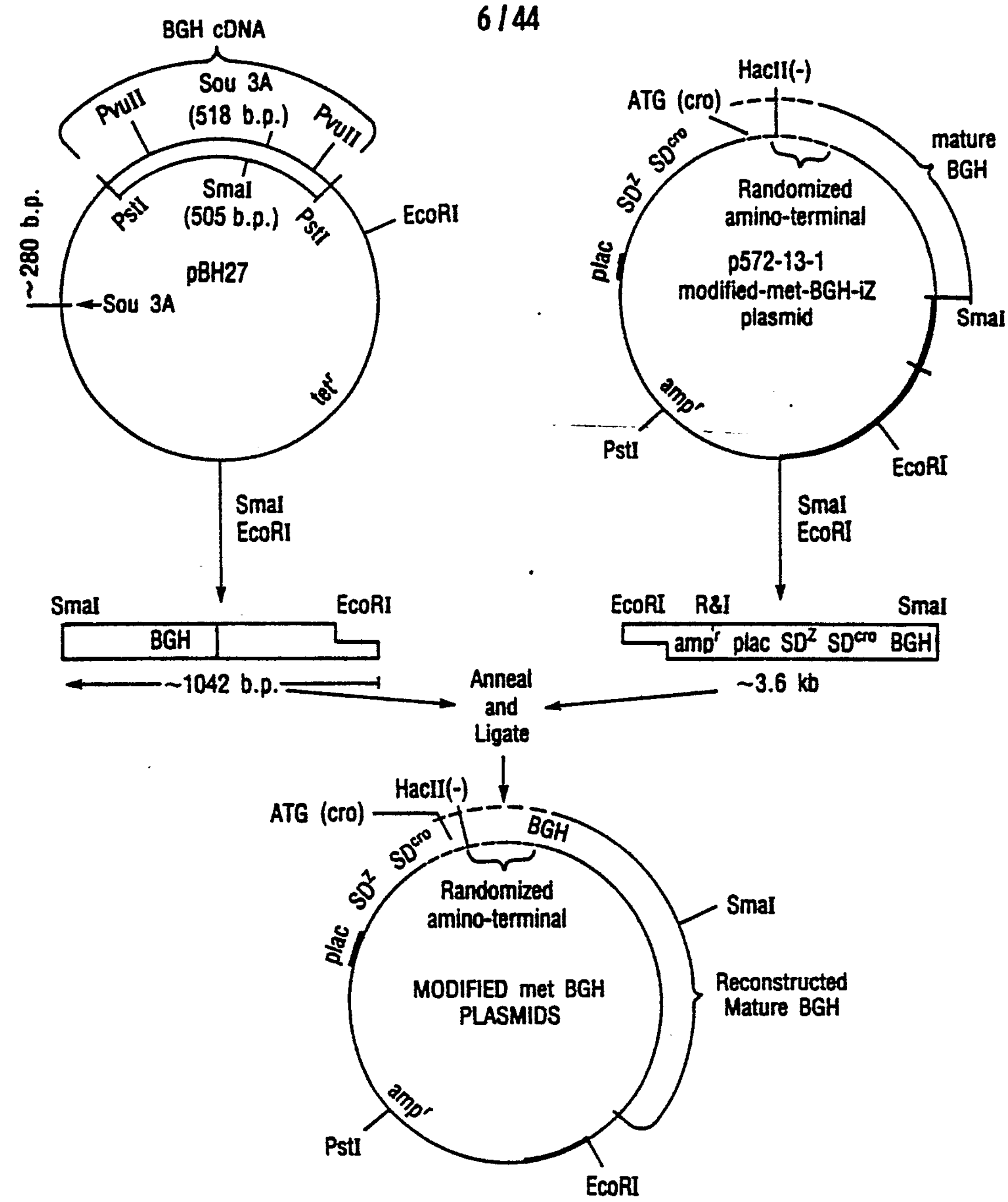
BGH cDNA
Sou 3A
(518 b.p.)
PvuII
PvuII
PstI
SmaI
(505 b.p.)
PstI
EcoRI
~280 b.p.
pBH27
Sou 3A
tet^r
HacII(-)
ATG (cro)
mature BGH
SD^z
SD^cro
Randomized amino-terminal
plac
p572-13-1 modified-met-BGH-iZ plasmid
SmaI
amp^r
PstI
EcoRI
SmaI
EcoRI
SmaI
EcoRI
SmaI
EcoRI
BGH
~1042 b.p.
EcoRI
R&I
SmaI
amp^r plac SD^Z SD^cro BGH
~3.6 kb
Anneal and Ligate
HacII(-)
ATG (cro)
BGH
SD^cro
SD^Z
Randomized amino-terminal
plac
SmaI
MODIFIED met BGH PLASMIDS
Reconstructed Mature BGH
amp^r
PstI
EcoRI

FIG. 6

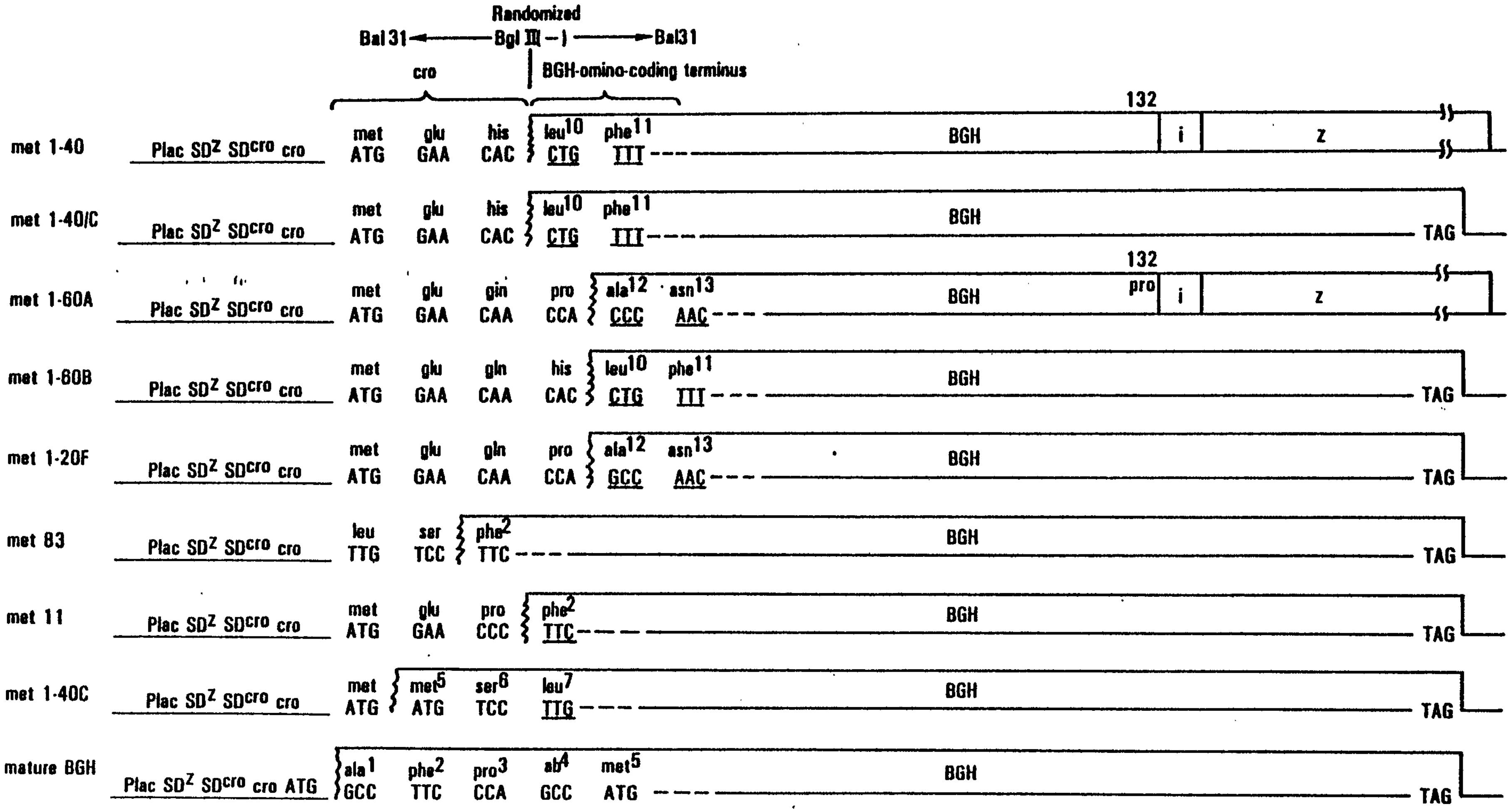

Randomized
Bal31 ← Bgl III (--) → Bal31
cro
BGH-amino-coding terminus
132
met 1-40
Plac SD^Z SD^cro cro
met ATG glu GAA his CAC leu10 CTG phe11 TTT
BGH i z
met 1-40/C
Plac SD^Z SD^cro cro
met ATG glu GAA his CAC leu10 CTG phe11 TTT
BGH TAG
met 1-60A
Plac SD^Z SD^cro cro
met ATG glu GAA gln CAA pro CCA ala12 CCC asn13 AAC
BGH pro i z
met 1-60B
Plac SD^Z SD^cro cro
met ATG glu GAA gln CAA his CAC leu10 CTG phe11 TTT
BGH TAG
met 1-20F
Plac SD^Z SD^cro cro
met ATG glu GAA gln CAA pro CCA ala12 GCC asn13 AAC
BGH TAG
met 83
Plac SD^Z SD^cro cro
leu TTG ser TCC phe2 TTC
BGH TAG
met 11
Plac SD^Z SD^cro cro
met ATG glu GAA pro CCC phe2 TTC
BGH TAG
met 1-40C
Plac SD^Z SD^cro cro
met ATG met5 ATG ser6 TCC leu7 TTG
BGH TAG
mature BGH
Plac SD^Z SD^cro cro ATG
ala1 GCC phe2 TTC pro3 CCA ala4 GCC met5 ATG
BGH TAG


FIG. 7

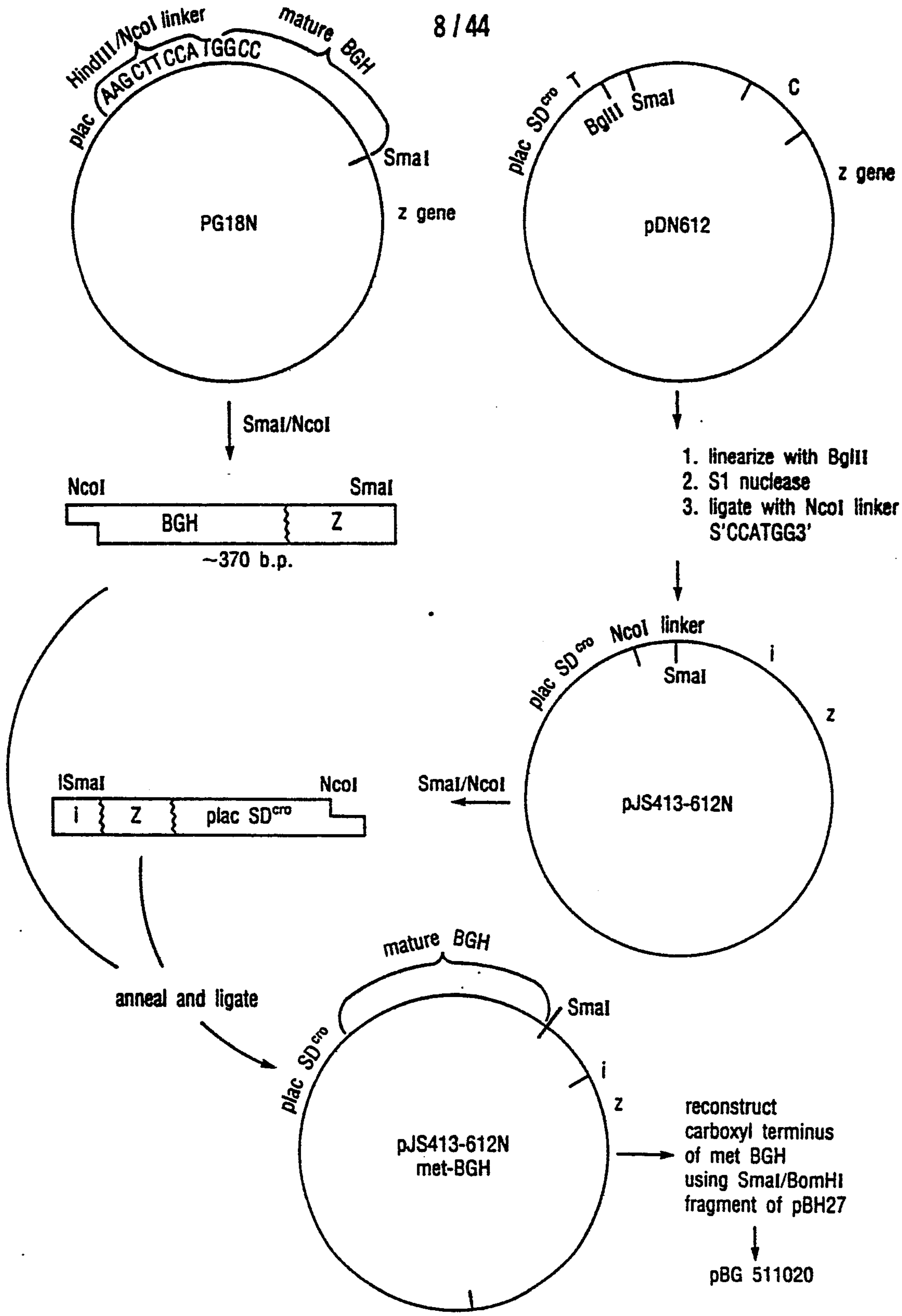
HindIII/NcoI linker
AAGCTTCCATGGCC
mature BGH
plac
SmaI
PG18N
z gene
plac SD^cro T
BglII SmaI
C
z gene
pDN612
SmaI/NcoI
NcoI
SmaI
BGH
Z
~370 b.p.
1. linearize with BglII
2. S1 nuclease
3. ligate with NcoI linker
S'CCATGG3'
plac SD^cro NcoI linker
SmaI
i
z
pJS413-612N
SmaI/NcoI
ISmaI
NcoI
i
Z
plac SD^cro
anneal and ligate
mature BGH
SmaI
plac SD^cro
i
z
pJS413-612N
met-BGH
reconstruct
carboxyl terminus
of met BGH
using SmaI/BomHI
fragment of pBH27
pBG 511020

FIG. 8A

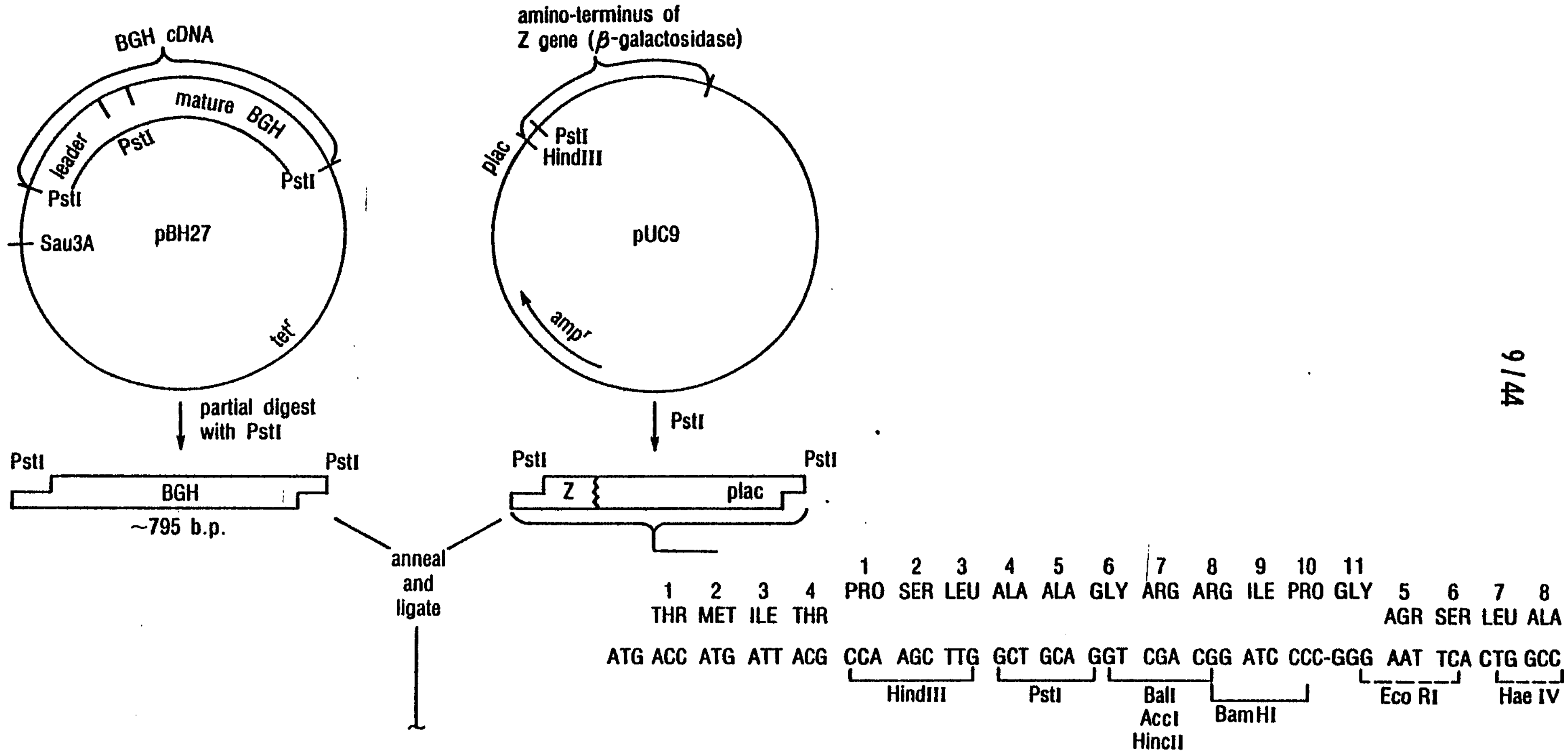
BGH cDNA
leader
mature BGH
PstI
PstI
PstI
Sau3A
pBH27
tet^r
partial digest with PstI
PstI
PstI
BGH
~795 b.p.
amino-terminus of Z gene (β-galactosidase)
PstI
HindIII
plac
pUC9
amp^r
PstI
PstI
PstI
Z
plac
anneal and ligate
1 2 3 4 5 6 7 8 9 10 11
PRO SER LEU ALA ALA GLY ARG ARG ILE PRO GLY
1 2 3 4
THR MET ILE THR
5 6 7 8
AGR SER LEU ALA
ATG ACC ATG ATT ACG CCA AGC TTG GCT GCA GGT CGA CGG ATC CCC-GGG AAT TCA CTG GCC
HindIII
PstI
BalI
AccI
HincII
BamHI
Eco RI
Hae IV

FIG. 8I

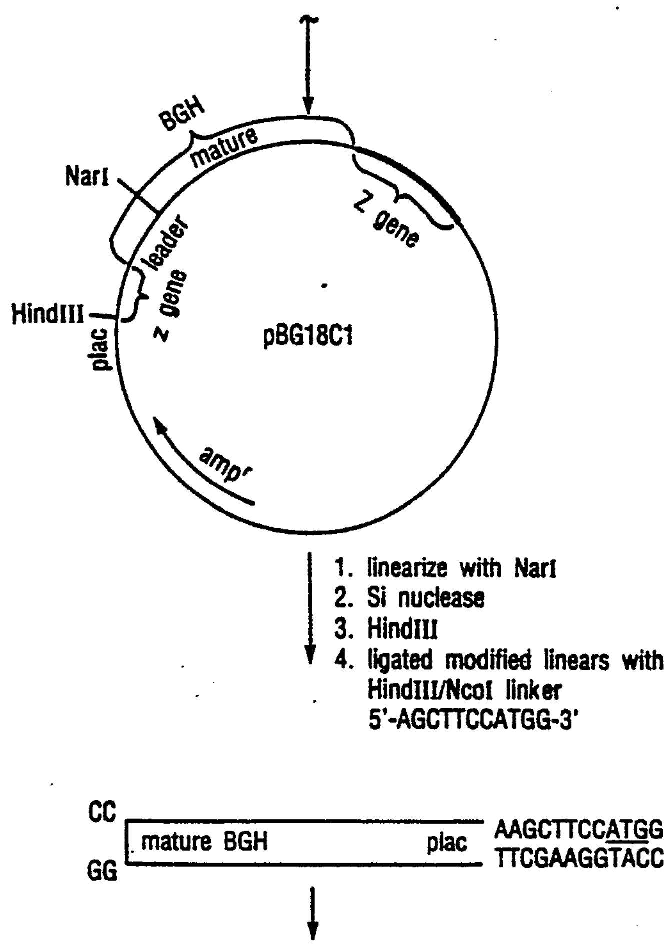
BGH
mature
NarI
leader
z gene
HindIII
plac
Z gene
pBG18C1
amp^r
1. linearize with NarI
2. Si nuclease
3. HindIII
4. ligated modified linears with
HindIII/NcoI linker
5'-AGCTTCCATGG-3'
CC
GG
mature BGH
plac
AAGCTTCCATGG
TTCGAAGGTACC

FIG. 8 II

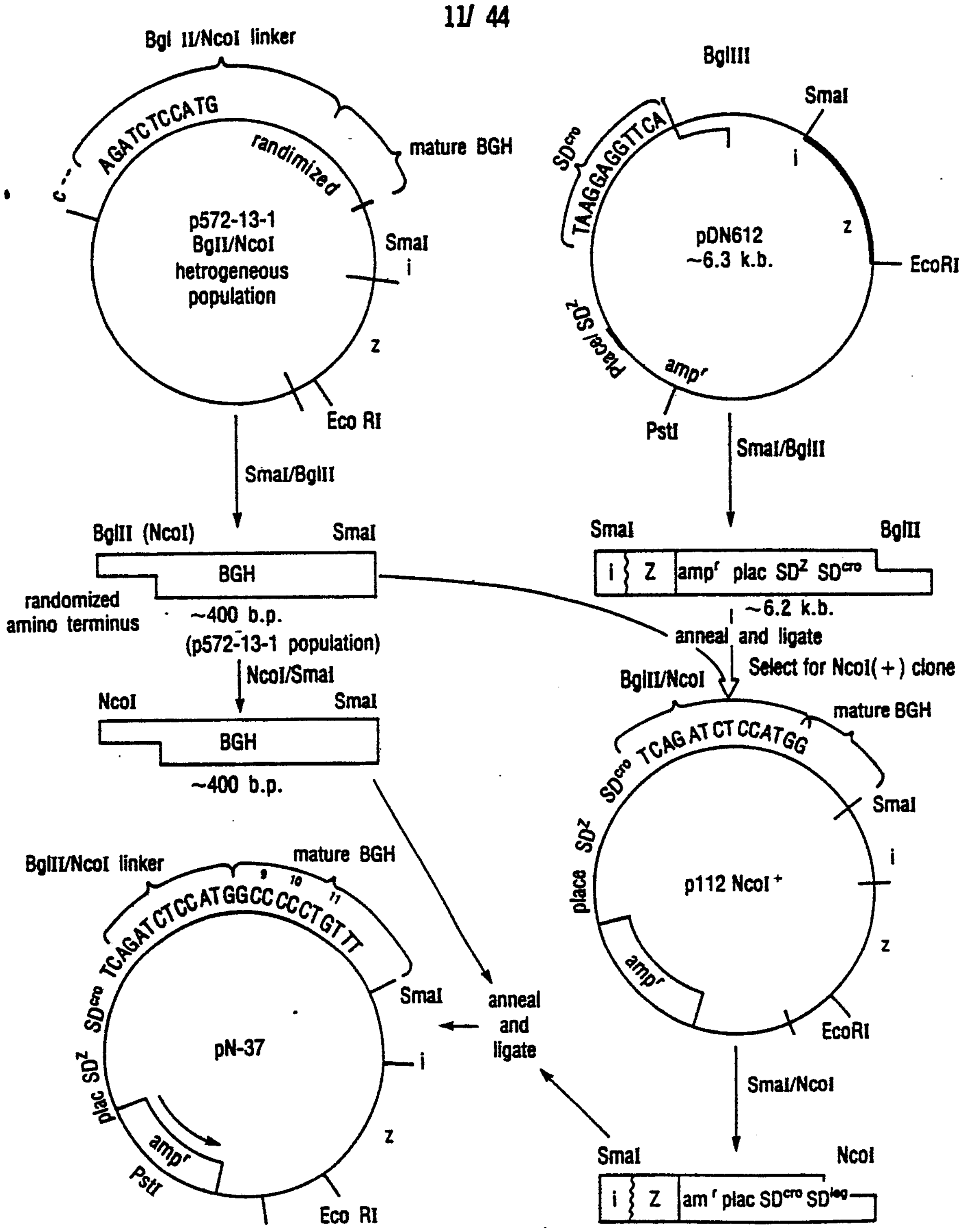

Bgl II/NcoI linker
AGATCTCCATG
c
randimized
mature BGH
p572-13-1
BglI/NcoI
hetrogeneous
population
SmaI
i
z
Eco RI
SmaI/BglII
BglII (NcoI)
SmaI
BGH
randomized
amino terminus
~400 b.p.
(p572-13-1 population)
NcoI/SmaI
NcoI
SmaI
BGH
~400 b.p.
BglII
SmaI
SDcro
TAAGGAGGTTCA
pDN612
~6.3 k.b.
EcoRI
place/SDz
ampr
PstI
SmaI/BglII
SmaI
BglII
i
Z
ampr plac SDz SDcro
~6.2 k.b.
anneal and ligate
Select for NcoI(+) clone
BglII/NcoI
mature BGH
SDcro TCAGATCTCCATGG
SmaI
i
p112 NcoI+
placz SDz
ampr
z
EcoRI
SmaI/NcoI
SmaI
NcoI
i
Z
amr plac SDcro SDleg
anneal
and
ligate
BglII/NcoI linker
mature BGH
9
10
11
SDcro TCAGATCTCCATGGCCCCCTGTTT
SmaI
pN-37
i
plac SDz
ampr
PstI
z
Eco RI


FIG. 9

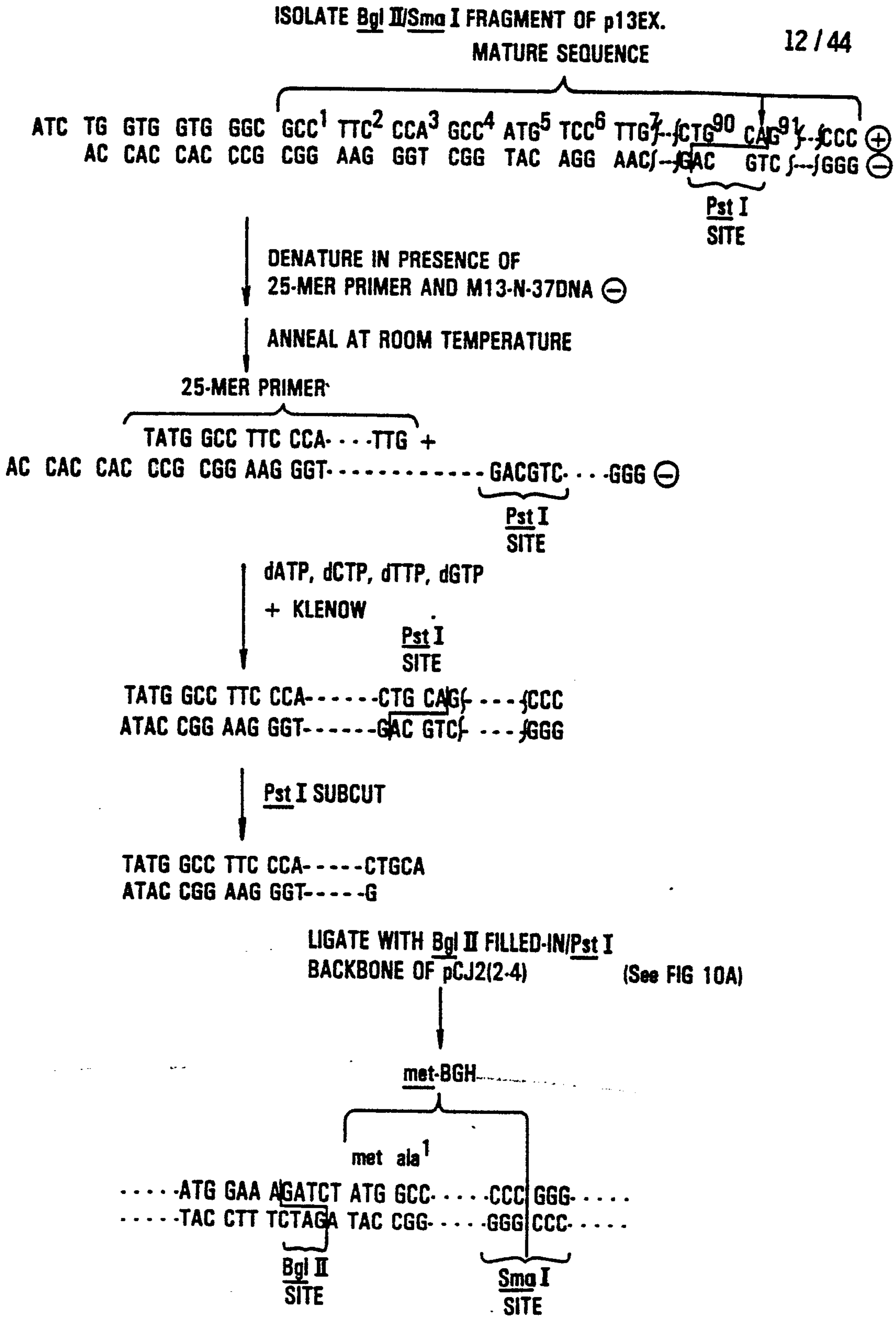
ISOLATE Bgl II/Sma I FRAGMENT OF p13EX.
MATURE SEQUENCE
ATC TG GTG GTG GGC GCC1 TTC2 CCA3 GCC4 ATG5 TCC6 TTG7 CTG90 CAG91 CCC ⊕
AC CAC CAC CCG CGG AAG GGT CGG TAC AGG AAC GAC GTC GGG ⊖
Pst I SITE
DENATURE IN PRESENCE OF
25-MER PRIMER AND M13-N-37DNA ⊖
ANNEAL AT ROOM TEMPERATURE
25-MER PRIMER
TATG GCC TTC CCA- - - -TTG +
AC CAC CAC CCG CGG AAG GGT- - - - - - - - - - - - GACGTC- - - -GGG ⊖
Pst I SITE
dATP, dCTP, dTTP, dGTP
+ KLENOW
Pst I SITE
TATG GCC TTC CCA- - - - - -CTG CAG - - - - CCC
ATAC CGG AAG GGT- - - - - -GAC GTC - - - - GGG
Pst I SUBCUT
TATG GCC TTC CCA- - - - -CTGCA
ATAC CGG AAG GGT- - - - -G
LIGATE WITH Bgl II FILLED-IN/Pst I
BACKBONE OF pCJ2(2-4)
(See FIG 10A)
met-BGH
met ala1
- - - - -ATG GAA AGATCT ATG GCC- - - - -CCC GGG- - - - -
- - - - -TAC CTT TCTAGA TAC CGG- - - - -GGG CCC- - - - -
Bgl II SITE
Sma I SITE

FIG. 10

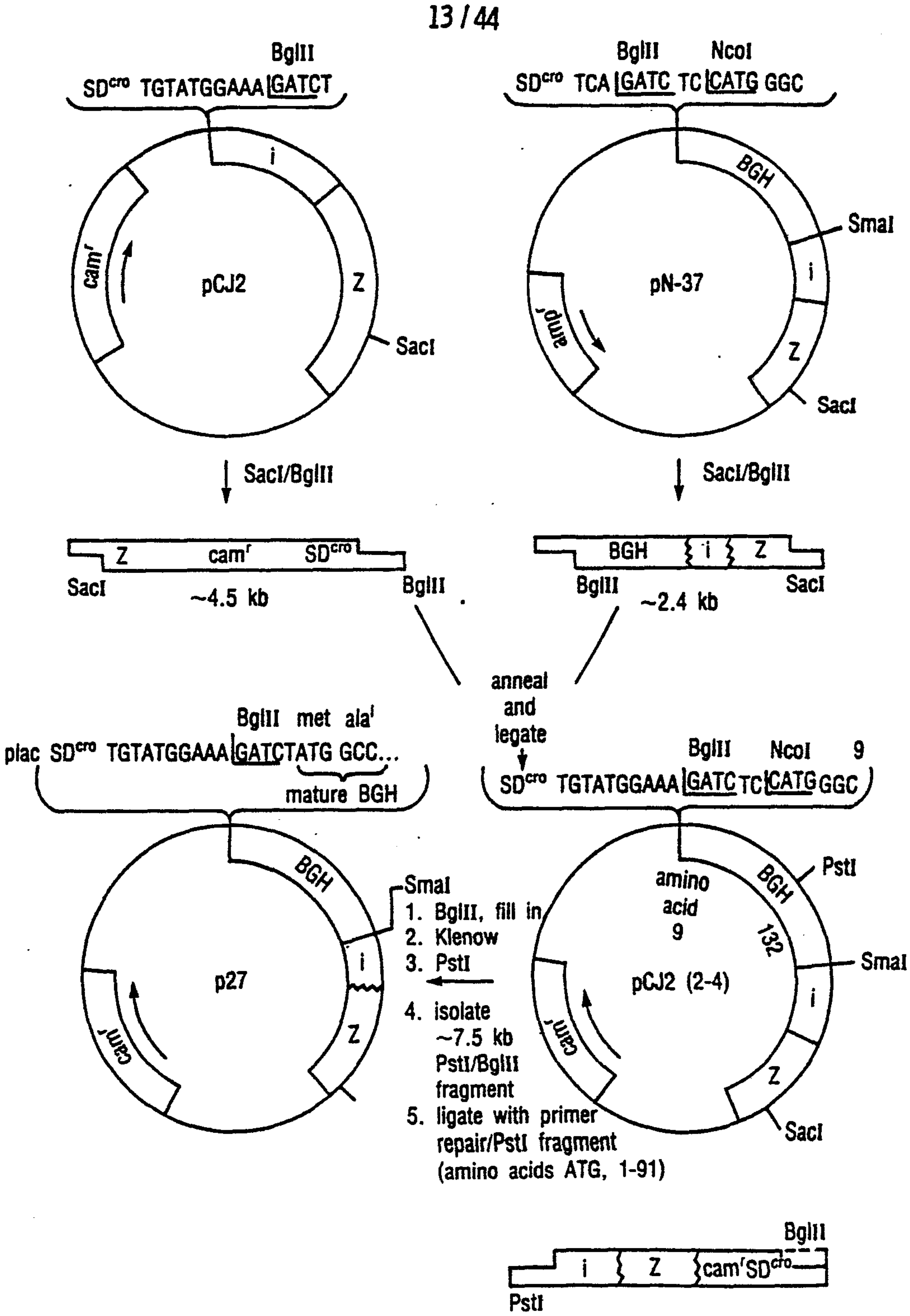

BglII
SD$^{cro}$ TGTATGGAAA GATCT
i
cam$^r$
pCJ2
Z
SacI
BglII NcoI
SD$^{cro}$ TCA GATC TC CATG GGC
BGH
SmaI
i
amp$^r$
pN-37
Z
SacI
SacI/BglII
SacI/BglII
Z cam$^r$ SD$^{cro}$
SacI ~4.5 kb BglII
BGH i Z
BglII ~2.4 kb SacI
anneal and legate
BglII met ala$^1$
plac SD$^{cro}$ TGTATGGAAA GATCTATG GCC...
mature BGH
BGH
SmaI
i
p27
Z
cam$^r$
1. BglII, fill in
2. Klenow
3. PstI
4. isolate ~7.5 kb PstI/BglII fragment
5. ligate with primer repair/PstI fragment (amino acids ATG, 1-91)
BglII NcoI 9
SD$^{cro}$ TGTATGGAAA GATC TC CATG GGC
amino acid 9
BGH
PstI
132
SmaI
pCJ2 (2-4)
i
cam$^r$
Z
SacI
BglII
i Z cam$^r$SD$^{cro}$
PstI


FIG. 10A

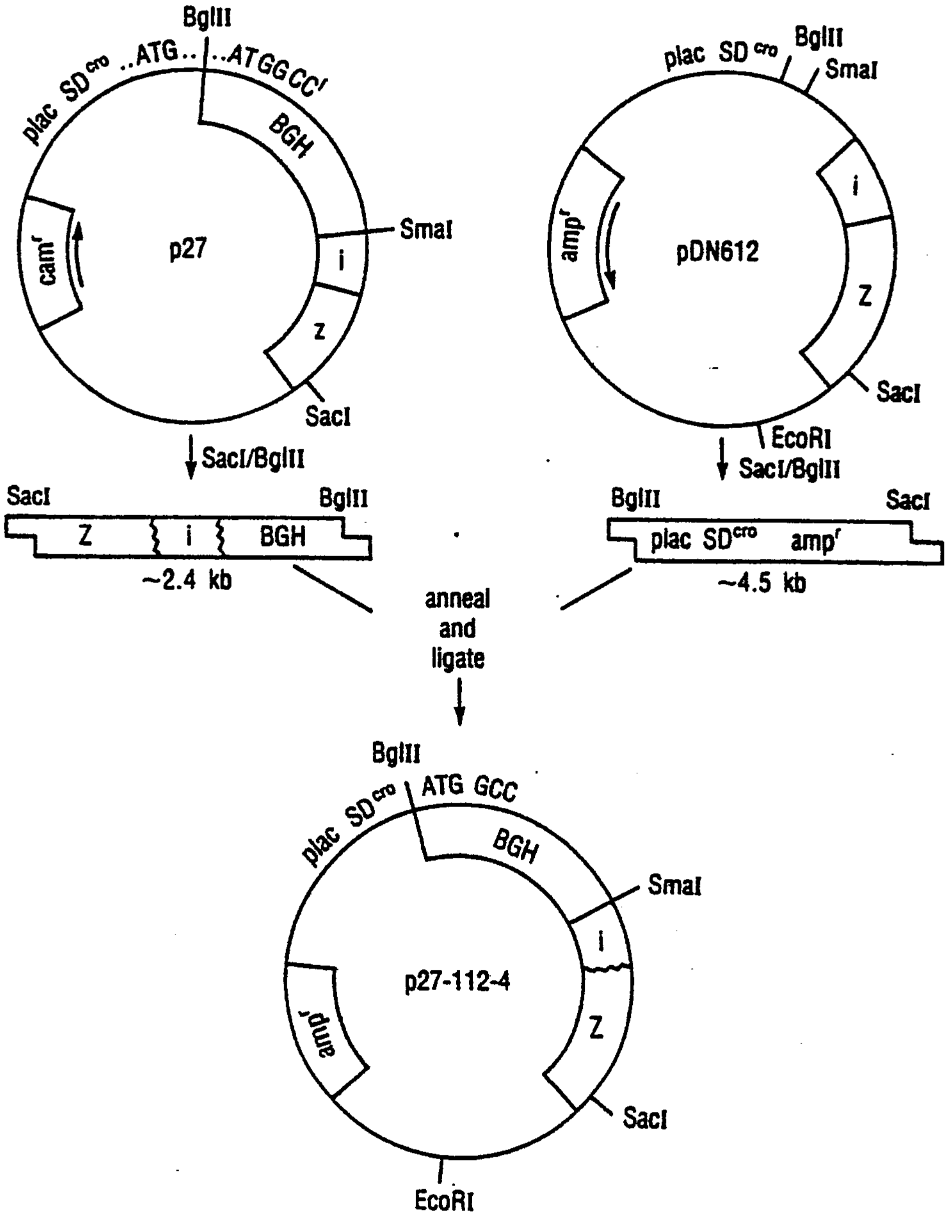
BglII
plac SD^cro ..ATG.. ..ATGGCC'
BGH
SmaI
i
z
SacI
cam^r
p27
SacI/BglII
SacI
BglII
Z
i
BGH
~2.4 kb
plac SD cro
BglII
SmaI
i
amp^r
pDN612
Z
SacI
EcoRI
SacI/BglII
BglII
SacI
plac SD^cro
amp^r
~4.5 kb
anneal
and
ligate
BglII
plac SD^cro
ATG GCC
BGH
SmaI
i
Z
SacI
p27-112-4
amp^r
EcoRI

FIG. 10B

SYNTHETIC FRONT END

Reasons for change:

1. Replace "non-preferred" codons with "preferred"

2. Secondary Structure predictions of the mRNA

| | | | | | | | | | | | | |
|---|---|---|---|---|---|---|---|---|---|---|---|---|
| SFE | ATG | GCT | TTC | CCG | GCT | ATG | TCT | CTG | TCT | GGT | CTG | TTC |
| NFE | ATG | GCC | TTC | CCA | GCC | ATG | TCC | TTG | TCC | GGC | CTG | TTT |
| SFE | GCT | AAC | GCT | GTT | CTG | CGT | GCT | CAG | CAC | CTG | CAC | CAG |
| NFE | GCC | AAC | GCT | GTG | CTC | CGG | GCT | CAG | CAC | CTG | CAT | CAG |

*FIG. 11*

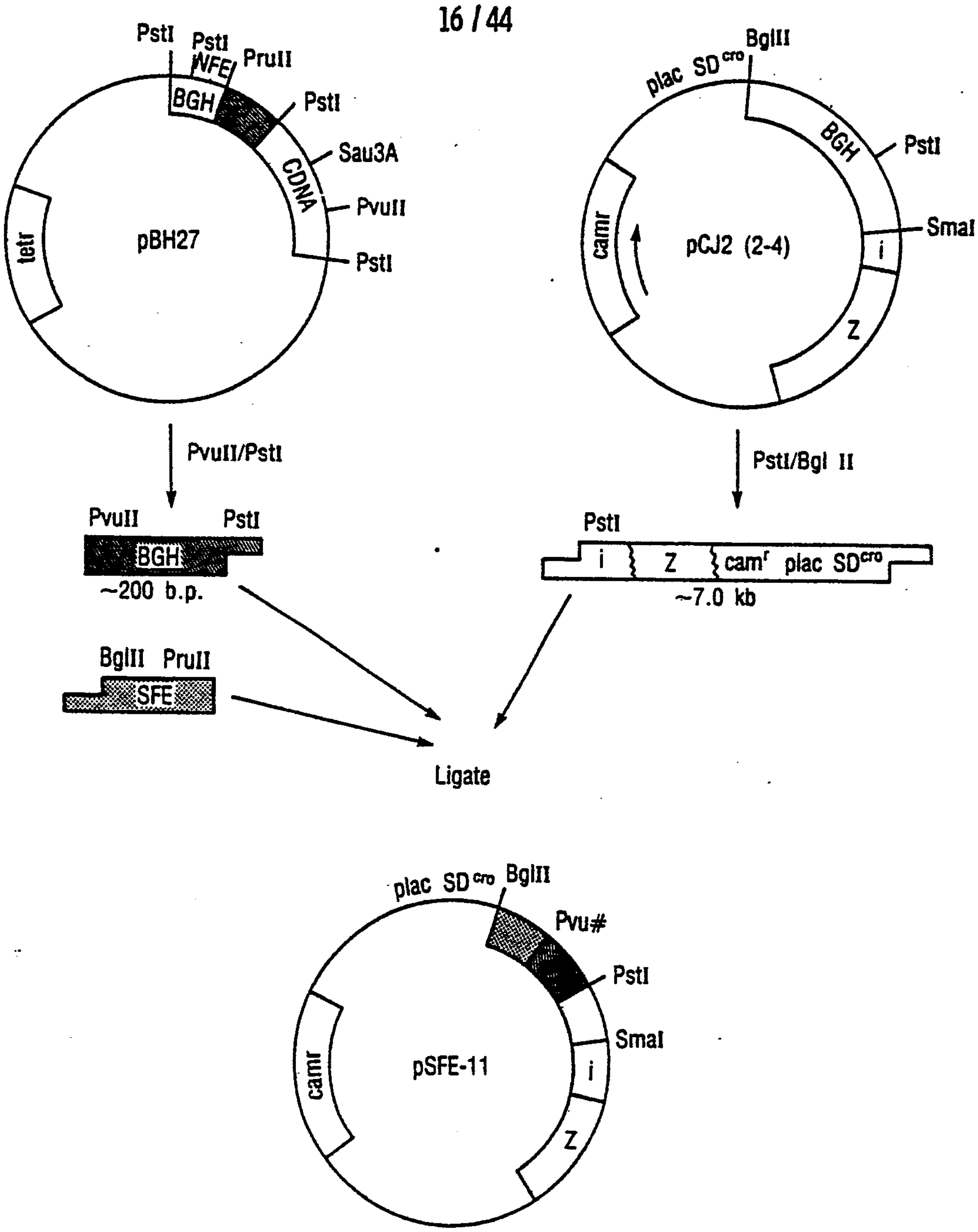
PstI
PstI
WFE
PruII
BGH
PstI
Sau3A
CDNA
PvuII
PstI
tetr
pBH27
plac SD cro
BglII
BGH
PstI
SmaI
camr
pCJ2 (2-4)
i
Z
PvuII/PstI
PstI/Bgl II
PvuII
PstI
BGH
~200 b.p.
PstI
i
Z
camr plac SDcro
~7.0 kb
BglII PruII
SFE
Ligate
plac SDcro BglII
Pvu#
PstI
SmaI
camr
pSFE-11
i
Z

FIG. 12

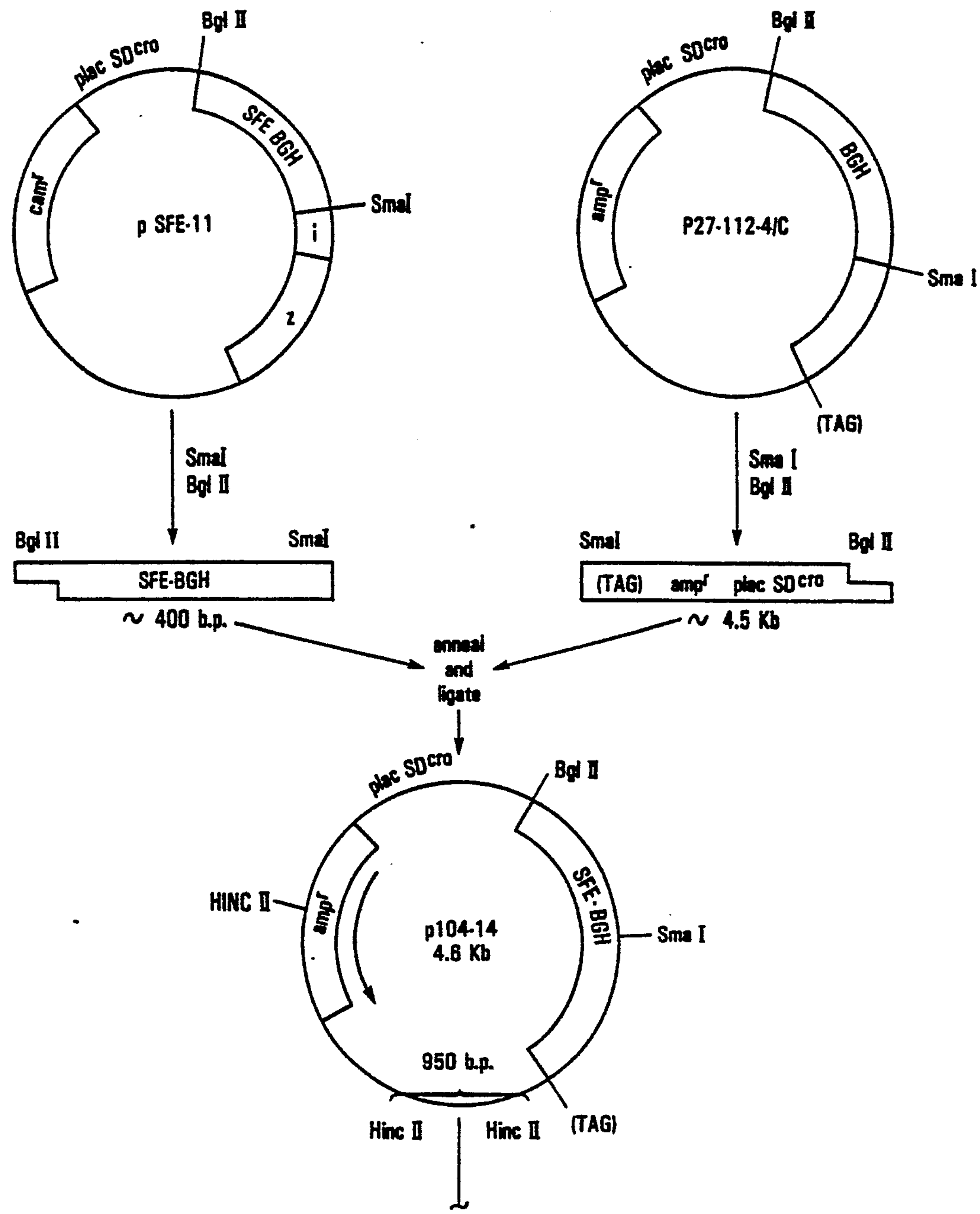
Bgl II
plac SDcro
SFE BGH
SmaI
camr
p SFE-11
i
z
Bgl II
plac SDcro
BGH
ampr
P27-112-4/C
Sma I
(TAG)
SmaI
Bgl II
Sma I
Bgl II
Bgl II
SmaI
SFE-BGH
~ 400 b.p.
SmaI
Bgl II
(TAG) ampr plac SD cro
~ 4.5 Kb
anneal
and
ligate
plac SDcro
Bgl II
HINC II
ampr
p104-14
4.6 Kb
SFE-BGH
Sma I
950 b.p.
Hinc II
Hinc II
(TAG)

FIG. 12A

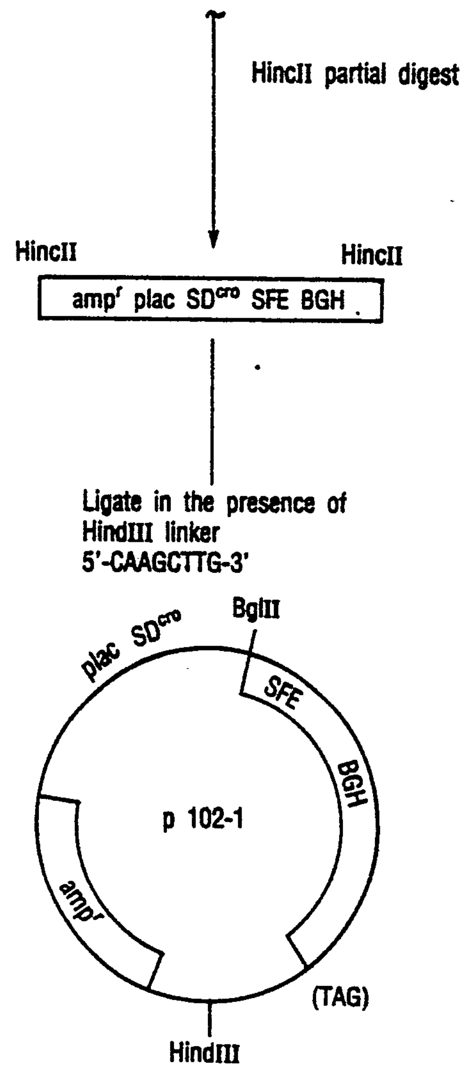

HincII partial digest
HincII
HincII
amp$^r$ plac SD$^{cro}$ SFE BGH
Ligate in the presence of
HindIII linker
5'-CAAGCTTG-3'
plac SD$^{cro}$
BglII
SFE
BGH
p 102-1
amp$^r$
(TAG)
HindIII


FIG. 12B

```
                 -35                      -10           1                     SDtrp
Ptrp     GAGCTGTTGACAATTAATCATCGAACTAGTTAACTAGTACGCAAGTTCACGTAAAAAGGGTA

                 -35                      -10           1                                     SDlac
PlacUV5  CCAGGCTTTACACTTTATGCTTCCGGCTCGTATAATGTGTGGAATTGTGAGCGGATAACAATTTCACACAGGAAA

                 -35                      -10           1                                     SDlac
Ptac     GAGCTGTTGACAATTAATCATCGGCTCGTATAATGTGTGGAATTGTGAGCGGATAACAATTTCACACAGGAAA
```

FIG. 13

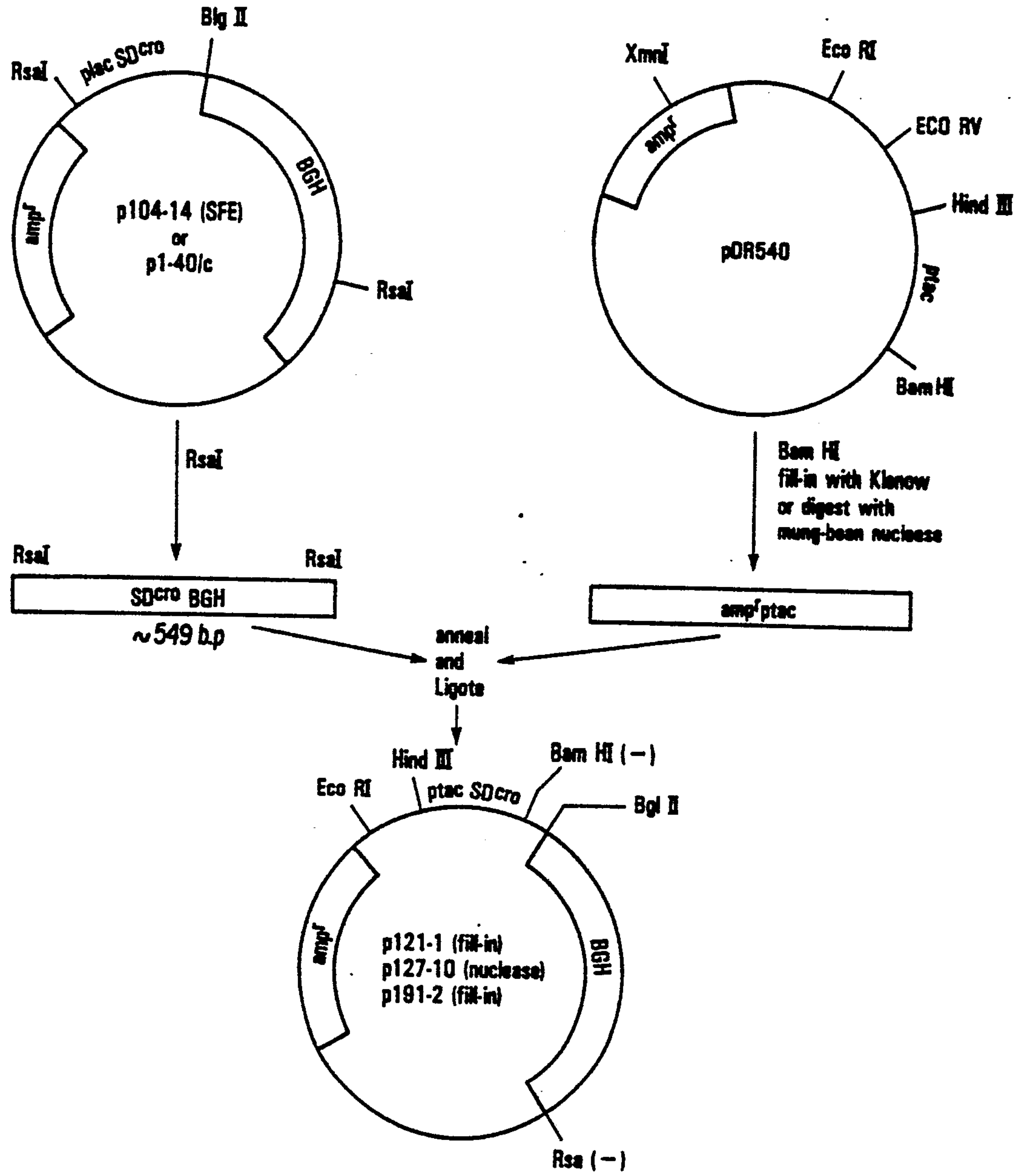
Blg II
RsaI
plac SDcro
BGH
ampr
p104-14 (SFE)
or
p1-40/c
RsaI
XmnI
Eco RI
ECO RV
Hind III
ampr
pDR540
ptac
Bam HI
RsaI
Bam HI
fill-in with Klenow
or digest with
mung-bean nuclease
RsaI
RsaI
SDcro BGH
~549 b.p
amprptac
anneal
and
Ligate
Hind III
Eco RI
ptac SDcro
Bam HI (−)
Bgl II
ampr
p121-1 (fill-in)
p127-10 (nuclease)
p191-2 (fill-in)
BGH
Rsa (−)

FIG. 14

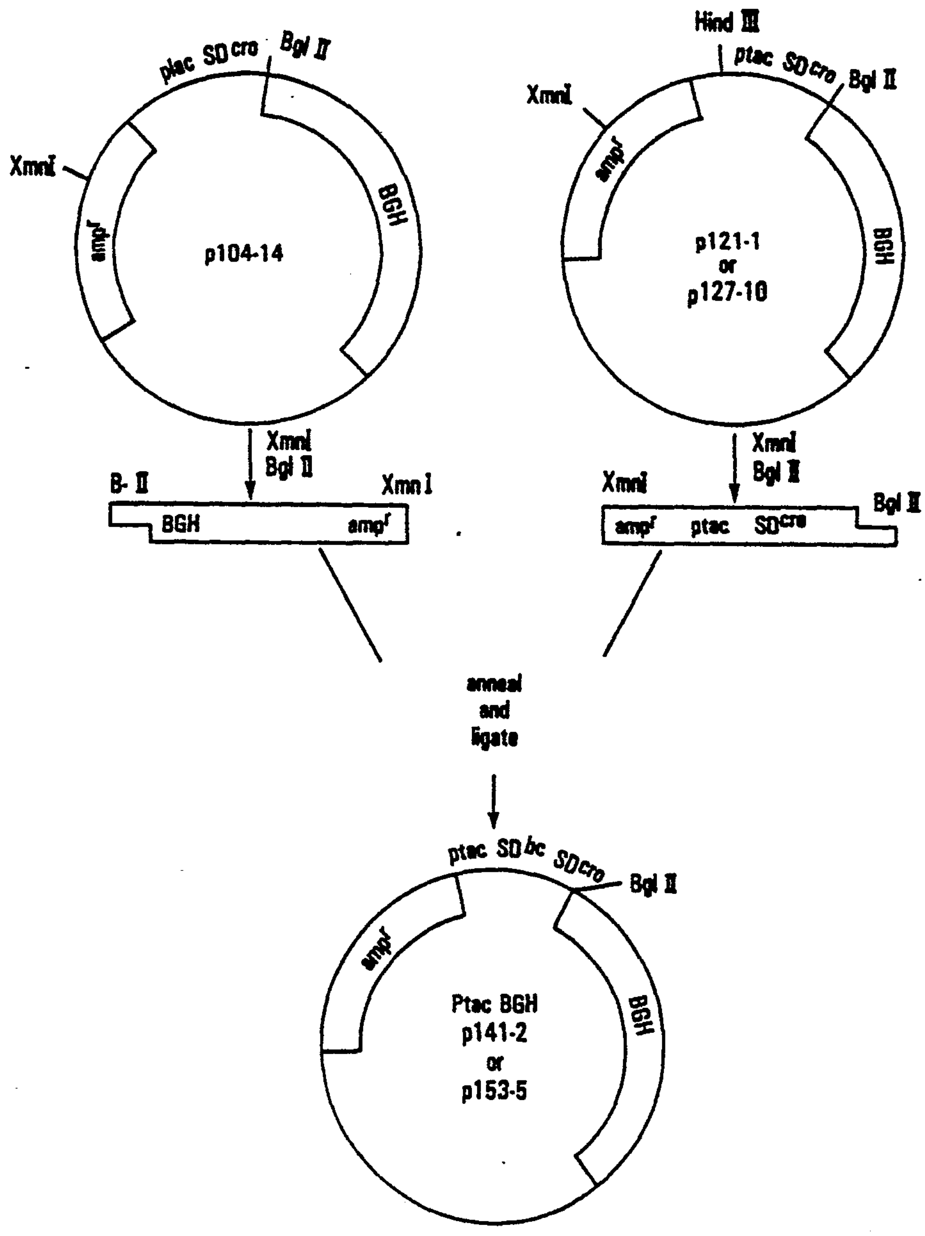
plac SDcro BglII
XmnI
ampr
p104-14
BGH
Hind III
ptac SDcro BglII
XmnI
ampr
p121-1
or
p127-10
BGH
XmnI
BglII
B-II
BGH
ampr
XmnI
XmnI
BglII
XmnI
ampr ptac SDcro
BglII
anneal
and
ligate
ptac SDbc SDcro
BglII
ampr
Ptac BGH
p141-2
or
p153-5
BGH

FIG. 14A

SDlac SDcro Bg III
p141-1 ptac------AGGAACAGGATCACTAAGGAGGTTCAGATCT ATG----BGH

SDlac SDcro Bg III
p153-1 ptac------AGGAAACAGACTAAGGAGGTTCAGATCT-ATG----BGH

FIG. 14 B

A/T rich replacement

Bg III
p102-1 SDcro-TCAGATCT-ATG-BGH

Aha III
p128-7/2 SDcro-TCAAATTTAAATT-ATG-BGH

p146-1 SDcro-TCAAAATT-ATG-BGH

FIG. 16

INSERTION OF A/T RICH SEQUENCES



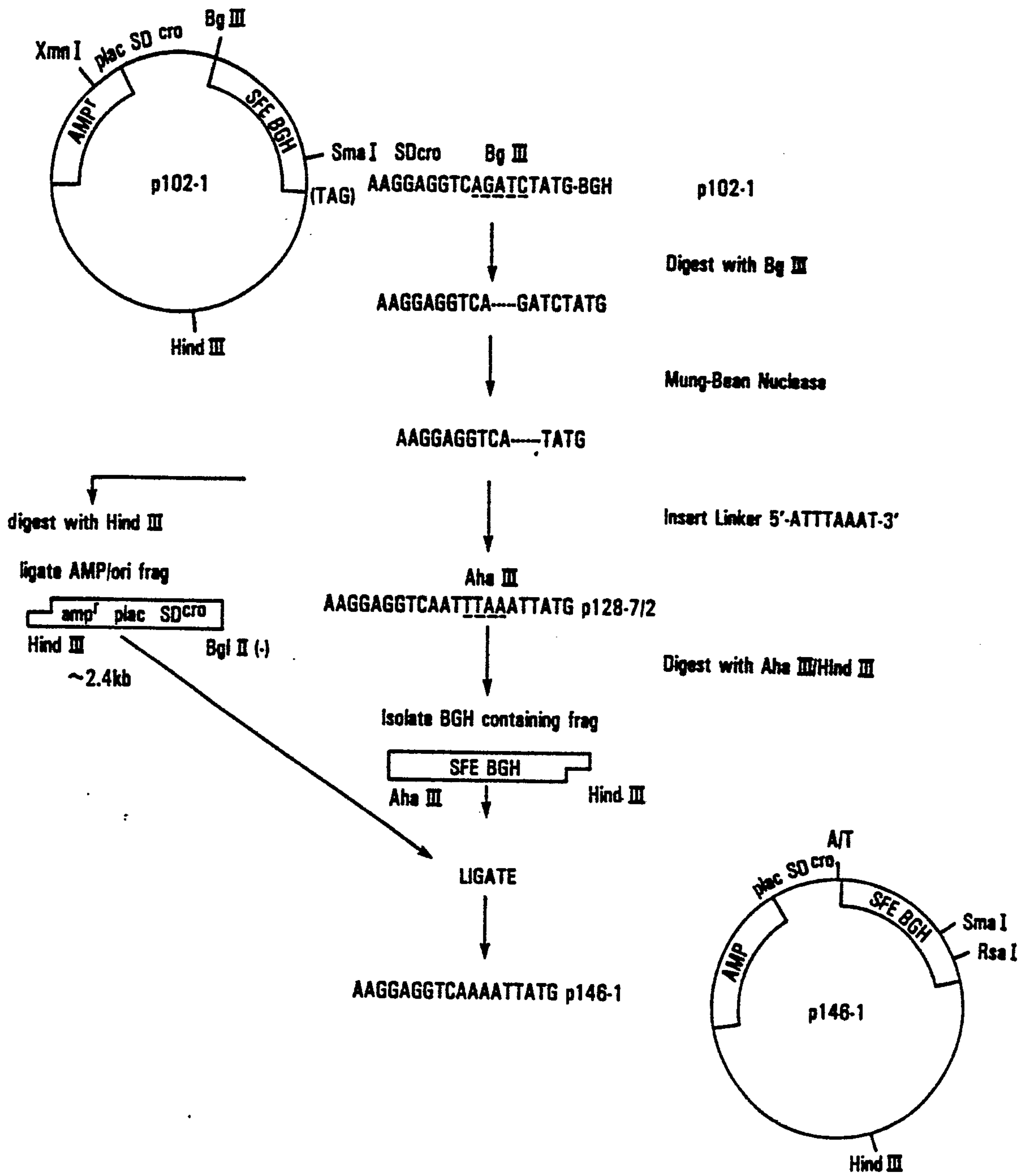


FIG. 15

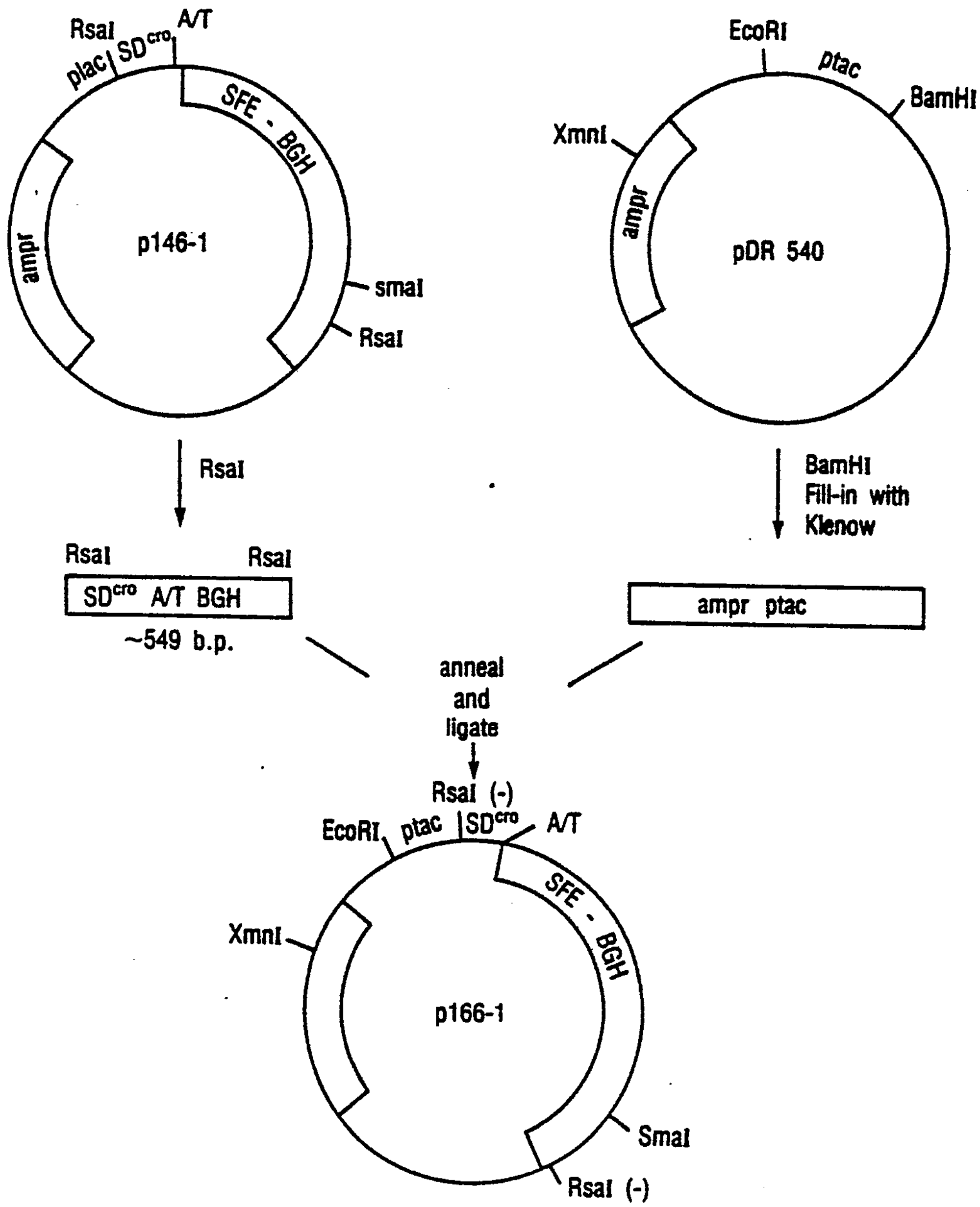
RsaI
SDcro
A/T
plac
SFE - BGH
ampr
p146-1
smaI
RsaI
EcoRI
ptac
BamHI
XmnI
ampr
pDR 540
RsaI
BamHI
Fill-in with
Klenow
RsaI
RsaI
SDcro A/T BGH
~549 b.p.
ampr ptac
anneal
and
ligate
RsaI (-)
EcoRI
ptac
SDcro
A/T
SFE - BGH
XmnI
p166-1
SmaI
RsaI (-)

FIG. 17

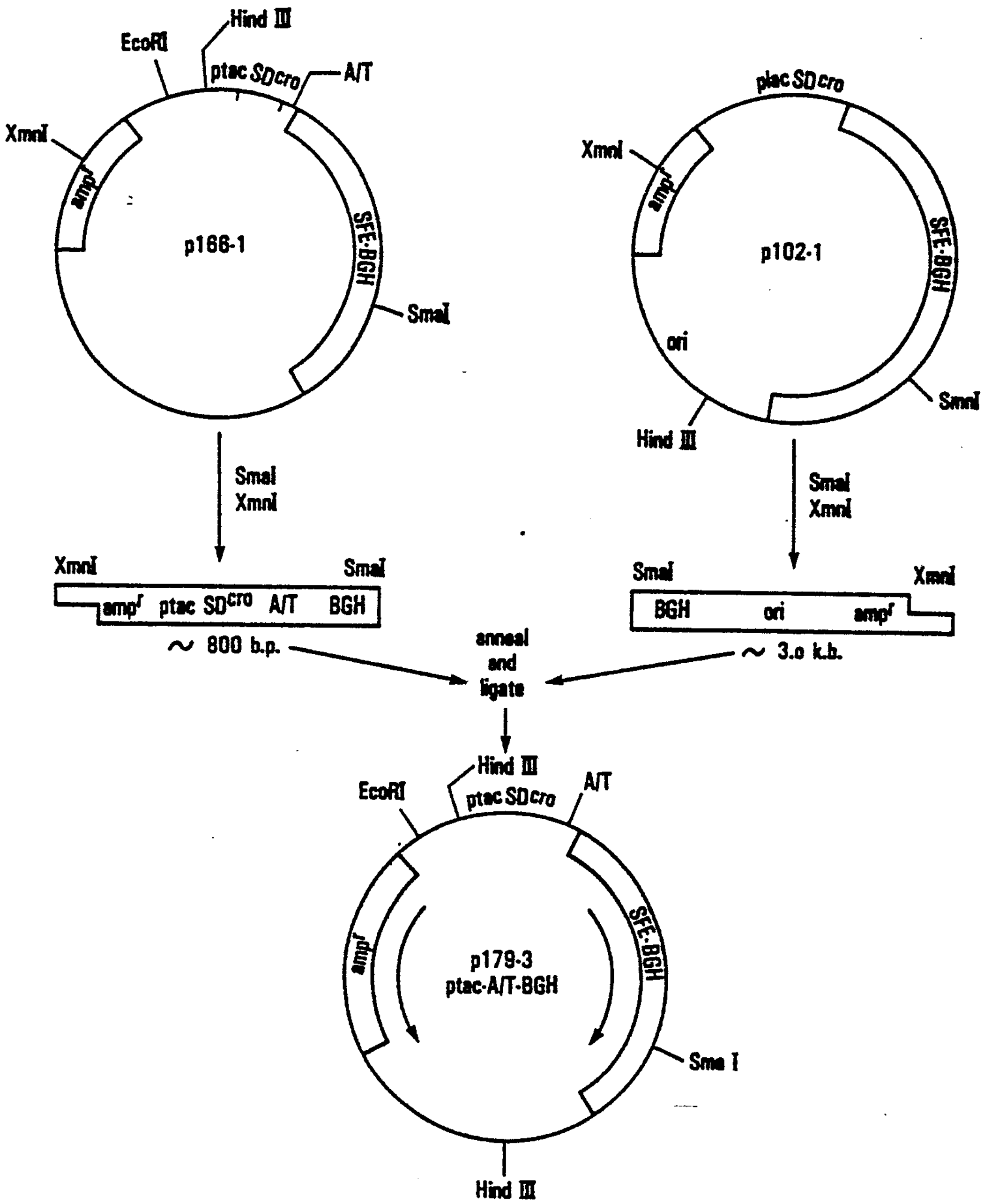

Hind III
EcoRI
ptac SDcro
A/T
XmnI
ampr
p166-1
SFE-BGH
SmaI
placSDcro
XmnI
ampr
p102-1
SFE-BGH
ori
SmaI
Hind III
SmaI
XmnI
SmaI
XmnI
XmnI
SmaI
ampr ptac SDcro A/T BGH
~ 800 b.p.
SmaI
XmnI
BGH ori ampr
~ 3.0 k.b.
anneal
and
ligate
Hind III
EcoRI
ptac SDcro
A/T
ampr
p179-3
ptac-A/T-BGH
SFE-BGH
Sma I
Hind III


FIG. 17A

INSERTION OF TRIPLE TRANSLATIONAL STOP SEQUENCES



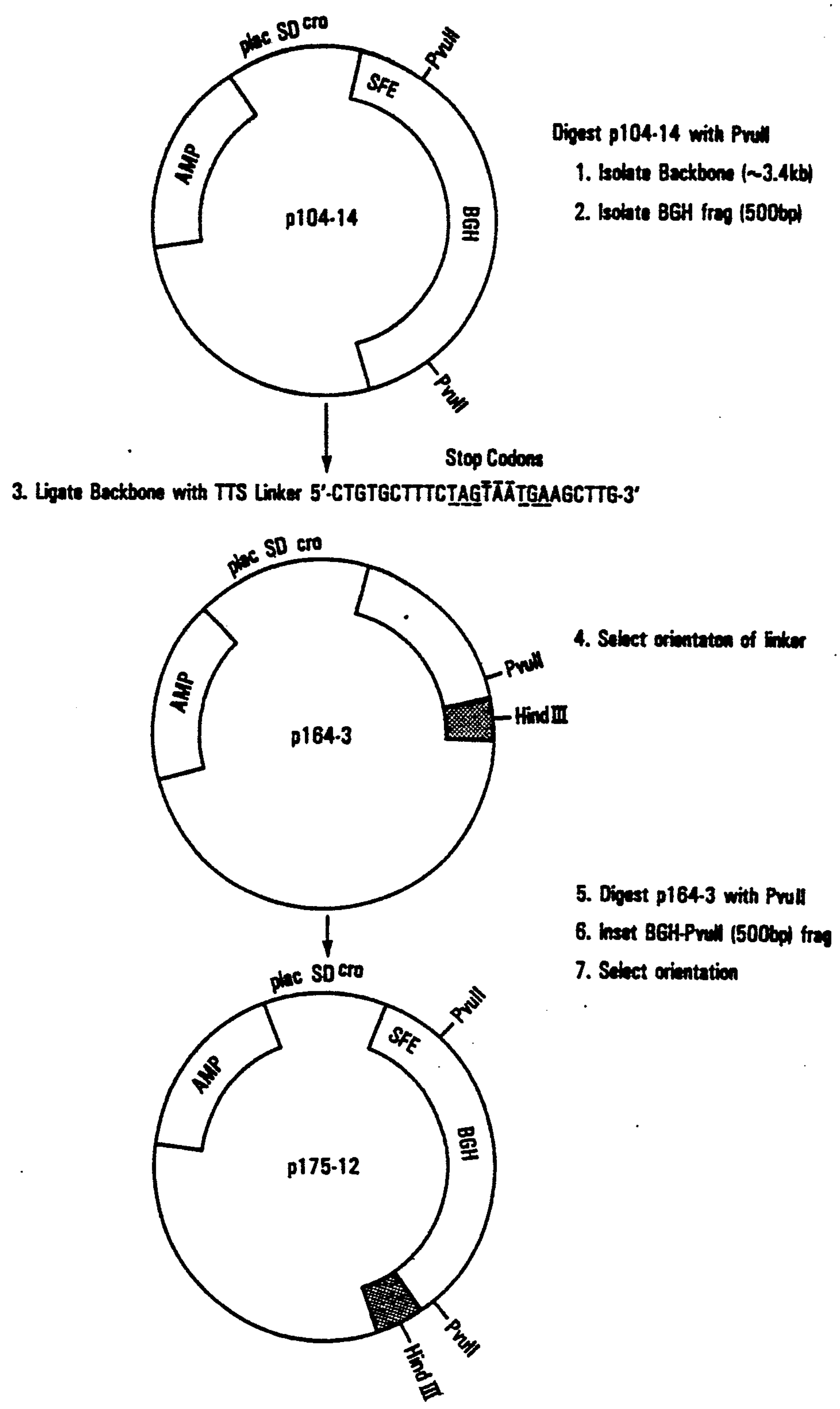


FIG. 18

INSERTION OF trp ATTENUATOR SEQUENCES 

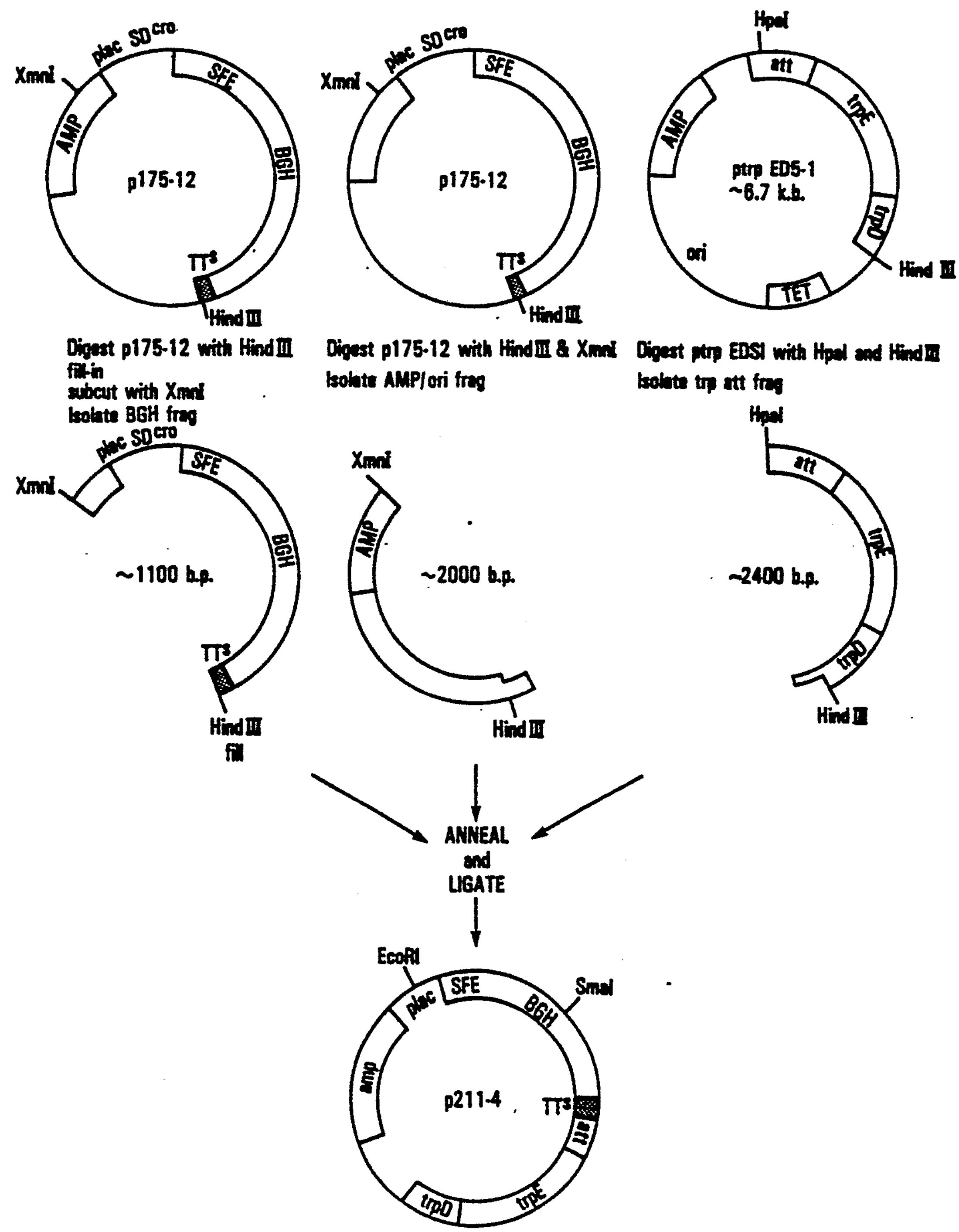


FIG. 19

CONSTRUCTION OF pTAC-(A/T)-BGH-TERM

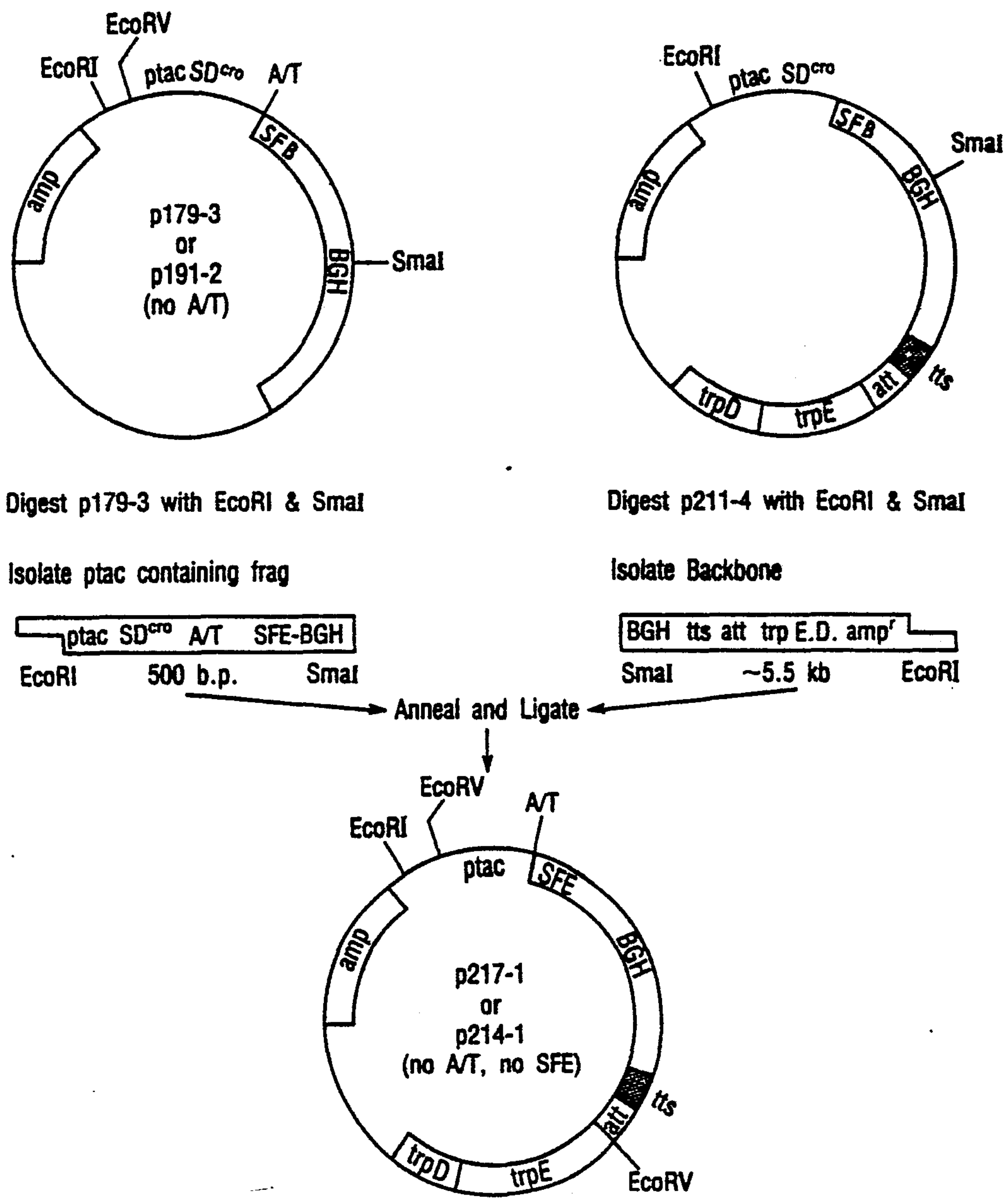


FIG. 20

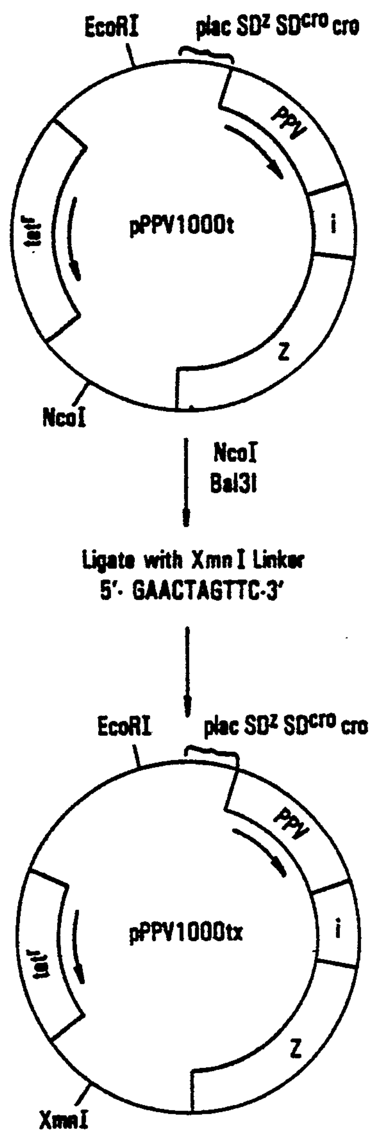
EcoRI
plac SD^z SD^cro cro
PPV
i
Z
tet^r
pPPV1000t
NcoI
NcoI
Bal31
Ligate with XmnI Linker
5'- GAACTAGTTC-3'
EcoRI
plac SD^z SD^cro cro
PPV
i
Z
tet^r
pPPV1000tx
XmnI

FIG. 21

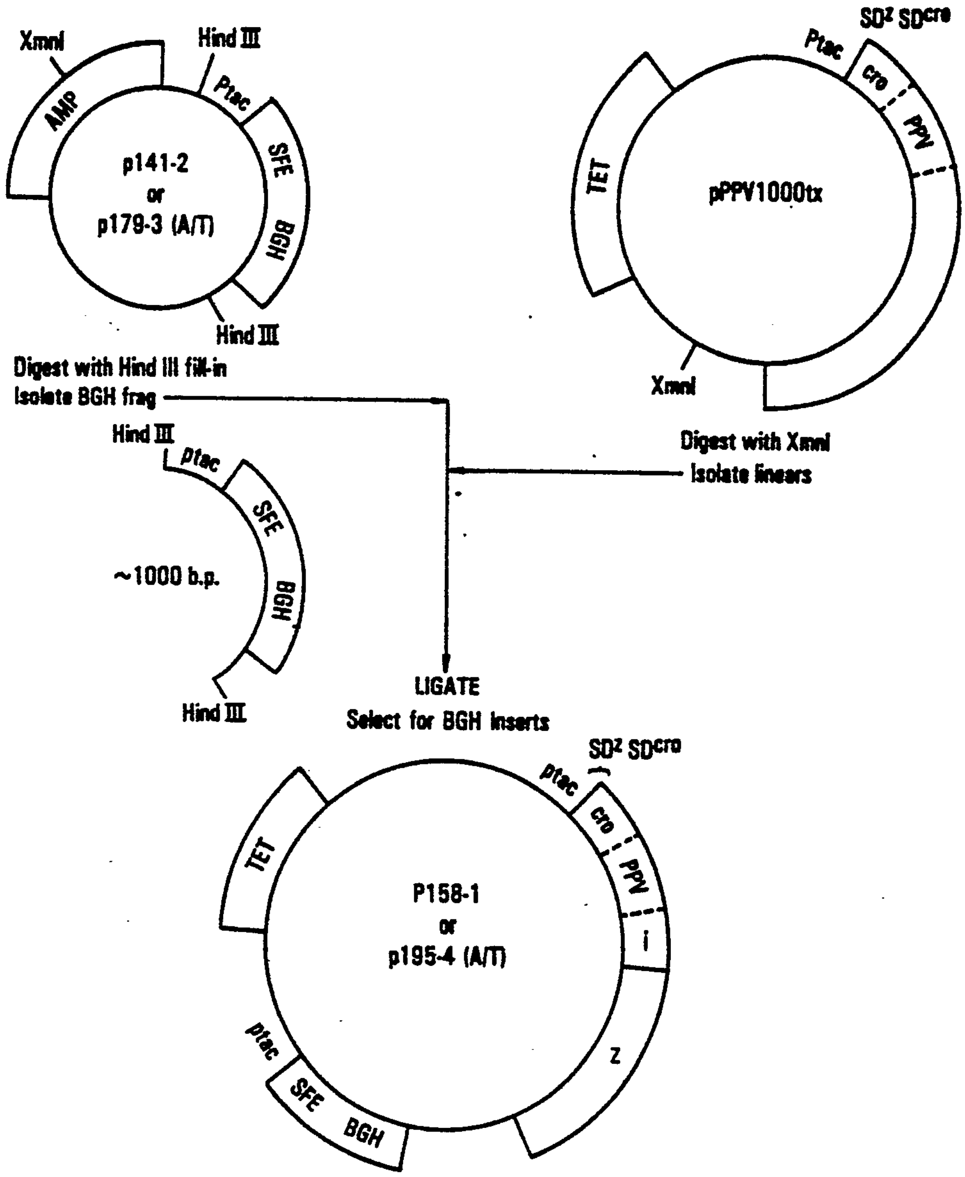
XmnI
Hind III
AMP
Ptac
SFE
BGH
p141-2
or
p179-3 (A/T)
Hind III
Digest with Hind III fill-in
Isolate BGH frag
SD$^z$ SD$^{cro}$
Ptac
cro
PPV
TET
pPPV1000tx
XmnI
Digest with XmnI
Isolate linears
Hind III
ptac
SFE
~1000 b.p.
BGH
Hind III
LIGATE
Select for BGH inserts
SD$^z$ SD$^{cro}$
ptac
cro
PPV
TET
P158-1
or
p195-4 (A/T)
i
z
ptac
SFE
BGH

FIG. 22

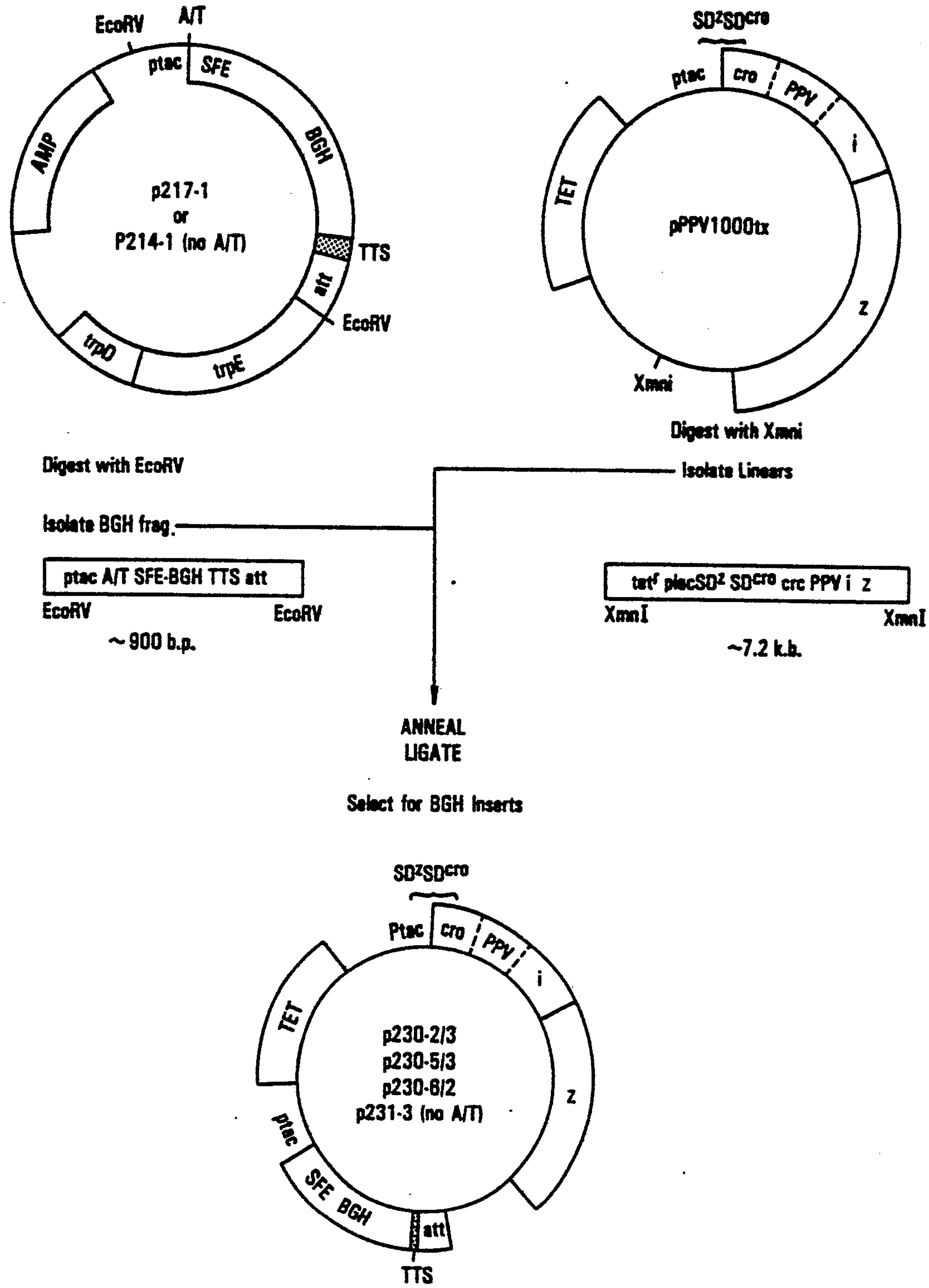

EcoRV
A/T
ptac
SFE
BGH
TTS
att
EcoRV
trpE
trpD
AMP
p217-1
or
P214-1 (no A/T)
SD^z SD^cro
ptac
cro
PPV
i
z
TET
pPPV1000tx
Xmni
Digest with Xmni
Digest with EcoRV
Isolate Linears
Isolate BGH frag.
ptac A/T SFE-BGH TTS att
EcoRV
EcoRV
~900 b.p.
tet^r placSD^z SD^cro crc PPV i z
XmnI
XmnI
~7.2 k.b.
ANNEAL
LIGATE
Select for BGH Inserts
SD^z SD^cro
Ptac
cro
PPV
i
z
TET
ptac
SFE BGH
att
TTS
p230-2/3
p230-5/3
p230-6/2
p231-3 (no A/T)


FIG. 23

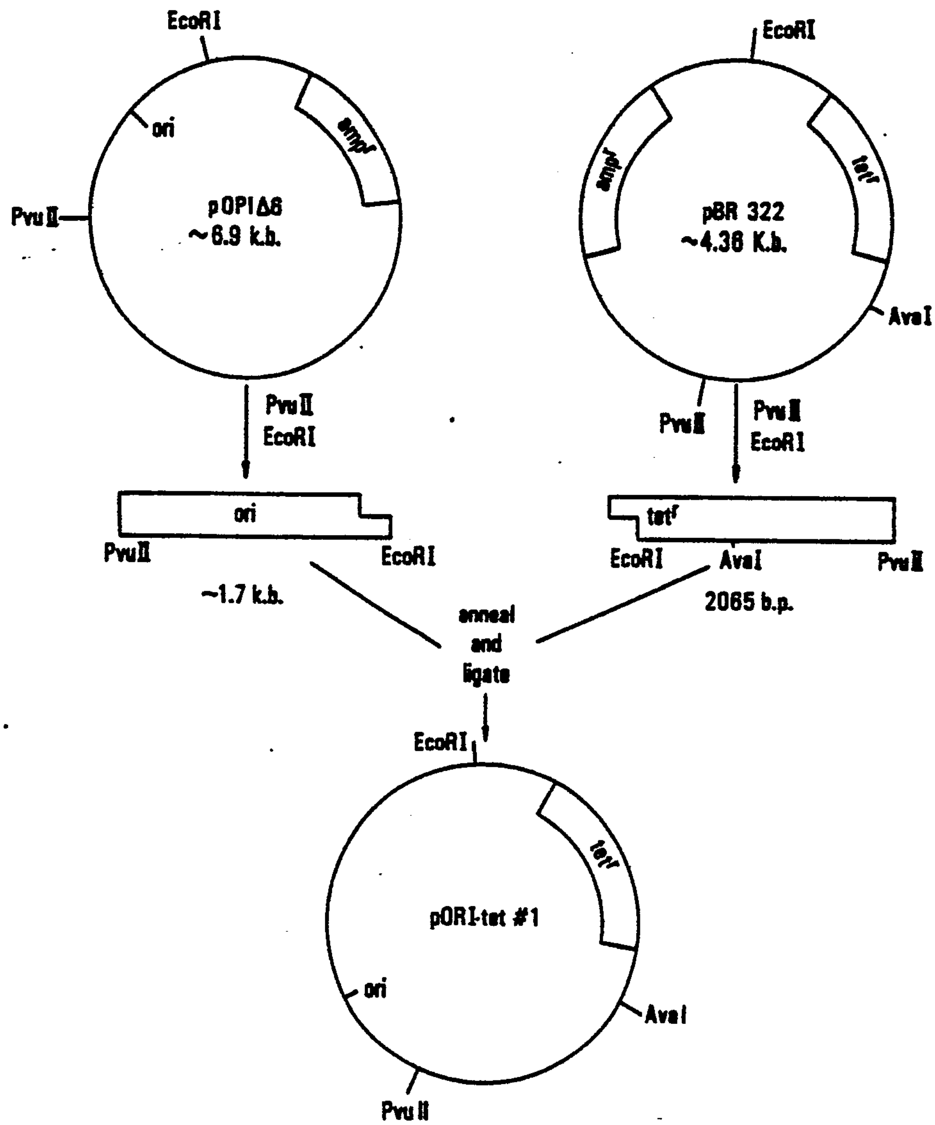
EcoRI
ori
amp^r
PvuII
pOPIΔ6
~6.9 k.b.
PvuII
EcoRI
ori
PvuII
EcoRI
~1.7 k.b.
EcoRI
amp^r
tet^r
pBR 322
~4.36 K.b.
AvaI
PvuII
PvuII
EcoRI
tet^r
EcoRI
AvaI
PvuII
2065 b.p.
anneal
and
ligate
EcoRI
tet^r
pORI-tet #1
ori
AvaI
PvuII

FIG. 24

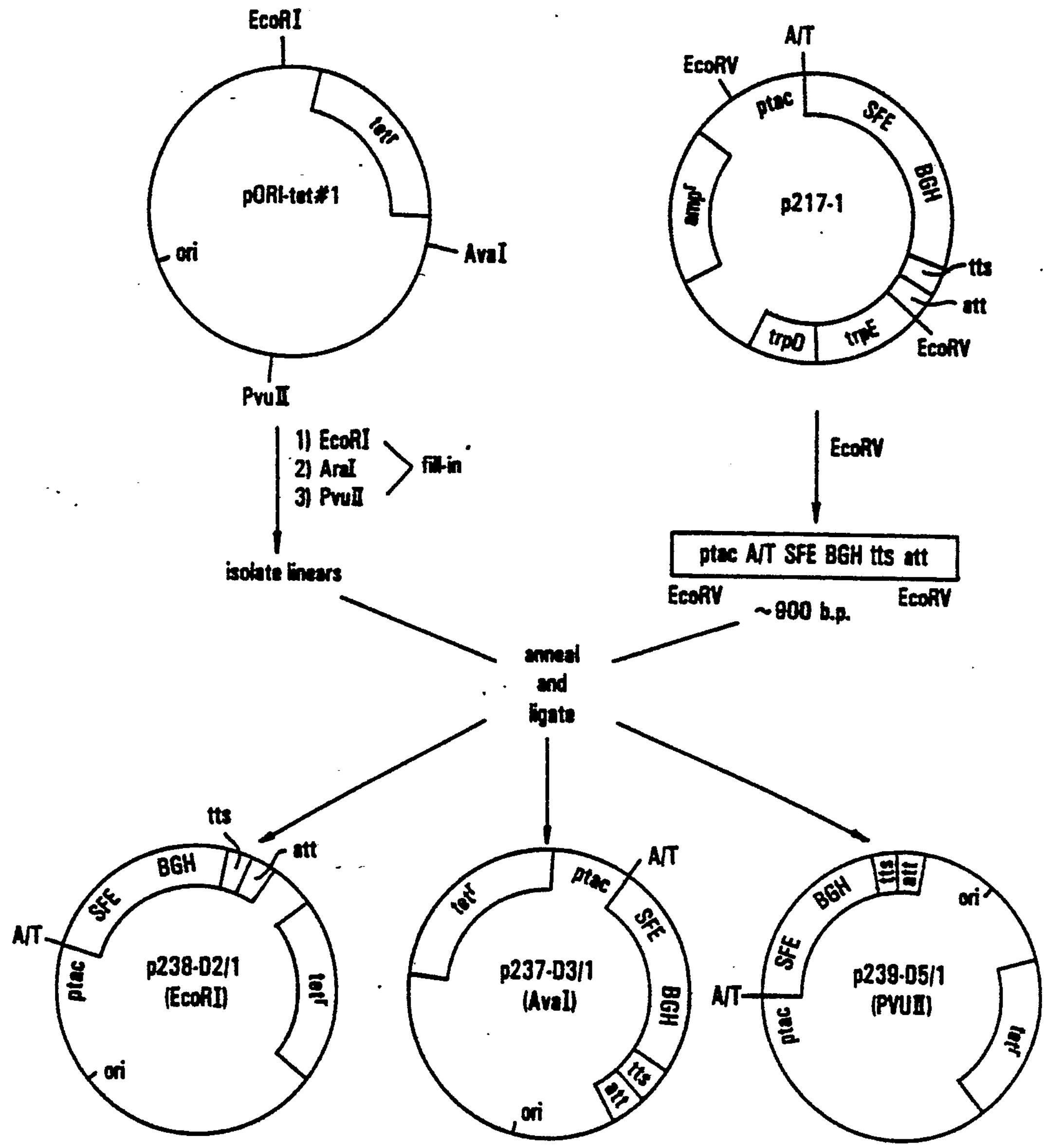
EcoRI
pORI-tet#1
tet
AvaI
ori
PvuII
1) EcoRI
2) AraI
3) PvuII
fill-in
isolate linears
A/T
EcoRV
ptac
SFE
BGH
p217-1
amp
tts
att
trpD
trpE
EcoRV
EcoRV
ptac A/T SFE BGH tts att
EcoRV
~900 b.p.
EcoRV
anneal
and
ligate
tts
att
BGH
SFE
A/T
ptac
p238-D2/1
(EcoRI)
tet
ori
A/T
ptac
tet
SFE
p237-D3/1
(AvaI)
BGH
tts
att
ori
BGH
tts
att
ori
SFE
A/T
ptac
p239-D5/1
(PVUII)
tet

FIG. 25

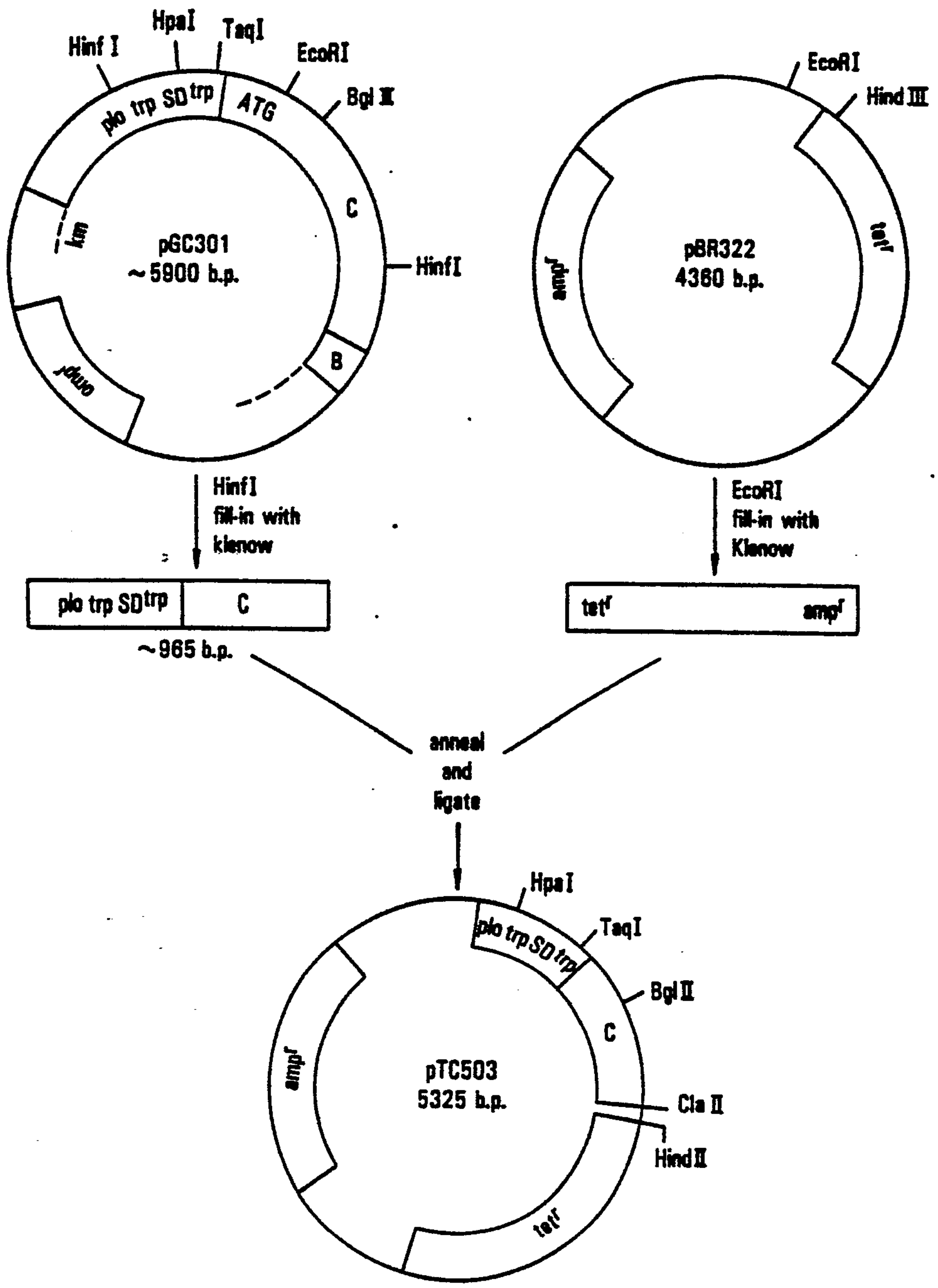

Hinf I
HpaI
TaqI
EcoRI
Bgl II
plo trp SD trp
ATG
C
km
pGC301
~5900 b.p.
HinfI
B
amp^r
EcoRI
Hind III
pBR322
4360 b.p.
amp^r
tet^r
HinfI
fill-in with
klenow
EcoRI
fill-in with
Klenow
plo trp SD trp
C
~965 b.p.
tet^r
amp^r
anneal
and
ligate
HpaI
TaqI
plo trp SD trp
Bgl II
C
amp^r
pTC503
5325 b.p.
Cla II
Hind II
tet^r


FIG. 26

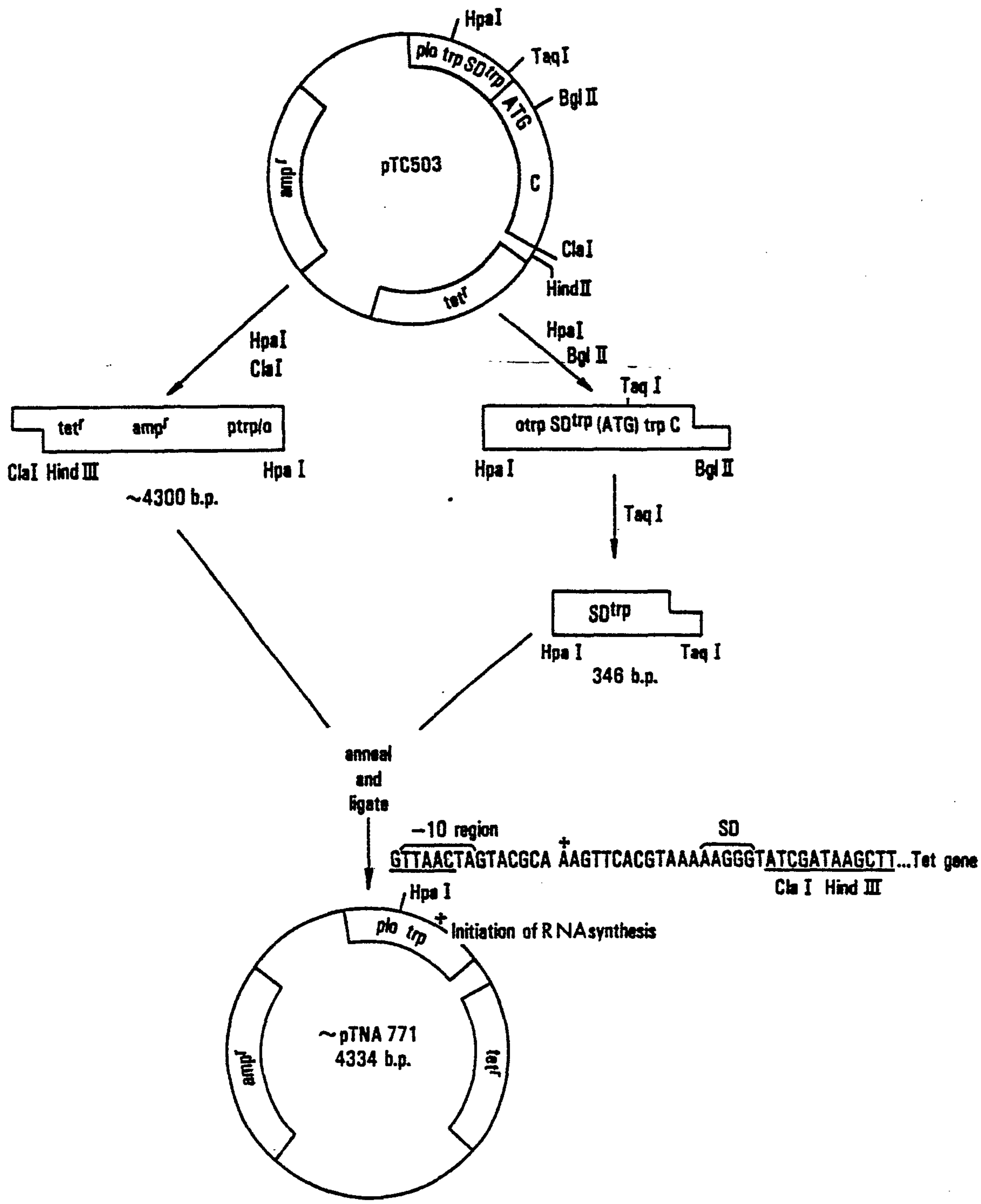
HpaI
plo trp SDtrp
TaqI
ATG
Bgl II
ampr
pTC503
C
ClaI
HindII
tetr
HpaI
ClaI
HpaI
Bgl II
Taq I
tetr ampr ptrp/o
ClaI Hind III
Hpa I
~4300 b.p.
otrp SDtrp (ATG) trp C
Hpa I
Bgl II
Taq I
SDtrp
Hpa I
Taq I
346 b.p.
anneal
and
ligate
−10 region
SD
GTTAACTAGTACGCA AAGTTCACGTAAAAAGGGTATCGATAAGCTT...Tet gene
Cla I Hind III
Hpa I
plo trp
Initiation of RNAsynthesis
~pTNA 771
4334 b.p.
ampr
tetr

FIG. 27

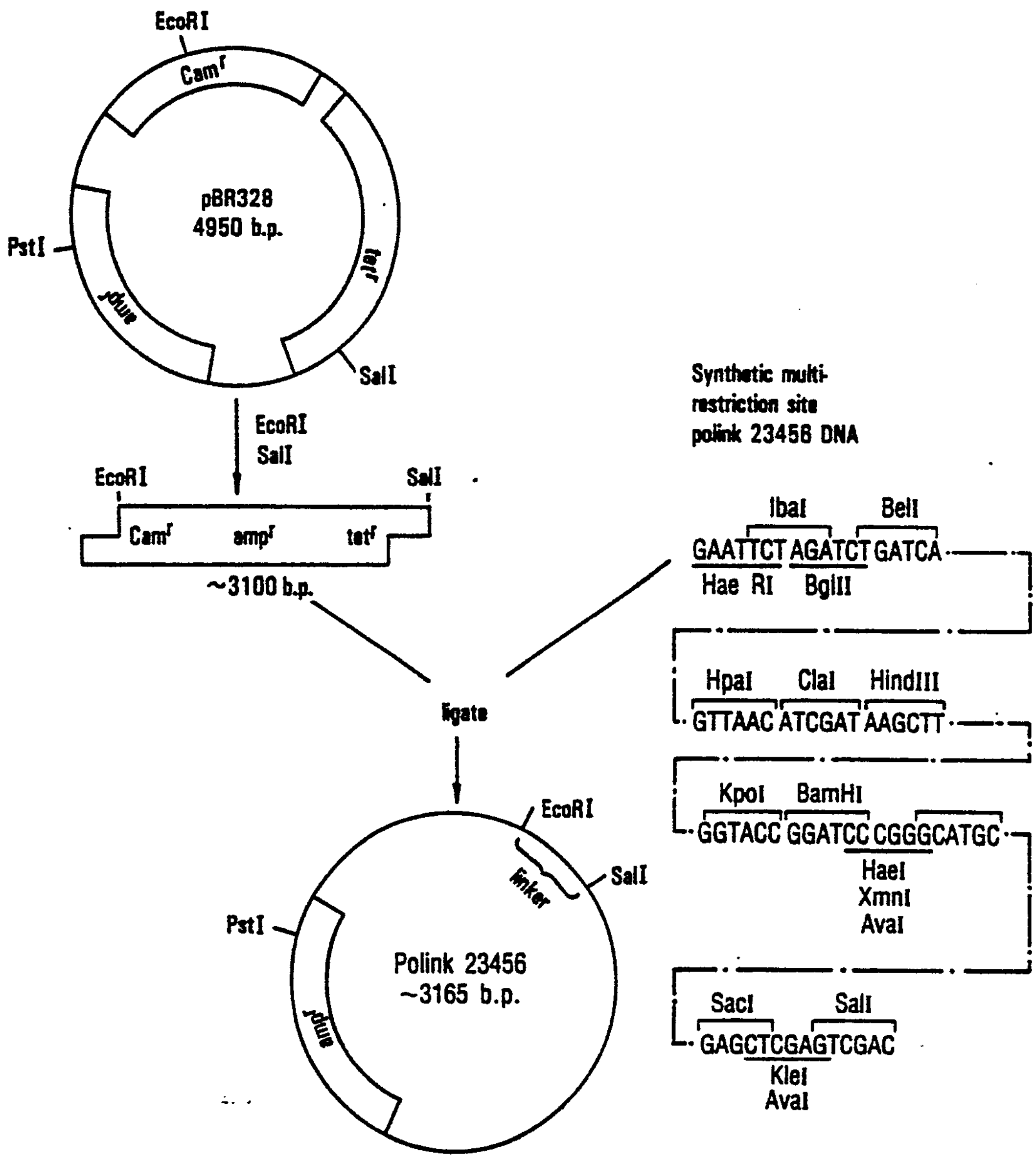
EcoRI
Cam$^r$
pBR328
4950 b.p.
PstI
amp$^r$
tet$^r$
SalI
EcoRI
SalI
EcoRI
SalI
Cam$^r$
amp$^r$
tet$^r$
~3100 b.p.
Synthetic multi-restriction site polink 23456 DNA
IbaI
BelI
GAATTCT AGATCT GATCA
Hae RI
BglII
HpaI
ClaI
HindIII
GTTAAC ATCGAT AAGCTT
KpoI
BamHI
GGTACC GGATCC CGGGCATGC
HaeI
XmnI
AvaI
SacI
SalI
GAGCTCGAGTCGAC
KleI
AvaI
ligate
EcoRI
SalI
linker
PstI
Polink 23456
~3165 b.p.
amp$^r$

FIG. 28

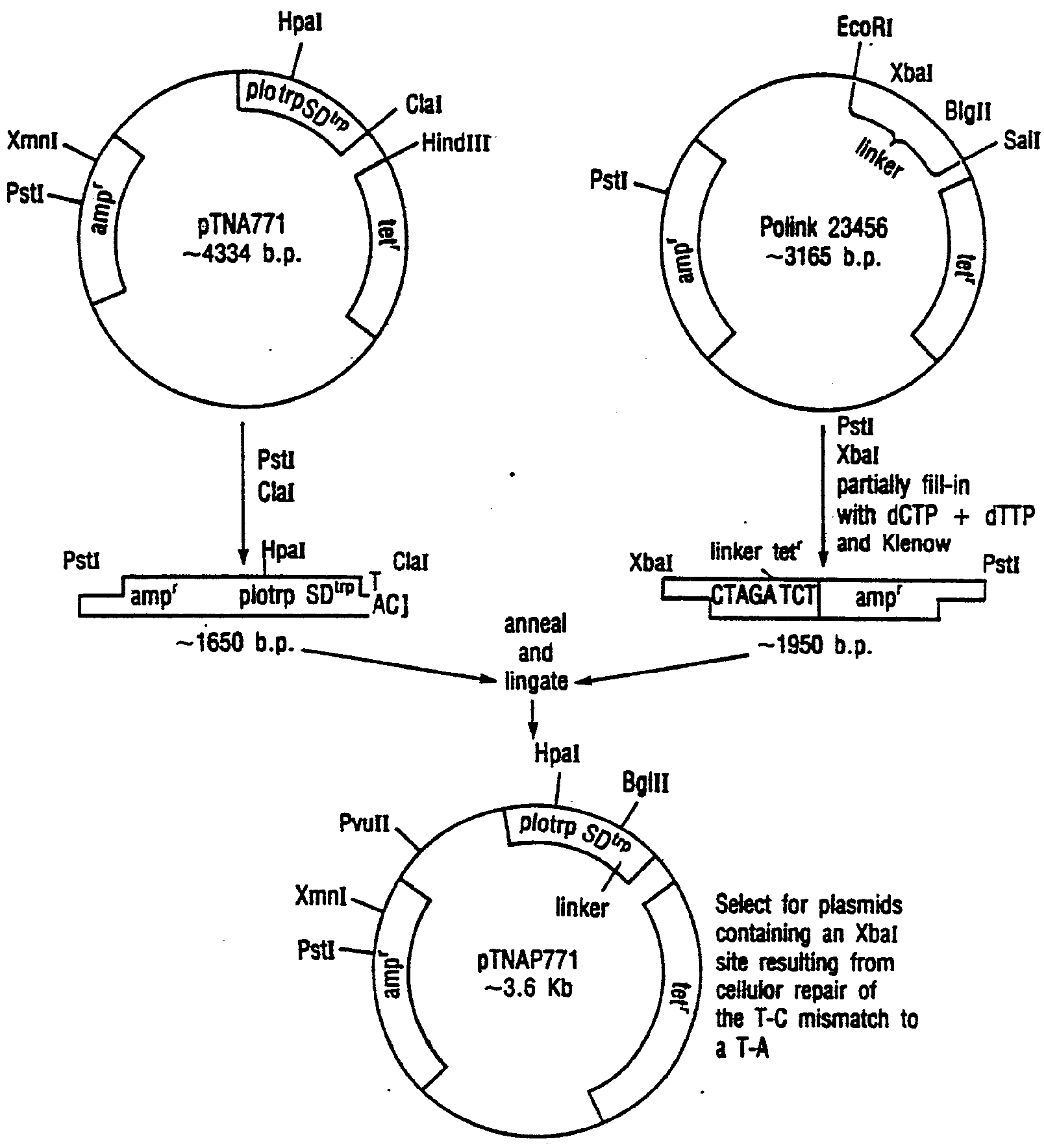

HpaI
plotrpSDtrp
ClaI
HindIII
XmnI
PstI
ampr
pTNA771
~4334 b.p.
tetr
EcoRI
XbaI
BglI
SalI
linker
PstI
ampr
Polink 23456
~3165 b.p.
tetr
PstI
ClaI
PstI
XbaI
partially fill-in
with dCTP + dTTP
and Klenow
PstI
HpaI
ClaI
ampr
plotrp SDtrp L
T
AC
~1650 b.p.
XbaI
linker tetr
PstI
CTAGATCT
ampr
~1950 b.p.
anneal
and
lingate
HpaI
BglII
PvuII
plotrp SDtrp
XmnI
linker
PstI
ampr
pTNAP771
~3.6 Kb
tetr
Select for plasmids
containing an XbaI
site resulting from
cellulor repair of
the T-C mismatch to
a T-A


FIG. 29

pTNAP771
PvuII/BglII

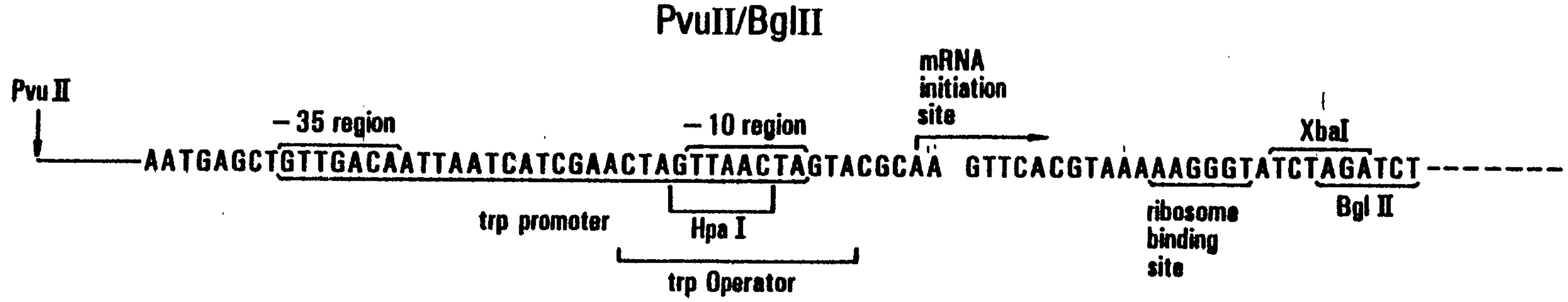


FIG. 30

pMTE-407-29

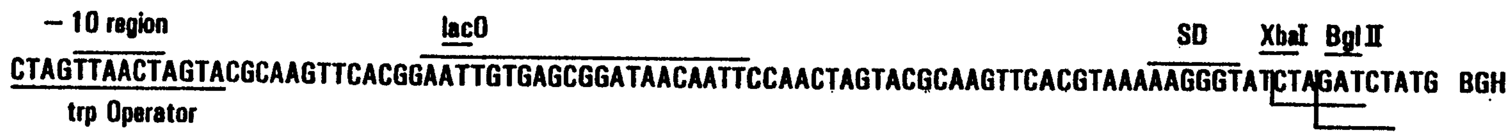


FIG. 34C

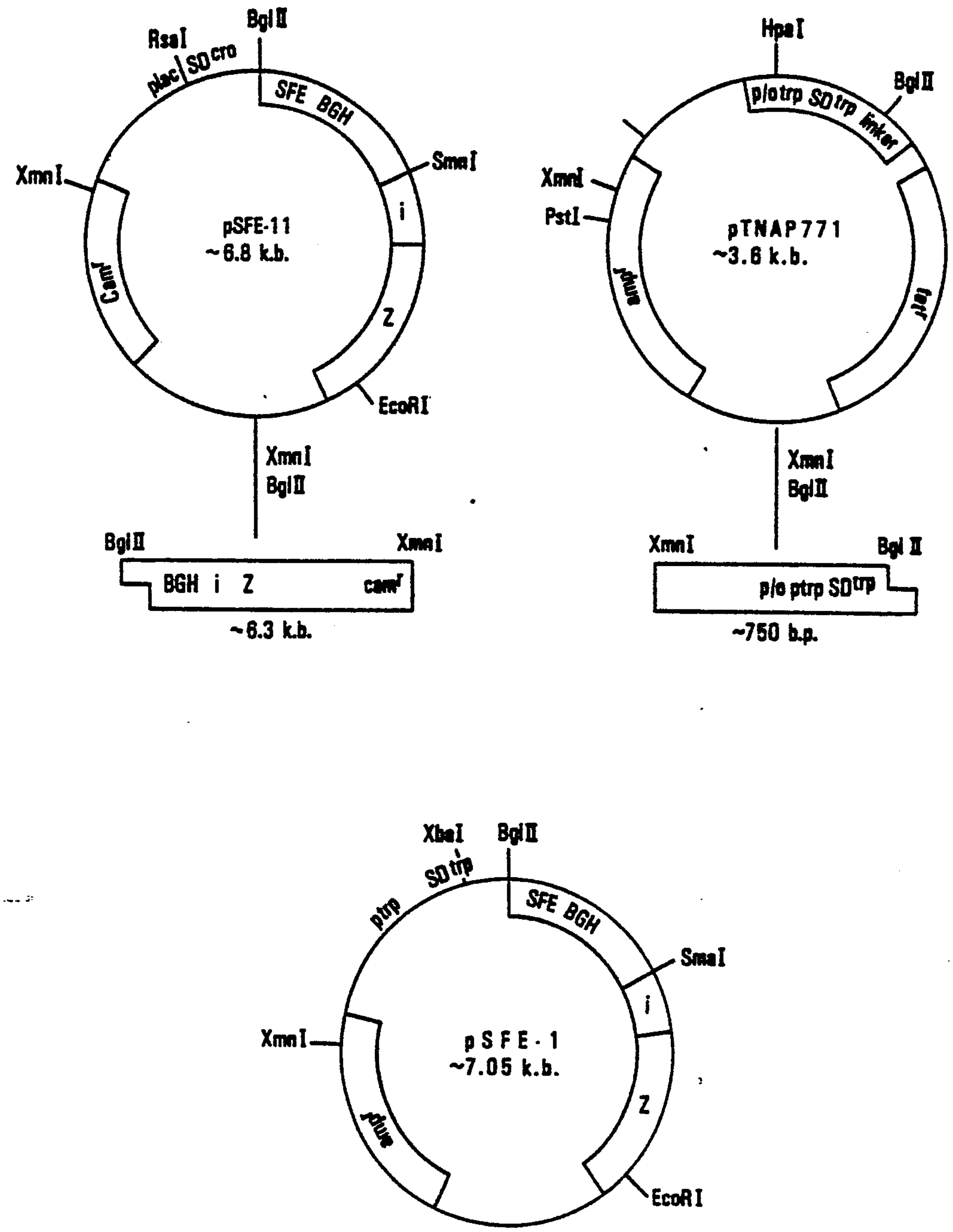
RsaI
BglII
plac
SD cro
SFE BGH
SmaI
XmnI
i
pSFE-11
~6.8 k.b.
Z
EcoRI
XmnI
BglII
BglII
XmnI
BGH i Z
~6.3 k.b.
HpaI
p/otrp SD trp linker
BglII
XmnI
PstI
pTNAP771
~3.6 k.b.
XmnI
BglII
XmnI
Bgl II
p/o ptrp SD trp
~750 b.p.
XbaI
BglII
ptrp
SD trp
SFE BGH
SmaI
XmnI
i
pSFE-1
~7.05 k.b.
Z
EcoRI

FIG. 31

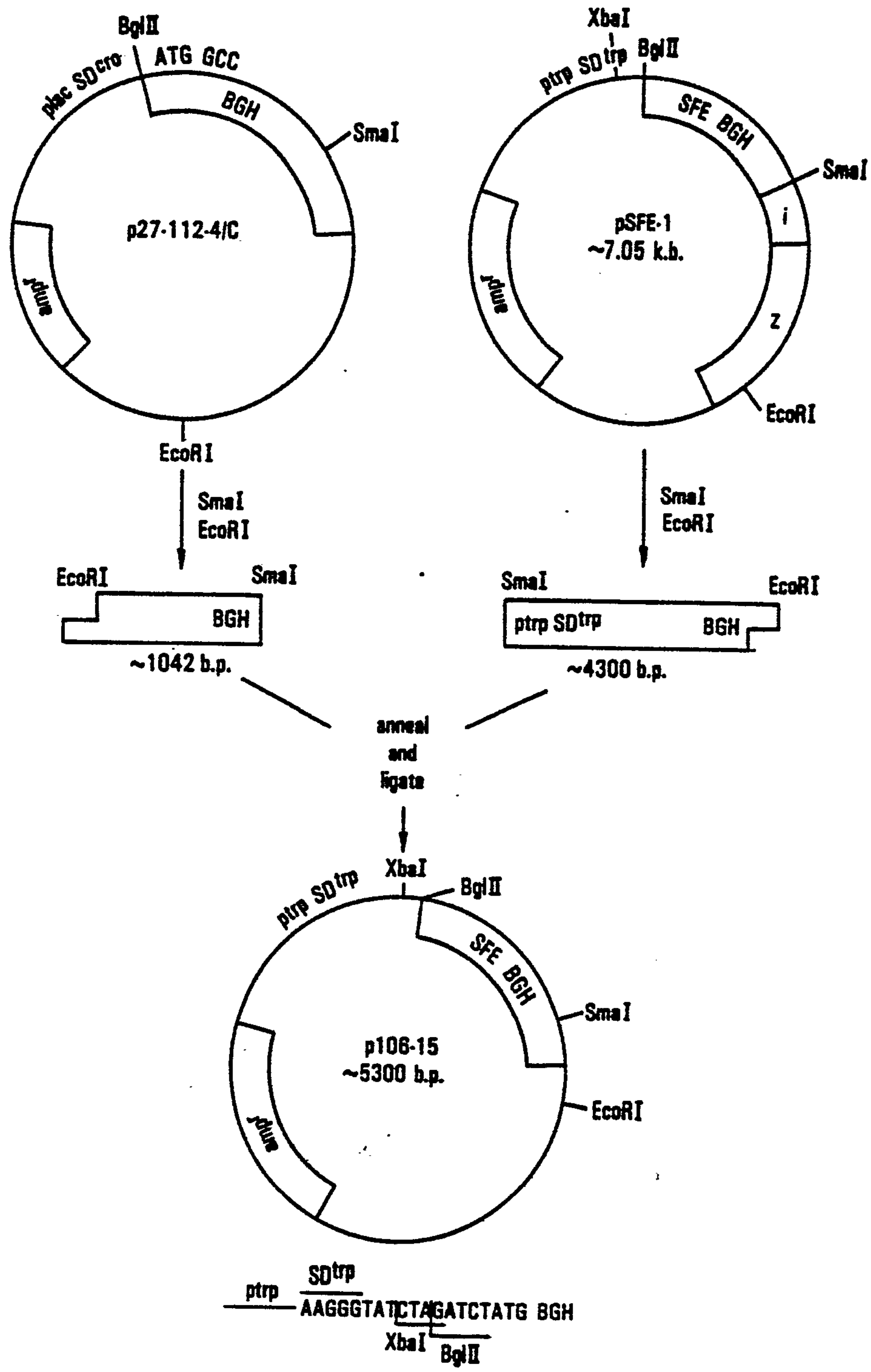
BglII
ATG GCC
plac SD^cro
BGH
SmaI
p27-112-4/C
amp^r
EcoRI
SmaI
EcoRI
EcoRI
SmaI
BGH
~1042 b.p.
XbaI
ptrp SD trp
BglII
SFE BGH
SmaI
pSFE-1
~7.05 k.b.
i
amp^r
z
EcoRI
SmaI
EcoRI
SmaI
EcoRI
ptrp SD^trp
BGH
~4300 b.p.
anneal
and
ligate
ptrp SD^trp
XbaI
BglII
SFE BGH
SmaI
p106-15
~5300 b.p.
amp^r
EcoRI
SD^trp
ptrp
AAGGGTATCTAGATCTATG BGH
XbaI
BglII

FIG. 32

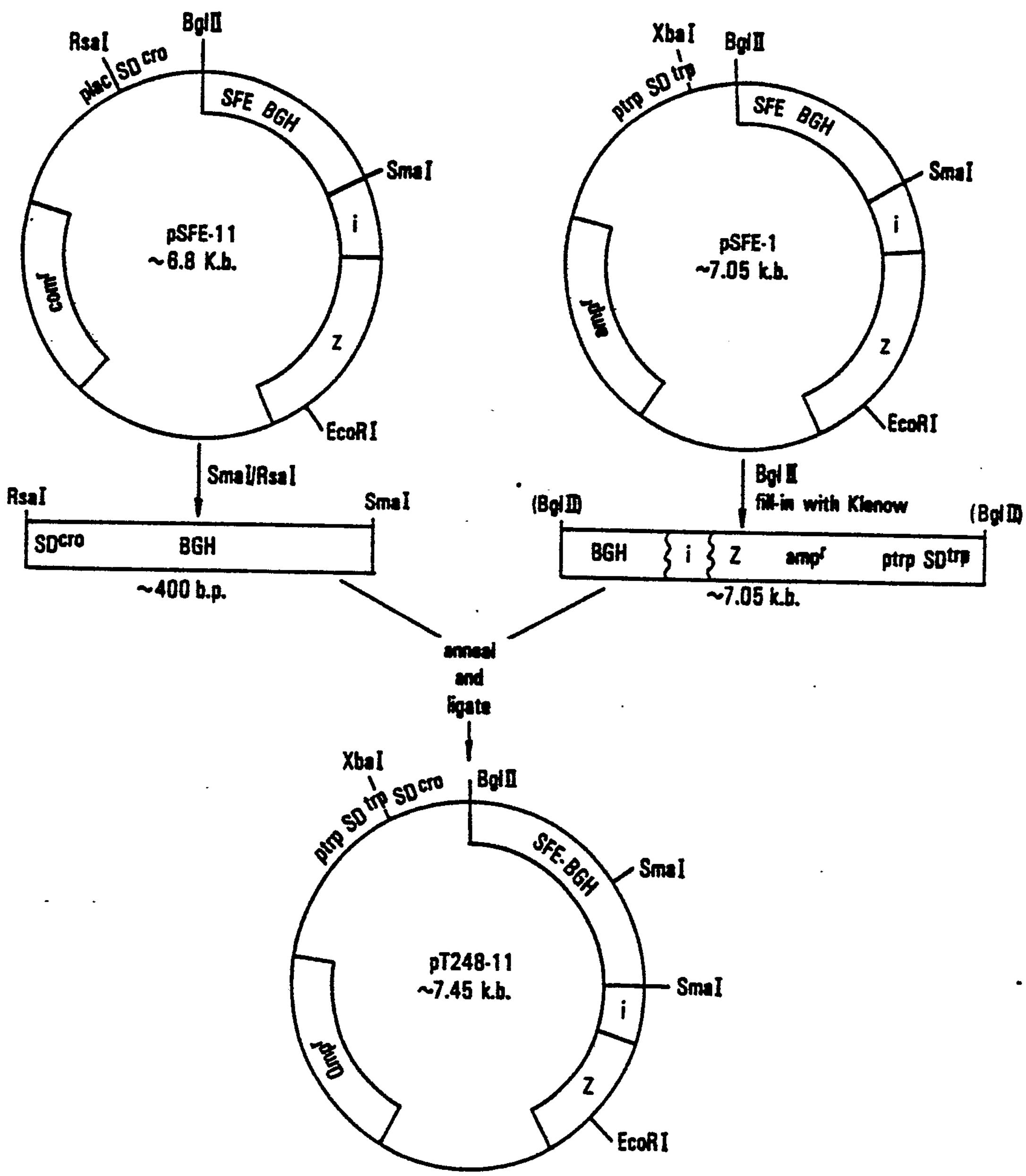
RsaI
plac
SD cro
BglII
SFE BGH
SmaI
i
pSFE-11
~6.8 K.b.
camr
Z
EcoRI
XbaI
ptrp SD trp
BglII
SFE BGH
SmaI
i
pSFE-1
~7.05 k.b.
ampr
Z
EcoRI
SmaI/RsaI
RsaI
SmaI
SDcro
BGH
~400 b.p.
BglII
fill-in with Klenow
(BglII)
(BglII)
BGH
i
Z
ampr
ptrp SDtrp
~7.05 k.b.
anneal
and
ligate
XbaI
ptrp SD trp SD cro
BglII
SFE-BGH
SmaI
SmaI
i
pT248-11
~7.45 k.b.
ampr
Z
EcoRI

FIG. 33

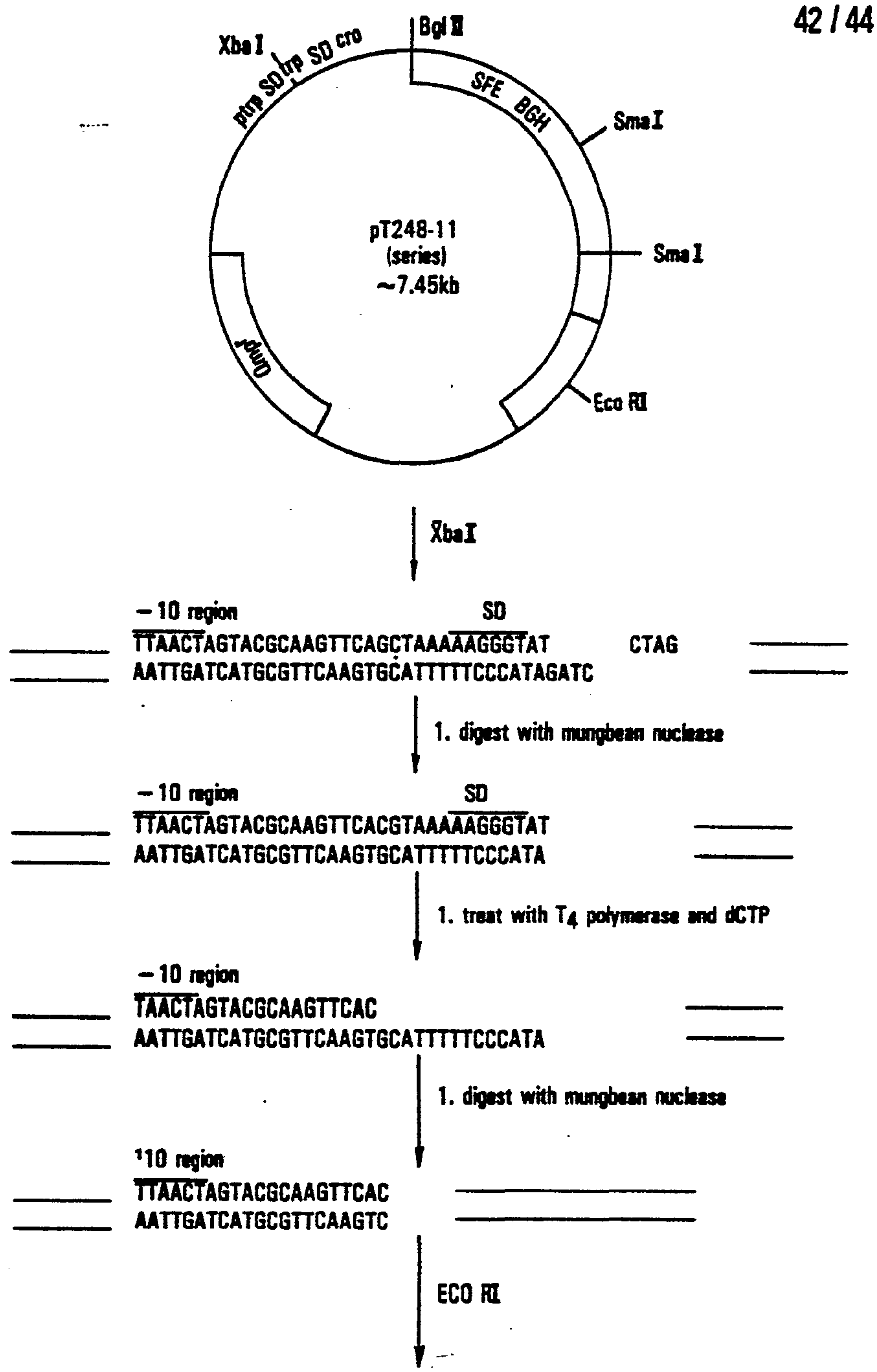
Xba I
ptrp SD
trp SD
cro
Bgl II
SFE
BGH
Sma I
Sma I
pT248-11
(series)
~7.45kb
Eco RI
Xba I
− 10 region
SD
TTAACTAGTACGCAAGTTCAGCTAAAAAGGGTAT
CTAG
AATTGATCATGCGTTCAAGTGCATTTTTCCCATAGATC
1. digest with mungbean nuclease
− 10 region
SD
TTAACTAGTACGCAAGTTCACGTAAAAAGGGTAT
AATTGATCATGCGTTCAAGTGCATTTTTCCCATA
1. treat with $T_4$ polymerase and dCTP
− 10 region
TAACTAGTACGCAAGTTCAC
AATTGATCATGCGTTCAAGTGCATTTTTCCCATA
1. digest with mungbean nuclease
10 region
TTAACTAGTACGCAAGTTCAC
AATTGATCATGCGTTCAAGTC
ECO RI

FIG. 34

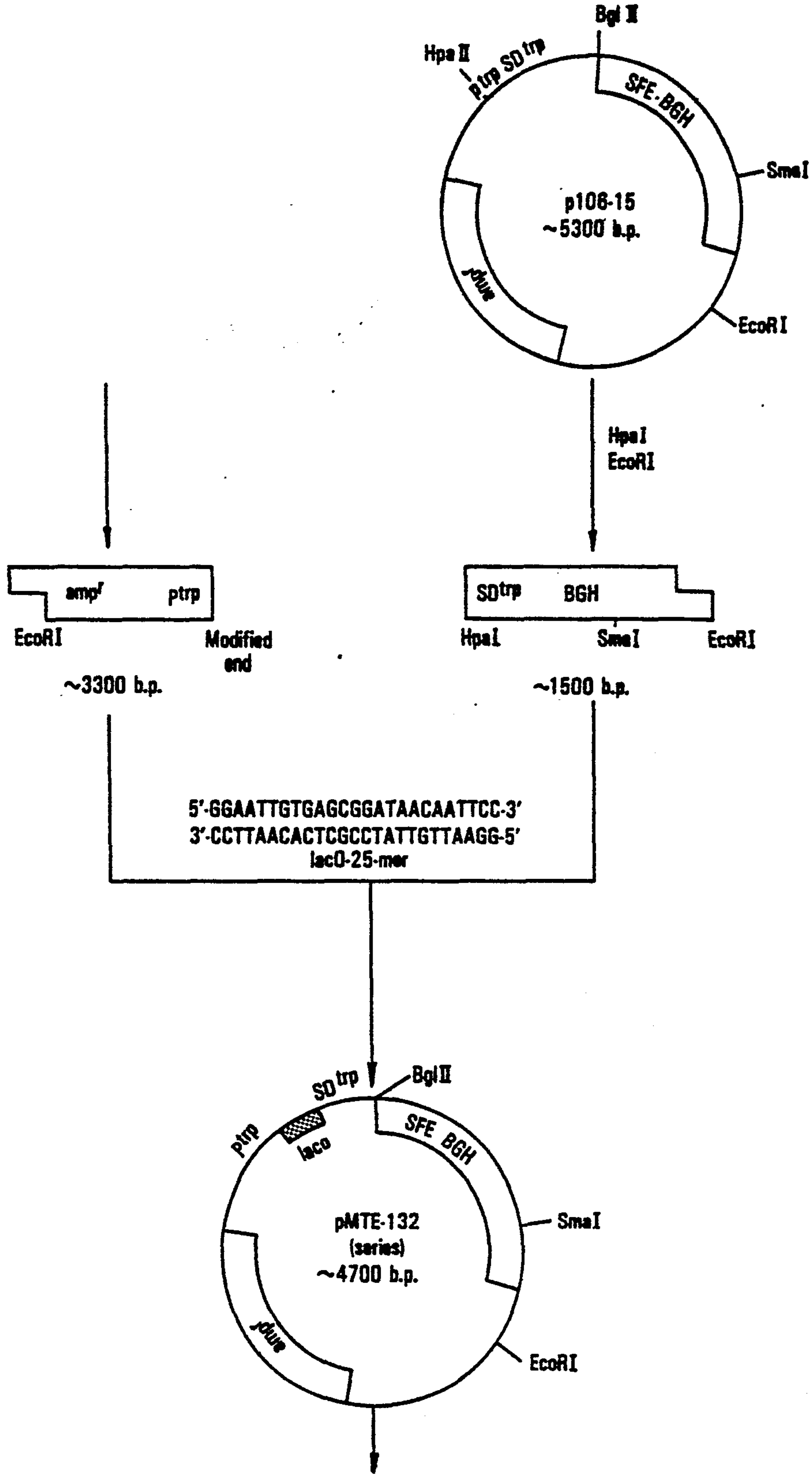
Bgl II
Hpa II
ptrp SD trp
SFE-BGH
SmaI
p108-15
~5300 b.p.
ampr
EcoRI
HpaI
EcoRI
ampr
ptrp
EcoRI
Modified end
~3300 b.p.
SDtrp
BGH
HpaI
SmaI
EcoRI
~1500 b.p.
5'-GGAATTGTGAGCGGATAACAATTCC-3'
3'-CCTTAACACTCGCCTATTGTTAAGG-5'
lacO-25-mer
SD trp
BglII
ptrp
laco
SFE BGH
SmaI
pMTE-132
(series)
~4700 b.p.
ampr
EcoRI

FIG. 34A

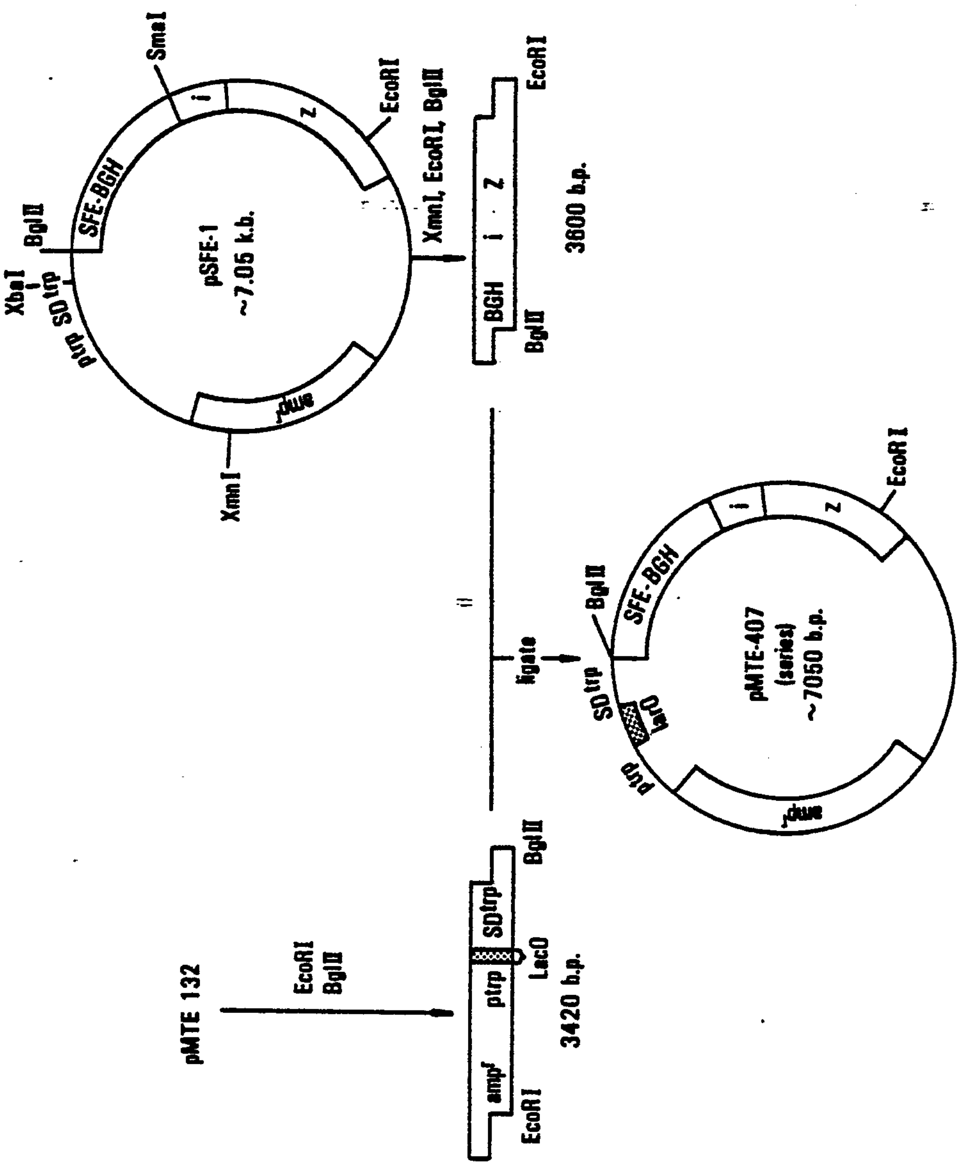

pSFE-1
~7.05 k.b.
SmaI
EcoRI
XbaI
BglII
ptrp SDtrp
SFE-BGH
i
Z
amp^r
XmnI
XmnI, EcoRI, BglII
BglII
BGH
EcoRI
3600 b.p.
pMTE 132
EcoRI
BglII
amp^r
ptrp
LacO
SDtrp
3420 b.p.
ligate
pMTE-407
(series)
~7050 b.p.
lacO


FIG. 34B